(19) Europäisches Patentamt
European Patent Office
Office européen des brevets

(11) EP 1 163 227 B1

(12) EUROPEAN PATENT SPECIFICATION

(45) Date of publication and mention of the grant of the patent:
**28.09.2005 Bulletin 2005/39**

(21) Application number: **00909360.0**

(22) Date of filing: **14.03.2000**

(51) Int Cl.[7]: **C07D 239/94**, C07D 215/54, C07D 401/12, C07D 413/12, C07D 405/12, A61K 31/517, A61P 35/00

(86) International application number:
**PCT/EP2000/002228**

(87) International publication number:
**WO 2000/055141 (21.09.2000 Gazette 2000/38)**

(54) **BICYCLIC HETEROCYCLES, PHARMACEUTICAL COMPOSITIONS CONTAINING THESE COMPOUNDS, AND PROCESSES FOR PREPARING THEM**

BIZYKLISCHE HETEROZYKLEN, DIESE VERBINDUNGEN ENTHALTENDE ZUSAMMENSTELLUNGEN,UND VERFAHREN ZU DEREN HERSTELLUNG

HETEROCYCLES BICYCLIQUES, COMPOSITIONS PHARMACEUTIQUES CONTENANT CES COMPOSES, UTILISATIONS ET PROCEDES DE PREPARATION DE CES DERNIERS

(84) Designated Contracting States:
**AT BE CH CY DE DK ES FI FR GB GR IE IT LI LU MC NL PT SE**
Designated Extension States:
**LT LV RO SI**

(30) Priority: **15.03.1999 DE 19911509**

(43) Date of publication of application:
**19.12.2001 Bulletin 2001/51**

(73) Proprietor: **Boehringer Ingelheim Pharma GmbH & Co.KG**
**55218 Ingelheim am Rhein (DE)**

(72) Inventors:
- **HIMMELSBACH, Frank**
  **88441 Mittelbiberach (DE)**
- **LANGKOPF, Elke**
  **88447 Warthausen (DE)**
- **BLECH, Stefan**
  **88447 Warthausen (DE)**
- **JUNG, Birgit**
  **55270 Schwabenheim (DE)**
- **METZ, Thomas**
  **1030 Vienna (AT)**
- **SOLCA, Flavio**
  **1230 Vienna (AT)**

(74) Representative: **Kläs, Heinz-Gerd et al**
**Boehringer Ingelheim GmbH**
**Binger Strasse 173**
**55216 Ingelheim/Rhein (DE)**

(56) References cited:
**EP-A- 0 566 226**
**WO-A-96/33980**
**WO-A-97/30035**
**WO-A-97/32856**
**WO-A-98/13354**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 163 227 B1

## Description

The present invention relates to bicyclic heterocyclic compounds of general formula

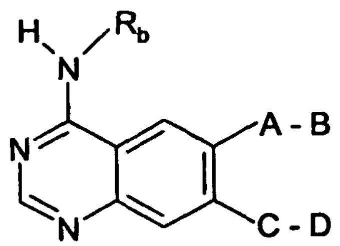

, (I)

the tautomers, the stereoisomers and the salts thereof, particularly the physiologically acceptable salts thereof with inorganic or organic acids or bases which have valuable pharmacological properties, particularly an inhibiting effect on the signal transduction mediated by tyrosine kinases, their use in treating diseases, particularly tumoral diseases, diseases of the lungs and respiratory tract and the preparation thereof.

Both WO 97/30035 and WO 98/13354 describe quinazoline derivatives with a similar substitution pattern, which are useful as VEGF inhibitors for the treatment of cancer.

In the above general formula I

$R_b$ denotes a phenyl, benzyl or 1-phenylethyl group wherein the phenyl nucleus is substituted in each case by the groups $R_1$ to $R_2$, whilst

$R_1$ and $R_2$, which may be identical or different, each denote a hydrogen, fluorine, chlorine or bromine atom,

or a methyl, trifluoromethyl, methoxy, ethynyl or cyano group,

A denotes an -O-$C_{1-4}$-alkylene or -O-$CH_2$-CH(OH)-$CH_2$ group, whilst the oxygen atom of the abovementioned groups in each case is linked to the bicyclic heteroaromatic ring,

B denotes an $R_6$O-CO-alkylene-$NR_5$ group wherein the alkylene moiety, which is straight-chained and contains 1 or 2 carbon atoms, may additionally be substituted by an $R_6$O-CO or $R_6$O-CO-methyl group, whilst

$R_5$ denotes a hydrogen atom,

a $C_{1-2}$-alkyl group which may be substituted by an $R_6$O-CO group,

a $C_{2-4}$-alkyl group which is substituted from position 2 onwards by a hydroxy group,

a $C_{3-6}$-cycloalkyl or $C_{3-6}$-cycloalkylmethyl group and

$R_6$ denotes a hydrogen atom,

a $C_{1-6}$-alkyl, cyclopentyl, cyclopentylmethyl, cyclohexyl, cyclohexylmethyl, phenyl, benzyl, 5-indanyl or $R_g$-CO-O-($R_e$C$R_f$) group, whilst

$R_e$ denotes a hydrogen atom or a $C_{1-4}$-alkyl group,

$R_f$ denotes a hydrogen atom and

$R_g$ denotes a $C_{1-4}$-alkyl, cyclopentyl, cyclohexyl, $C_{1-4}$-alkoxy, cyclopentyloxy or cyclohexyloxy group,

a pyrrolidino or piperidino group which is substituted by an $R_6$O-CO or $R_6$O-CO-$C_{1-2}$-alkyl group wherein $R_6$ is as hereinbefore defined,

a pyrrolidino or piperidino group which is substituted by two $R_6O$-CO or $R_6O$-CO-$C_{1-2}$-alkyl groups wherein $R_6$ is as hereinbefore defined,

a piperazino group which is substituted in the 4 position by the group $R_{10}$ and additionally at a cyclic carbon atom by an $R_6O$-CO or $R_6O$-CO-$C_{1-2}$-alkyl group wherein $R_6$ is as hereinbefore defined and

$R_{10}$ denotes a hydrogen atom, a methyl or ethyl group,

a piperazino group which is substituted in the 4 position by an $R_6O$-CO-$C_{1-4}$-alkyl, bis- ($R_6O$-CO)-$C_{1-4}$-alkyl, ($R_7O$-PO-$OR_8$)-methyl or ($R_7O$-PO-$R_9$)-methyl group wherein $R_6$ is as hereinbefore defined,

$R_7$ and $R_8$, which may be identical or different, in each case denote a hydrogen atom, a methyl, ethyl, phenyl, benzyl, 5-indanyl or $R_gCO$-O-($R_eCR_f$) group, whilst

$R_e$ to $R_g$ are as hereinbefore defined,

and $R_9$ denotes a methyl or ethyl group,

a piperazino group which is substituted in the 4 position by an $R_6O$-CO-$C_{1-2}$-alkyl group and is additionally substituted at a cyclic carbon atom by an $R_6O$-CO or $R_6O$-CO-$C_{1-2}$-alkyl group wherein $R_6$ is as hereinbefore defined,

a morpholino group which is substituted by an $R_6O$-CO or $R_6O$-CO-$C_{1-2}$-alkyl group, whilst $R_6$ is as hereinbefore defined,

a pyrrolidinyl or piperidinyl group substituted in the 1 position by an $R_6O$-CO-$C_{1-4}$-alkyl, bis-($R_6O$-CO)-$C_{1-4}$-alkyl, ($R_7O$-PO-$OR_8$)-methyl or ($R_7O$-PO-$R_9$)-methyl group wherein $R_6$ to $R_9$ are as hereinbefore defined,

a 2-oxo-morpholino group which may be substituted by 1 or 2 methyl groups,

a 2-oxo-morpholinyl group which is substituted in the 4 position by a methyl, ethyl or $R_6O$-CO-$C_{1-2}$-alkyl group, whilst $R_6$ is as hereinbefore defined and the abovementioned 2-oxo-morpholinyl groups in each case are linked to a carbon atom of the group A, or

a $R_{11}N$($C_{1-2}$-alkyl) group wherein $R_{11}$ denotes a 2-oxo-tetrahydrofuran-3-yl or 2-oxo-tetrahydrofuran-4-yl group, or

C and D together denote a hydrogen atom, a methoxy, ethoxy, 2-methoxy-ethoxy, $C_{4-6}$-cycloalkoxy or $C_{3-6}$-cycloalkyl-$C_{1-3}$-alkoxy group,

particularly those compounds wherein

$R_b$ denotes a phenyl, benzyl or 1-phenylethyl group wherein the phenyl nucleus is substituted in each case by the groups $R_1$ to $R_2$, whilst

$R_1$ and $R_2$, which may be identical or different, each denote a hydrogen, fluorine, chlorine or bromine atom,

or a methyl, trifluoromethyl, methoxy, ethynyl or cyano group,

A and B together denote a hydrogen atom, a methoxy, ethoxy, 2-methoxy-ethoxy, $C_{4-6}$-cycloalkoxy or $C_{3-6}$-cycloalkyl-$C_{1-3}$-alkoxy group,

C denotes an -O-$C_{1-4}$-alkylene or -O-$CH_2$-CH(OH)-$CH_2$ group, whilst the oxygen atom of the abovementioned groups in each case is linked to the bicyclic heteroaromatic ring, and

D denotes an $R_6O$-CO-alkylene-$NR_5$ group wherein the alkylene moiety, which is straight-chained and contains 1 or 2 carbon atoms, may additionally be substituted by an $R_6O$-CO or $R_6O$-CO-methyl group, whilst

$R_5$ denotes a hydrogen atom,

a $C_{1-2}$-alkyl group which may be substituted by an $R_6O$-CO group,

a $C_{2-4}$-alkyl group which is substituted from position 2 by a hydroxy group,

a $C_{3-6}$-cycloalkyl or $C_{3-6}$-cycloalkylmethyl group and

$R_6$ denotes a hydrogen atom,

a $C_{1-6}$-alkyl, cyclopentyl, cyclopentylmethyl, cyclohexyl, cyclohexylmethyl, phenyl, benzyl, 5-indanyl or $R_g$-CO-O-($R_eCR_f$) group, whilst

$R_e$ denotes a hydrogen atom or a $C_{1-4}$-alkyl group,

$R_f$ denotes a hydrogen atom and

$R_g$ denotes a $C_{1-4}$-alkyl, cyclopentyl, cyclohexyl, $C_{1-4}$-alkoxy, cyclopentyloxy or cyclohexyloxy group,

a pyrrolidino or piperidino group which is substituted by an $R_6O$-CO or $R_6O$-CO-$C_{1-2}$-alkyl group wherein $R_6$ is as hereinbefore defined,

a pyrrolidino or piperidino group which is substituted by two $R_6O$-CO or $R_6O$-CO-$C_{1-2}$-alkyl groups wherein $R_6$ is as hereinbefore defined,

a piperazino group which is substituted in the 4 position by the group $R_{10}$ and additionally at a cyclic carbon atom by an $R_6O$-CO or $R_6O$-CO-$C_{1-2}$-alkyl group wherein $R_6$ is as hereinbefore defined and

$R_{10}$ denotes a hydrogen atom, a methyl or ethyl group,

a piperazino group which is substituted in the 4 position by an $R_6O$-CO-$C_{1-4}$-alkyl, bis-($R_6O$-CO)-$C_{1-4}$-alkyl, ($R_7O$-PO-$OR_8$)-methyl or ($R_7O$-PO-$R_9$)-methyl group wherein $R_6$ is as hereinbefore defined,

$R_7$ and $R_8$, which may be identical or different, in each case denote a hydrogen atom, a methyl, ethyl, phenyl, benzyl, 5-indanyl or $R_g$CO-O-($R_eCR_f$) group, whilst

$R_e$ to $R_g$ are as hereinbefore defined,

and $R_9$ denotes a methyl or ethyl group,

a piperazino group which is substituted in the 4 position by an $R_6O$-CO-$C_{1-2}$-alkyl group and is additionally substituted at a cyclic carbon atom by an $R_6O$-CO or $R_6O$-CO-$C_{1-2}$-alkyl group wherein $R_6$ is as hereinbefore defined,

a morpholino group which is substituted by an $R_6O$-CO or $R_6O$-CO-$C_{1-2}$-alkyl group, whilst $R_6$ is as hereinbefore defined,

a pyrrolidinyl or piperidinyl group substituted in the 1 position by an $R_6O$-CO-$C_{1-4}$-alkyl, bis-($R_6O$-CO)-$C_{1-4}$-alkyl, ($R_7O$-PO-$OR_8$)-methyl or ($R_7O$-PO-$R_9$)-methyl group wherein $R_6$ to $R_9$ are as hereinbefore defined,

a 2-oxo-morpholino group which may be substituted by 1 or 2 methyl groups,

a 2-oxo-morpholinyl group which is substituted in the 4 position by a methyl, ethyl or $R_6O$-CO-$C_{1-2}$-alkyl group, whilst $R_6$ is as hereinbefore defined and the abovementioned 2-oxo-morpholinyl groups are in each case linked to a carbon atom of the group C, or

a $R_{11}N$($C_{1-2}$-alkyl) group wherein $R_{11}$ denotes a 2-oxo-tetrahydrofuran-3-yl or 2-oxo-tetrahydrofuran-4-yl group,

the tautomers, stereoisomers and salts thereof.

The most preferred bicyclic heterocyclic compounds of general formula I, however, are those wherein

$R_b$ denotes a phenyl group wherein the phenyl nucleus is substituted in each case by the groups $R_1$ to $R_2$, whilst

$R_1$ and $R_2$, which may be identical or different, each denote a hydrogen, fluorine, chlorine or bromine atom,

A denotes an $-O-C_{1-4}$-alkylene or $-O-CH_2-CH(OH)-CH_2$ group, whilst the oxygen atom of the abovementioned groups in each case is linked to the bicyclic heteroaromatic ring,

B denotes an $R_6O-CO-CH_2-NR_5$ group wherein

$R_5$ denotes a hydrogen atom or a methyl group which may be substituted by an $R_6O-CO$ group, or

a $C_{2-4}$-alkyl group substituted from position 2 onwards by a hydroxy group, and

$R_6$ denotes a hydrogen atom, a methyl or ethyl group,

a pyrrolidino or piperidino group which is substituted by an $R_6O-CO$ group, whilst $R_6$ is as hereinbefore defined,

a piperazino group which is substituted in the 4 position by an $R_6O-CO-CH_2$ or bis-$(R_6O-CO)-C_{1-3}$-alkyl group, whilst $R_6$ is as hereinbefore defined,

a pyrrolidinyl or piperidinyl group substituted in the 1 position by an $R_6O-CO-CH_2$ group, whilst $R_6$ is as hereinbefore defined,

a 2-oxo-morpholino group which may be substituted by one or two methyl groups, or

a $R_{11}N(C_{1-2}$-alkyl) group wherein $R_{11}$ denotes a 2-oxo-tetrahydrofuran-3-yl or 2-oxo-tetrahydrofuran-4-yl group, and

C and D together denote a methoxy, $C_{4-6}$-cycloalkoxy or $C_{3-6}$-cycloalkylmethoxy group,

particular those compounds wherein

$R_b$ denotes a phenyl group wherein the phenyl nucleus is substituted in each case by the groups $R_1$ to $R_2$, whilst

$R_1$ and $R_2$, which may be identical or different, each denote a hydrogen, fluorine, chlorine or bromine atom,

A and B together denote a $C_{4-6}$-cycloalkoxy or $C_{3-6}$-cycloalkylmethoxy group,

C denotes an $-O-CH_2CH_2$ group, whilst the oxygen atom of the abovementioned group is linked to the bicyclic heteroaromatic ring,

D denotes an $R_6O-CO-CH_2-NR_5$ group wherein

$R_5$ denotes a $C_{2-4}$-alkyl group substituted from position 2 onwards by a hydroxy group, and

$R_6$ denotes a methyl or ethyl group,

a 2-oxo-morpholino group which may be substituted by one or two methyl groups, or

a $R_{11}N(C_{1-2}$-alkyl) group wherein $R_{11}$ denotes a 2-oxo-tetrahydrofuran-3-yl or 2-oxo-tetrahydrofuran-4-yl group,

the tautomers, stereoisomers and salts thereof.

The following particularly preferred compounds of general formula I are mentioned by way of example:

(1) 4-(3-chloro-4-fluorophenylamino)-6-{3-[4-(methoxycarbonylmethyl)-1-piperazinyl]propyloxy}-7-methoxy-quinazoline,

(2) 4-[(3-bromophenyl)amino]-6-(2-{4-[(ethoxycarbonyl)methyl]-piperazin-1-yl}ethoxy)-7-methoxy-quinazoline,

(3) (*S*)-4-[(3-bromophenyl)amino]-6-[3-(2-methoxycarbonylpyrrolidin-1-yl)propyloxy]-7-methoxy-quinazoline,

(4) 4-[(3-bromophenyl)amino]-6-(3-{4-[(ethoxycarbonyl)methyl]-piperazin-1-yl}-2-hydroxy-propyloxy)-7-methoxy-quinazoline,

(5) (*S*)-4-[(3-bromophenyl)amino]-6-({1-[(ethoxycarbonyl)methyl]-pyrrolidine-2-yl}methoxy)-7-methoxy-quinazoline and

(6) 4-[(3-bromophenyl)amino]-6-(2-{4-[1,2 bis (methoxycarbonyl)ethyl]-piperazin-1-yl}ethoxy)-7-methoxy-quinazoline

and the salts thereof.

The compounds of general formula I may, for example, be prepared by the following methods:

a) reacting a compound of general formula

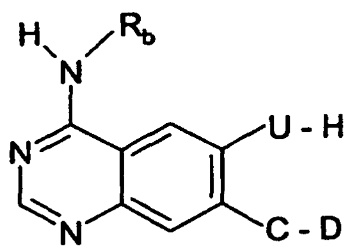

, (II)

wherein
$R_b$, C and D are as hereinbefore defined and
U denotes an oxygen atom or an $R_4N$ group, whilst $R_4$ is as hereinbefore defined, with a compound of general formula

$$Z_1 - A' - B , \quad (III)$$

wherein
B is as hereinbefore defined,
A' denotes one of the optionally substituted alkylene, cycloalkylene, alkylene-cycloalkylene, cycloalkylene-alkylene or alkylene-cycloalkylene-alkylene moieties mentioned above for the group A, which are linked to the heteroaromatic group via an oxygen atom or via an $NR_4$ group, and
$Z_1$ denotes a leaving group such as a halogen atom or a sulphonyloxy group such as a chlorine or bromine atom, a methanesulphonyloxy or p-toluenesulphonyloxy group.

The reaction is optionally carried out in a solvent or mixture of solvents such as methylene chloride, dimethylformamide, dimethylsulphoxide, sulpholane, benzene, toluene, chlorobenzene, tetrahydrofuran, benzene/tetrahydrofuran or dioxane conveniently in the presence of a tertiary organic base such as triethylamine, pyridine or 2-dimethylaminopyridine, in the presence of N-ethyl-diisopropylamine (Hünig's base), whilst these organic bases may simultaneously serve as solvents, or in the presence of an inorganic base such as sodium carbonate, potassium carbonate or sodium hydroxide solution conveniently at temperatures between -20 and 200°C, preferably at temperatures between 0 and 150°C.

b) reacting a compound of general formula

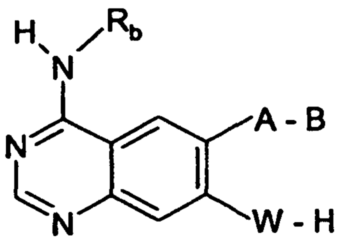

, (IV)

wherein
$R_b$, A and B are as hereinbefore defined and
W denotes an oxygen atom or an $R_4N$ group, whilst $R_4$ is as hereinbefore defined, with a compound of general formula

$$Z_2 - C' - D , \qquad (V)$$

wherein
D is as hereinbefore defined,
C' denotes one of the optionally substituted alkylene, cycloalkylene, alkylene-cycloalkylene, cycloalkylene-alkylene or alkylene-cycloalkylene-alkylene moieties mentioned above for the group C, which are linked to the heteroaromatic group via an oxygen atom or via an $NR_4$ group, and
$Z_2$ denotes a leaving group such as a halogen atom or a sulphonyloxy group such as a chlorine or bromine atom, a methanesulphonyloxy or p-toluenesulphonyloxy group.

The reaction is optionally carried out in a solvent or mixture of solvents such as methylene chloride, dimethylformamide, dimethylsulphoxide, sulpholane, benzene, toluene, chlorobenzene, tetrahydrofuran, benzene/tetrahydrofuran or dioxane conveniently in the presence of a tertiary organic base such as triethylamine, pyridine or 2-dimethylaminopyridine, in the presence of N-ethyl-diisopropylamine (Hünig's base), whilst these organic bases may simultaneously serve as solvents, or in the presence of an inorganic base such as sodium carbonate, potassium carbonate or sodium hydroxide solution or in the presence of an alkali or alkaline earth metal alkoxide such as sodium ethoxide or potassium tert.butoxide conveniently at temperatures between -20 and 200°C, preferably at temperatures between 0 and 150°C.

c) In order to prepare a compound of general formula I wherein A is as hereinbefore defined with the exception of the oxygen atom and the $-NR_4$ group:
reacting a compound of general formula

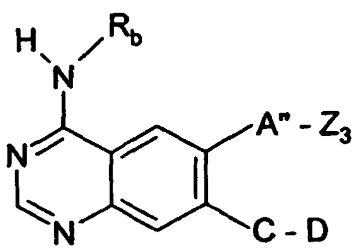

, (VI)

wherein
$R_b$, C and D are as hereinbefore defined and
A" has the meanings given for A hereinbefore with the exception of the oxygen atom and the $-NR_4$ group and $Z_3$ denotes a leaving group such as a halogen atom or a sulphonyloxy group such as a chlorine or bromine atom, a methanesulphonyloxy or p-toluenesulphonyloxy group or together with a hydrogen atom of an adjacent hydrocarbon group denotes an oxygen atom, with a compound of general formula

H - B , (VII)

wherein
B is as hereinbefore defined.

The reaction is optionally carried out in a solvent or mixture of solvents such as acetonitrile, ethanol, methylene chloride, dimethylformamide, dimethylsulphoxide, sulpholane, benzene, toluene, chlorobenzene, tetrahydrofuran, benzene/tetrahydrofuran or dioxane, optionally in the presence of a tertiary organic base such as triethylamine, pyridine or 2-dimethylaminopyridine, in the presence of N-ethyl-diisopropylamine (Hünig's base), whilst these organic bases may simultaneously serve as solvents, or in the presence of an inorganic base such as sodium carbonate, potassium carbonate or sodium hydroxide solution or in the presence of an alkali or alkaline earth metal alkoxide such as sodium ethoxide or potassium tert.butoxide, conveniently at temperatures between -20 and 200°C, preferably at temperatures between 0 and 150°C.

d) In order to prepare a compound of general formula I wherein C is as hereinbefore defined with the exception of the oxygen atom and the $-NR_4$ group:
reacting a compound of general formula

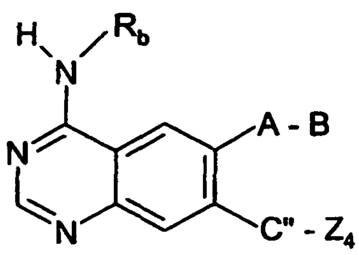

, (VIII)

wherein
$R_b$, A and B are as hereinbefore defined and
C" has the meanings given for C hereinbefore with the exception of the oxygen atom and the $-NR_4$ group and $Z_4$ denotes a leaving group such as a halogen atom or a sulphonyloxy group such as a chlorine or bromine atom, a methanesulphonyloxy or p-toluenesulphonyloxy group or together with a hydrogen atom of an adjacent hydrocarbon group denotes an oxygen atom, with a compound of general formula

H - D , (IX)

wherein
D is as hereinbefore defined.

The reaction is optionally carried out in a solvent or mixture of solvents such as acetonitrile, ethanol, methylene chloride, dimethylformamide, dimethylsulphoxide, sulpholane, benzene, toluene, chlorobenzene, tetrahydrofuran, benzene/tetrahydrofuran or dioxane optionally in the presence of a tertiary organic base such as triethylamine, pyridine or 2-dimethylaminopyridine, in the presence of N-ethyl-diisopropylamine (Hünig's base), whilst these organic bases may simultaneously serve as solvents, or in the presence of an inorganic base such as sodium carbonate, potassium carbonate or sodium hydroxide solution or in the presence of an alkali or alkaline earth metal alkoxide such as sodium ethoxide or potassium tert.butoxide, conveniently at temperatures between -20 and 200°C, preferably at temperatures between 0 and 150°C.

e) In order to prepare a compound of general formula I wherein B denotes an $R_6O$-CO-alkylene-$NR_5$ group wherein the alkylene moiety, which is straight-chained and contains 1 to 6 carbon atoms, may additionally be substituted by one or two $C_{1-2}$-alkyl groups or by an $R_6O$-CO or $R_6O$-CO-$C_{1-2}$-alkyl group, a piperazino or homopiperazino group substituted in the 4 position by an $R_6O$-CO-$C_{1-4}$-alkyl or bis-($R_6O$-CO)-$C_{1-4}$-alkyl group or a pyrrolidinyl, piperidinyl or hexahydroazepinyl group substituted in the 1 position by an $R_6O$-CO-$C_{1-4}$-alkyl or bis-($R_6O$-CO)-$C_{1-4}$-alkyl group, whilst in each case $R_5$ and $R_6$ are as hereinbefore defined:
reacting a compound of general formula

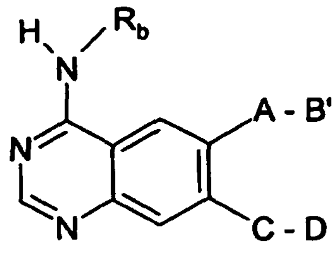

, (X)

wherein

$R_b$, A, C and D are as hereinbefore defined and

B' denotes an $R_5NH$ group wherein $R_5$ is as hereinbefore defined, a piperazino or homopiperazino group unsubstituted in the 4 position, a pyrrolidinyl, piperidinyl or hexahydroazepinyl group unsubstituted in the 1 position, with a compound of general formula

$$R_6O\text{-}CO\text{-alkylene-}Z_5 , \qquad (XI)$$

wherein

the alkylene moiety, which is straight-chained and contains 1 to 6 carbon atoms, may additionally be substituted by one or two $C_{1-2}$-alkyl groups or by an $R_6O$-CO or $R_6O$-CO-$C_{1-2}$-alkyl group, whilst $R_6$ in each case is as hereinbefore defined, and

$Z_5$ denotes an exchangeable group such as a halogen atom or a substituted sulphonyloxy group, e.g. a chlorine or bromine atom, a methylsulphonyloxy, propylsulphonyloxy, phenylsulphonyloxy or benzylsulphonyloxy group.

The reaction is optionally carried out in a solvent or mixture of solvents such as acetonitrile, methylene chloride, dimethylformamide, dimethylsulphoxide, sulpholane, benzene, toluene, chlorobenzene, tetrahydrofuran, benzene/tetrahydrofuran or dioxane conveniently in the presence of a tertiary organic base such as triethylamine or N-ethyl-diisopropylamine (Hünig's base), whilst these organic bases may simultaneously serve as solvents, or in the presence of an inorganic base such as sodium carbonate, potassium carbonate or sodium hydroxide solution conveniently at temperatures between -20 and 200°C, preferably at temperatures between 0 and 150°C.

f) In order to prepare a compound of general formula I wherein D denotes an $R_6O$-CO-alkylene-$NR_5$ group wherein the alkylene moiety, which is straight-chained and contains 1 to 6 carbon atoms, may additionally be substituted by one or two $C_{1-2}$-alkyl groups or by an $R_6O$-CO or $R_6O$-CO-$C_{1-2}$-alkyl group, a piperazino or homopiperazino group substituted in the 4 position by an $R_6O$-CO-$C_{1-4}$-alkyl or bis-($R_6O$-CO)-$C_{1-4}$-alkyl group or a pyrrolidinyl, piperidinyl or hexahydroazepinyl group substituted in the 1 position by an $R_6O$-CO-$C_{1-4}$-alkyl or bis-($R_6O$-CO)-$C_{1-4}$-alkyl group, whilst in each case $R_5$ and $R_6$ are as hereinbefore defined:

reacting a compound of general formula

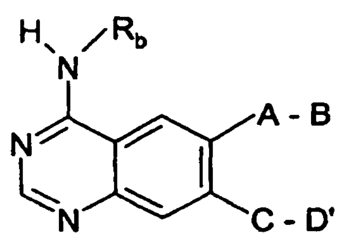

, (XII)

wherein

$R_b$, A, B and C are as hereinbefore defined and

D' denotes an $R_5NH$ group wherein $R_5$ is as hereinbefore defined, a piperazino or homopiperazino group unsubstituted in the 4 position, a pyrrolidinyl, piperidinyl or hexahydroazepinyl group unsubstituted in the 1 position, with a compound of general formula

$$R_6O\text{-}CO\text{-}alkylene\text{-}Z_5\ ,\qquad (XI)$$

wherein
the alkylene moiety, which is straight-chained and contains 1 to 6 carbon atoms, may additionally be substituted by one or two $C_{1-2}$-alkyl groups or by an $R_6O$-CO or $R_6O$-CO-$C_{1-2}$-alkyl group, whilst $R_6$ in each case is as hereinbefore defined, and
$Z_5$ denotes an exchangeable group such as a halogen atom or a substituted sulphonyloxy group, e.g. a chlorine or bromine atom, a methylsulphonyloxy, propylsulphonyloxy, phenylsulphonyloxy or benzylsulphonyloxy group.

The reaction is optionally carried out in a solvent or mixture of solvents such as acetonitrile, methylene chloride, dimethylformamide, dimethylsulphoxide, sulpholane, benzene, toluene, chlorobenzene, tetrahydrofuran, benzene/tetrahydrofuran or dioxane conveniently in the presence of a tertiary organic base such as triethylamine or N-ethyldiisopropylamine (Hünig's base), whilst these organic bases may simultaneously serve as solvents, or in the presence of an inorganic base such as sodium carbonate, potassium carbonate or sodium hydroxide solution conveniently at temperatures between -20 and 200°C, preferably at temperatures between 0 and 150°C.

g) In order to prepare a compound of general formula I wherein at least one of the groups $R_6$ to $R_8$ denotes a hydrogen atom:
Converting a compound of general formula

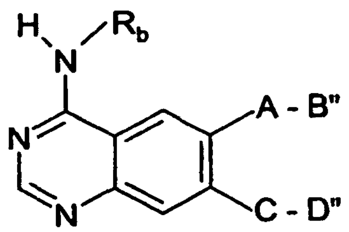

, (XIII)

wherein
$R_a$ to $R_d$, A, C and X are as hereinbefore defined,
B" and D" have the meanings given for B and D hereinbefore, with the proviso that at least one of the groups B" or D" contains an $R_6O$-CO, ($R_7O$-PO-$OR_8$) or ($R_7O$-PO-$R_9$) group wherein $R_9$ is as hereinbefore defined and at least one of the groups $R_6$ to $R_8$ does not represent a hydrogen atom, by hydrolysis, treating with acids, thermolysis or hydrogenolysis, into a compound of general formula I wherein at least one of the groups $R_6$ to $R_8$ denotes a hydrogen atom.

The hydrolysis is conveniently carried out either in the presence of an acid such as hydrochloric acid, sulphuric acid, phosphoric acid, acetic acid, trichloroacetic acid, trifluoroacetic acid or mixtures thereof or in the presence of a base such as lithium hydroxide, sodium hydroxide or potassium hydroxide in a suitable solvent such as water, water/methanol, water/ethanol, water/isopropanol, methanol, ethanol, water/tetrahydrofuran or water/dioxane at temperatures between -10 and 120°C, e.g. at temperatures between ambient temperature and the boiling temperature of the reaction mixture.

If B" or D" in a compound of formula X for example contains the tert.butyloxycarbonyl group, the tert.butyl group may also be cleaved by treating with an acid such as trifluoroacetic acid, formic acid, p-toluenesulphonic acid, sulphuric acid, hydrochloric acid, phosphoric acid or polyphosphoric acid optionally in an inert solvent such as methylene chloride, chloroform, benzene, toluene, diethylether, tetrahydrofuran or dioxane preferably at temperatures between -10 and 120°C, e.g. at temperatures between 0 and 60°C, or thermally, optionally in an inert solvent such as methylene chloride, chloroform, benzene, toluene, tetrahydrofuran or dioxane and preferably in the presence of a catalytic amount of an acid such as p-toluenesulphonic acid, sulphuric acid, phosphoric acid or polyphosphoric acid preferably at the boiling temperature of the solvent used, e.g. at temperatures between 40 and 120°C. Under the reaction conditions mentioned above, any N-tert.butyloxycarbonylamino or N-tert.butyloxycarbonylimino groups present may be converted into the corresponding amino or imino groups.

If B" or D" in a compound of formula X for example contains the benzyloxycarbonyl group, the benzyl group may also be cleaved hydrogenolytically in the presence of a hydrogenation catalyst such as palladium/charcoal in a suitable solvent such as methanol, ethanol, ethanol/water, glacial acetic acid, ethyl acetate, dioxane or dimethylformamide, preferably at temperatures between 0 and 50°C, e.g. ambient temperature, and at a hydrogen pressure of 1 to 5 bar. During the hydrogenolysis other groups may simultaneously be converted, e.g. a nitro group may be converted into an amino group, a benzyloxy group into a hydroxy group and a N-benzylamino, N-benzylimino, N-benzyloxycarbonylamino or N-benzyloxycarbonylimino group into a corresponding amino or imino group.

If according to the invention a compound of general formula I is obtained which contains a carboxy or hydroxyphosphoryl group, this may be converted by esterification into a corresponding ester of general formula I or

If a compound of general formula I is obtained wherein B or D denotes an optionally substituted N-(2-hydroxyethyl)-glycine or N-(2-hydroxyethyl)-glycinester group, this may be converted by cyclisation in a corresponding 2-oxo-morpholino compound.

The subsequent esterification is optionally carried out in a solvent or mixture of solvents such as methylene chloride, dimethylformamide, benzene, toluene, chlorobenzene, tetrahydrofuran, benzene/tetrahydrofuran or dioxane or particularly advantageously in a corresponding alcohol, optionally in the presence of an acid such as hydrochloric acid or in the presence of a dehydrating agent, e.g. in the presence of isobutyl chloroformate, thionylchloride, trimethylchlorosilane, sulphuric acid, methanesulphonic acid, p-toluenesulphonic acid, phosphorus trichloride, phosphorus pentoxide, N,N'-dicyclohexylcarbodiimide, N,N'-dicyclohexylcarbodiimide/N-hydroxysuccinimide or 1-hydroxy-benzotriazole and optionally additionally in the presence of 4-dimethylamino-pyridine, N,N'-carbonyldiimidazole or triphenylphosphine/carbon tetrachloride, conveniently at temperatures between 0 and 150°C, preferably at temperatures between 0 and 80°C.

The subsequent ester formation may also be carried out by reacting a compound which contains a carboxy or hydroxyphosphoryl group with a corresponding alkyl halide.

The subsequent intramolecular cyclisation is optionally carried out in a solvent or mixture of solvents such as acetonitrile, methylene chloride, tetrahydrofuran, dioxane or toluene in the presence an acid such as hydrochloric acid or p-toluenesulphonic acid at temperatures between -10 and 120°C.

In the reactions described hereinbefore, any reactive groups present such as hydroxy, carboxy, phosphono, O-alkyl-phosphono, amino, alkylamino or imino groups may be protected during the reaction by conventional protecting groups which are cleaved again after the reaction.

For example, a protecting group for a hydroxy group may be a trimethylsilyl, acetyl, benzoyl, methyl, ethyl, tert.butyl, trityl, benzyl or tetrahydropyranyl group,

protecting groups for a carboxy group may be a trimethylsilyl, methyl, ethyl, tert.butyl, benzyl or tetrahydropyranyl group,

protecting groups for a phosphono group may be an alkyl group such as the methyl, ethyl, isopropyl or n-butyl group, the phenyl or benzyl group, and

protecting groups for an amino, alkylamino or imino group may be a formyl, acetyl, trifluoroacetyl, ethoxycarbonyl, tert. butoxycarbonyl, benzyloxycarbonyl, benzyl, methoxybenzyl or 2,4-dimethoxybenzyl group and additionally, for the amino group, a phthalyl group.

Any protecting group used is optionally subsequently cleaved for example by hydrolysis in an aqueous solvent, e.g. in water, isopropanol/water, acetic acid/water, tetrahydrofuran/water or dioxane/water, in the presence of an acid such as trifluoroacetic acid, hydrochloric acid or sulphuric acid or in the presence of an alkali metal base such as sodium hydroxide or potassium hydroxide or aprotically, e.g. in the presence of iodotrimethylsilane, at temperatures between 0 and 120°C, preferably at temperatures between 10 and 100°C.

However, a benzyl, methoxybenzyl or benzyloxycarbonyl group is cleaved, for example, hydrogenolytically, e.g. with hydrogen in the presence of a catalyst such as palladium/charcoal in a suitable solvent such as methanol, ethanol, ethyl acetate or glacial acetic acid, optionally with the addition of an acid such as hydrochloric acid at temperatures between 0 and 100°C, but preferably at temperatures between 20 and 60°C, and at a hydrogen pressure of 1 to 7 bar, but preferably 3 to 5 bar. A 2,4-dimethoxybenzyl group, however, is preferably cleaved in trifluoroacetic acid in the presence of anisol.

A tert.butyl or tert.butyloxycarbonyl group is preferably cleaved by treating with an acid such as trifluoroacetic acid or hydrochloric acid or by treating with iodotrimethylsilane optionally using a solvent such as methylene chloride, dioxane, methanol or diethylether.

A trifluoroacetyl group is preferably cleaved by treating with an acid such as hydrochloric acid, optionally in the presence of a solvent such as acetic acid at temperatures between 50 and 120°C or by treating with sodium hydroxide solution optionally in the presence of a solvent such as tetrahydrofuran at temperatures between 0 and 50°C.

A phthalyl group is preferably cleaved in the presence of hydrazine or a primary amine such as methylamine, ethylamine or n-butylamine in a solvent such as methanol, ethanol, isopropanol, toluene/water or dioxane at temperatures between 20 and 50°C.

A single alkyl group may be cleaved from an O,O'-dialkylphosphono group with sodium iodide, for example, in a solvent such as acetone, methylethylketone, acetonitrile or dimethylformamide at temperatures between 40 and 150°C, but preferably at temperatures between 60 and 100°C.

Both alkyl groups may be cleaved from an O,O'-dialkyl-phosphono group with iodotrimethylsilane, bromotrimethylsilane or chlorotrimethylsilane/sodium iodide, for example, in a solvent such as methylene chloride, chloroform or acetonitrile at temperatures between 0°C and the boiling temperature of the reaction mixture, but preferably at temperatures between 20 and 60°C.

Moreover, the compounds of general formula I obtained may be resolved into their enantiomers and/or diastereomers, as mentioned hereinbefore. Thus, for example, cis/trans mixtures may be resolved into their cis and trans isomers, and compounds with at least one optically active carbon atom may be separated into their enantiomers.

Thus, for example, the cis/trans mixtures may be resolved by chromatography into the cis and trans isomers thereof, the compounds of general formula I obtained which occur as racemates may be separated by methods known per se (cf. Allinger N. L. and Eliel E. L. in "Topics in Stereochemistry", Vol. 6, Wiley Interscience, 1971) into their optical antipodes and compounds of general formula I with at least 2 asymmetric carbon atoms may be resolved into their diastereomers on the basis of their physical-chemical differences using methods known per se, e.g. by chromatography and/or fractional crystallisation, and, if these compounds are obtained in racemic form, they may subsequently be resolved into the enantiomers as mentioned above.

The enantiomers are preferably separated by column separation on chiral phases or by recrystallisation from an optically active solvent or by reacting with an optically active substance which forms salts or derivatives such as e.g. esters or amides with the racemic compound, particularly acids and the activated derivatives or alcohols thereof, and separating the diastereomeric mixture of salts or derivatives thus obtained, e.g. on the basis of their differences in solubility, whilst the free antipodes may be released from the pure diastereomeric salts or derivatives by the action of suitable agents. Optically active acids in common use are e.g. the D- and L-forms of tartaric acid or dibenzoyltartaric acid, di-o-tolyltartaric acid, malic acid, mandelic acid, camphorsulphonic acid, glutamic acid, aspartic acid or quinic acid. An optically active alcohol may be for example (+) or (-)-menthol and an optically active acyl group in amides, for example, may be a (+)-or (-)-menthyloxycarbonyl.

Furthermore, the compounds of formula I may be converted into the salts thereof, particularly for pharmaceutical use into the physiologically acceptable salts with inorganic or organic acids. Acids which may be used for this purpose include for example hydrochloric acid, hydrobromic acid, sulphuric acid, phosphoric acid, fumaric acid, succinic acid, lactic acid, citric acid, tartaric acid or maleic acid.

Moreover, if the new compounds of formula I thus obtained contain a carboxy, hydroxyphosphoryl, sulpho or 5-tetrazolyl group, they may subsequently, if desired, be converted into the salts thereof with inorganic or organic bases, particularly for pharmaceutical use into the physiologically acceptable salts thereof. Suitable bases for this purpose include for example sodium hydroxide, potassium hydroxide, arginine, cyclohexylamine, ethanolamine, diethanolamine and triethanolamine.

The compounds of general formulae II to XIII used as starting materials are known from the literature in some cases or may be obtained by methods known from the literature (cf. Examples I to XVI).

As already mentioned hereinbefore, the compounds of general formula I according to the invention and their physiologically acceptable salts have valuable pharmacological properties, particularly an inhibiting effect on signal transduction mediated by the Epidermal Growth Factor receptor (EGF-R), whilst this may be achieved for example by inhibiting ligand bonding, receptor dimerisation or tyrosine kinase itself. It is also possible to block the transmission of signals to components located further down.

The biological properties of the new compounds were investigated as follows:

The inhibition of the EGF-R-mediated signal transmission can be demonstrated e.g. with cells which express human EGF-R and whose survival and proliferation depend on stimulation by EGF or TGF-alpha. A cell line of murine origin dependent on interleukin-3-(IL-3) which was genetically modified to express functional human EGF-R was used here. The proliferation of these cells known as F/L-HERc can therefore be stimulated either by murine IL-3 or by EGF (cf. von Rüden, T. et al. in EMBO J. 7, 2749-2756 (1988) and Pierce, J. H. et al. in Science 239, 628-631 (1988)).

The starting material used for the F/L-HERc cells was the cell line FDC-$P_1$, the production of which has been described by Dexter, T. M. et al. in J. Exp. Med. 152, 1036-1047 (1980). Alternatively, however, other growth-factor-dependent cells may also be used (cf. for example Pierce, J. H. et al. in Science 239, 628-631 (1988), Shibuya, H. et al. in Cell 70, 57-67 (1992) and Alexander, W. S. et al. in EMBO J. 10, 3683-3691 (1991)). For expressing the human EGF-R cDNA (cf. Ullrich, A. et al. in Nature 309, 418-425 (1984)) recombinant retroviruses were used as described by von Rüden, T. et al., EMBO J. 7, 2749-2756 (1988), except that the retroviral vector LXSN (cf. Miller, A. D. et al. in BioTechniques 7, 980-990 (1989)) was used for the expression of the EGF-R cDNA and the line GP+E86 (cf. Markowitz, D. et al. in J. Virol. 62, 1120-1124 (1988)) was used as the packaging cell.

The test was performed as follows:

F/L-HERc cells were cultivated in RPMI/1640 medium (BioWhittaker), supplemented with 10 % foetal calf serum (FCS, Boehringer Mannheim), 2 mM glutamine (BioWhittaker), standard antibiotics and 20 ng/ml of human EGF (Promega), at 37°C and 5% $CO_2$. In order to investigate the inhibitory activity of the compounds according to the invention, 1.5 x $10^4$ cells per well were cultivated in triplicate in 96-well dishes in the above medium (200 µl), the cell proliferation being stimulated with either EGF (20 ng/ml) or murine IL-3. The IL-3 used was obtained from culture

supernatants of the cell line X63/0 mIL-3 (cf. Karasuyama, H. et al. in Eur. J. Immunol. 18, 97-104 (1988)). The compounds according to the invention were dissolved in 100% dimethylsulphoxide (DMSO) and added to the cultures in various dilutions, the maximum DMSO concentration being 1%. The cultures were incubated for 48 hours at 37°C.

In order to determine the inhibitory activity of the compounds according to the invention the relative cell number was measured in O.D. units using the Cell Titer 96™ $AQ_{ueous}$ NonRadioactive Cell Proliferation Assay (Promega). The relative cell number was calculated as a percentage of the control (F/LHERc cells without inhibitor) and the concentration of active substance which inhibits the proliferation of the cells by 50% ($IC_{50}$) was derived therefrom. The following results were obtained:

| Compound (Example no.) | Inhibition of EGF-dependent proliferation $IC_{50}$ [nM] |
|---|---|
| 1 | 46 |
| 1(2) | 20 |
| 2 | 230 |
| 2(1) | 39 |
| 3 | 45 |
| 3(1) | 100 |
| 3(2) | 70 |
| 3(4) | 77 |
| 4 | 33 |

The compounds of general formula I according to the invention thus inhibit the signal transduction by tyrosine kinases, as demonstrated by the example of the human EGF receptor, and are therefore useful for treating pathophysiological processes caused by hyperfunction of tyrosinekinases. These are e.g. benign or malignant tumours, particularly tumours of epithelial and neuroepithelial origin, metastasisation and the abnormal proliferation of vascular endothelial cells (neoangiogenesis).

The compounds according to the invention are also useful for preventing and treating diseases of the airways and lungs which are accompanied by increased or altered production of mucus caused by stimulation by tyrosinekinases, e.g. in inflammatory diseases of the airways such as chronic bronchitis, chronic obstructive bronchitis, asthma, bronchiectasias, allergic or non-allergic rhinitis or sinusitis, cystic fibrosis, $\alpha$1-antitrypsin deficiency, or coughs, pulmonary emphysema, pulmonary fibrosis and hyperreactive airways.

The compounds are also suitable for treating diseases of the gastrointestinal tract and bile duct and gall bladder which are associated with disrupted activity of the tyrosinekinases, such as may be found e.g. in chronic inflammatory changes such as cholecystitis, Crohn's disease, ulcerative colitis, and ulcers in the gastrointestinal tract or such as may occur in diseases of the gastrointestinal tract which are associated with increased secretions, such as Ménétrier's disease, secreting adenomas and protein loss syndrome, and also for treating nasal polyps and polyps of the gastrointestinal tract of various origins such as e.g. villous or adenomatous polyps of the large bowel, but also polyps in familial polyposis coli, intestinal polyps in Gardner's syndrome, polyps throughout the entire gastrointestinal tract in Peutz-Jeghers syndrome, in inflammatory pseudopolyps, juvenile polyps, Colitis cystica profunda and Pneumatosis cystoides intestinales.

Moreover, the compounds of general formula I and the physiologically acceptable salts thereof may be used to treat kidney diseases, particularly in cystic changes such as cystic kidneys, for treating renal cysts which may be idiopathic in origin or occur in syndromes such as e.g. tuberculous sclerosis, in von-Hippel-Lindau Syndrome, in nephronophthisis and spongy kidney and other diseases caused by aberrant function of tyrosinekinases, such as e.g. epidermal hyperproliferation (psoriasis), inflammatory processes, diseases of the immune system, hyperproliferation of haematopoietic cells, etc.

By reason of their biological properties the compounds according to the invention may be used on their own or in conjunction with other pharmacologically active compounds, for example in tumour therapy, in monotherapy or in conjunction with other anti-tumour therapeutic agents, for example in combination with topoisornerase inhibitors (e. g. etoposide), mitosis inhibitors (e.g. vinblastin), compounds which interact with nucleic acids (e.g. cis-platin, cyclophosphamide, adriamycin), hormone antagonists (e.g. tamoxifen), inhibitors of metabolic processes (e.g. 5-FU etc.), cytokines (e.g. interferons), antibodies, etc. For treating respiratory tract diseases, these compounds may be used on their own or in conjunction with other therapeutic agents for the airways, such as substances with a secretolytic, broncholytic and/or antiinflammatory activity. For treating diseases in the region of the gastrointestinal tract, these compounds may also be administered on their own or in conjunction with substances having an effect on motility or secretion or antiinflammatory substances. These combinations may be administered either simultaneously or sequentially.

These compounds may be administered either on their own or in conjunction with other active substances by

intravenous, subcutaneous, intramuscular, intrarectal, intraperitoneal or intranasal route, by inhalation or transdermally or orally, whilst aerosol formulations are particularly suitable for inhalation.

For pharmaceutical use the compounds according to the invention are generally used for warm-blooded vertebrates, particularly humans, in doses of 0.01-100 mg/kg of body weight, preferably 0.1-15 mg/kg. For administration they are formulated with one or more conventional inert carriers and/or diluents, e.g. with corn starch, lactose, glucose, microcrystalline cellulose, magnesium stearate, polyvinylpyrrolidone, citric acid, tartaric acid, water, water/ethanol, water/glycerol, water/sorbitol, water/polyethyleneglycol, propyleneglycol, stearylalcohol, carboxymethylcellulose or fatty substances such as hard fat or suitable mixtures thereof in conventional galenic preparations such as plain or coated tablets, capsules, powders, suspensions, solutions, sprays or suppositories.

The following Examples are intended to illustrate the present invention without restricting it:

Preparation of the starting compounds:

Example I

4-(3-chloro-4-fluorophenylamino)-6-[3-(4-tert.butyloxycarbonylpiperazino)-propyloxy]-7-methoxy-quinazoline

500 mg of 4-(3-chloro-4-fluorophenylamino)-6-hydroxy-7-methoxy-quinazoline, 600 mg of 1-[3-(methanesulphonyloxy)propyl]-4-tert.butyloxycarbonyl-piperazine (prepared by reacting 1-(3-hydroxypropyl)-4-tert.butyloxycarbonyl-piperazine with methanesulphonic acid anhydride in the presence of triethylamine) and 520 mg of potassium carbonate are stirred in 20 ml of dimethylformamide for 8 hours at 80°C. A further 300 mg of the piperazino compound are added and stirring is continued for another 4 hours at 80°C. The reaction mixture is concentrated by evaporation and the residue is divided between water and ethyl acetate. The organic phase is concentrated by evaporation and the residue is purified by chromatography on a silica gel column with ethyl acetate.
Yield: 700 mg of (82 % of theory),
$R_f$ value: 0.29 (silica gel; ethyl acetate/methanol = 9:1)
Mass spectrum: (M-H) = 544, 546

The following compounds are obtained analogously to Example I:

(1) 4-(3-chloro-4-fluorophenylamino)-6-[3-(1-tert.butyloxycarbonyl-4-piperidinyl)-propyloxy]-7-methoxy-quinazoline $R_f$ value: 0.70 (silica gel; ethyl acetate/methanol = 9:1)

(2) (*S*)-4-[(3-bromophenyl)amino]-6-{[1- (tert.butyloxycarbonyl)-pyrrolidine-2-yl]methoxy}-7-methoxy-quinazoline
melting point: 178°C
Mass spectrum ($ESI^-$): m/z = 527, 529 $[M-H]^-$

(3) (*R*)-4-[(3-bromophenyl)amino]-6- {[1-(tert.butyloxycarbonyl)-pyrrolidine-2-yl]methoxy}-7-methoxy-quinazoline
$R_f$ value: 0.65 (silica gel, ethyl acetate/methanol = 9:1)
Mass spectrum (EI): m/z = 528, 530 $[M]^+$

(4) (*S*) -4-[(3-chloro-4-fluoro-phenyl)amino]-6-{[1-(tert.butyloxycarbonyl)-pyrrolidin-2-yl]methoxy}-7-cyclopentyloxy-quinazoline
$R_f$ value: 0.76 (silica gel, methylene chloride/methanol/concentrated aqueous ammonia = 90:10:0.1)
Mass spectrum ($ESI^-$): m/z = 555, 557 $[M-H]^-$

(5) (*S*)-4-[(3-chloro-4-fluoro-phenyl)amino]-6-{[1-(tert.butyloxycarbonyl)-pyrrolidin-2-yl]methoxy}-7-cyclopentylmethoxyquinazoline
Melting point: 210-211.5°C
Mass spectrum ($ESI^-$): m/z = 569, 571 $[M-H]^-$

Example II

4-(3-chloro-4-fluorophenylamino)-6-[3-(1-piperazinyl)-propyloxy]-7-methoxy-quinazoline

600 mg of 4-(3-chloro-4-fluorophenylamino)-6-[3-(4-tert.butyloxycarbonylpiperazino)propyloxy]-7-methoxyquinazoline in 5 ml methylene chloride are mixed with 1.5 ml of trifluoroacetic acid and stirred for 2 hours at ambient temperature. The reaction mixture is concentrated by evaporation and combined with 2N NaOH. It is decanted off the sticky residue, the residue is taken up in methanol, concentrated by evaporation and triturated with diethyl ether.

Yield: 280 mg of (50 % of theory),
$R_f$ value: 0.49 (aluminium oxide; ethyl acetate/methanol/conc. aqueous ammonia = 9:1:0.1)
Mass spectrum: $(M+H)^+$ = 446, 448

The following compounds are obtained analogously to Example II:

(1) 4- (3-chloro-4-fluorophenylamino)-6- [3-(4-piperidinyl)propyloxy]-7-methoxy-quinazoline
$R_f$ value: 0.33 (aluminium oxide; ethyl acetate/methanol/conc. aqueous ammonia = 9:1:0.1)
Mass spectrum: $(M+H)^+$ = 445, 447

(2) (*S*)-4-[(3-bromophenyl)amino]-6-[(pyrrolidine-2-yl)methoxy]-7-methoxy-quinazoline
melting point: 143°C
Mass spectrum ($ESI^+$): m/z = 429, 431 $[M+H]^+$

(3) (*R*)-4- [(3-bromophenyl)amino]-6-[(pyrrolidine-2-yl)methoxy]-7-methoxy-quinazoline
$R_f$ value: 0.21 (silica gel, ethyl acetate/methanol/concentrated aqueous ammonia solution = 9:1:0.1)

(4) (*S*)-4-[(3-chloro-4-fluoro-phenyl)amino]-6-[(pyrrolidin-2-yl)methoxy]-7-cyclopentyloxy-quinazoline
$R_f$ value: 0.18 (silica gel, methylene chloride/methanol/concentrated aqueous ammonia = 90:10:0.1)
Mass spectrum ($ESI^-$): m/z = 455, 457 $[M-H]^-$

(5) (*S*)-4-[(3-chloro-4-fluoro-phenyl)amino]-6-[(pyrrolidin-2-yl)methoxy]-7-cyclopentylmethoxy-quinazoline
$R_f$ value: 0.36 (silica gel, methylene chloride/methanol/concentrated aqueous ammonia = 90:10:0.1)
Mass spectrum ($ESI^+$): m/z = 471, 473 $[M+H]^+$

## Example III

### N-(3-Brompropyl)sarcosine ethyl ester and N-(3-chloropropyl)-sarcosine ethyl ester

6.9 ml of 1,3-dibromopropene in 20 ml acetonitrile are added dropwise to 2.4 g of sarcosine ethyl ester hydrochloride and 6 ml of N-ethyl-diisopropylamine in 50 ml of acetonitrile. After stirring overnight at ambient temperature the mixture is concentrated by evaporation and the residue is divided between ethyl acetate and water. The organic phase is concentrated by evaporation and the residue is purified by chromatography on silica gel (ethyl acetate/methanol = 9:1).
Yield: 0.77 g,
$R_f$ value: 0.80 (silica gel; ethyl acetate/methanol = 9:1)
Mass spectrum: $M^+$ = 237, 239 and 193, 195

The following compounds are obtained analogously to Example III:

(1) (*S*)-N-(3-Bromopropyl)proline methyl ester and (*S*)-N-(3-chloropropyl)proline methyl ester
$R_f$ value: 0.84 (silica gel, ethyl acetate/methanol = 9:1)
Mass spectrum (EI) : m/z = 249, 251 $[M]^+$ and 205, 207 $[M]^+$

(2) (*R*)-N-(3-bromopropyl)proline methyl ester and (*R*)-N-(3-chloropropyl)proline methyl ester
$R_f$ value: 0.84 (silica gel, ethyl acetate/methanol = 9:1)
Mass spectrum (EI) : m/z = 249, 251 $[M]^+$ and 205, 207 $[M]^+$

## Example IV

### 4-[(3-bromophenyl)amino]-6-(2-bromethoxy)-7-methoxy-quinazoline

7.00 g of potassium carbonate and 8.70 ml of dibromoethane are added to 3.50 g of 4-[(3-bromophenyl) amino]-6-hydroxy-7-methoxy-quinazoline in 350 ml dimethylformamide. The reaction mixture is stirred for two hours at 85°C. Then the mixture is concentrated by evaporation and the oily residue is stirred with methanol. The bright yellow precipitate formed is suction filtered and dried.
Yield: 3.70 g (81 % of theory),
$R_f$ value: 0.44 (silica gel, ethyl acetate)
Mass spectrum ($ESI^+$): m/z = 452, 454, 456 $[M+H]^+$

The following compounds are obtained analogously to Example IV:

(1) 4- [(3-chloro-4-fluoro-phenyl)amino]-6-(2-bromo-ethoxy)-7-cyclopentyloxy-quinazoline
$R_f$ value: 0.74 (silica gel, methylene chloride/methanol/concentrated aqueous ammonia = 90:10:1)
Mass spectrum ($ESI^-$): m/z = 478, 480, 482 $[M-H]^-$

(2) 4-[(3-chloro-4-fluoro-phenyl)amino]-6-cyclopentyloxy-7-(2-bromo-ethoxy)-quinazoline
$R_f$ value: 0.65 (silica gel, methylene chloride/methanol/concentrated aqueous ammonia = 90:10:0.1)
Mass spectrum ($ESI^-$): m/z = 478, 480, 482 $[M-H]^-$

Example V

4-[(3-bromophenyl)amino]-6-hydroxy-7-methoxy-quinazoline

34.50 g of 4-[(3-bromaphenyl)amino]-6-methylcarbonyloxy-7-methoxy-quinazoline in 350 ml ethanol are mixed with 35 ml of 40% sodium hydroxide solution. The reaction mixture is stirred for three hours at ambient temperature. Then the mixture is concentrated by evaporation, the residue is taken up in water and neutralised with 2N hydrochloric acid. The precipitate formed is suction filtered and dried overnight in the circulating air drier at 50°C.
Yield: 28.30 g (92 % of theory),
melting point: 299°C
Mass spectrum ($ESI^+$): m/z = 346, 348 $[M+H]^+$

The following compounds are obtained analogously to Example V:

(1) 4-[(3-chloro-4-fluoro-phenyl)amino]-6-benzyloxy-7-hydroxyquinazoline (The reaction is carried out with concentrated aqueous ammonia in methanol.)
$R_f$ value: 0.54 (silica gel, methylene chloride/methanol = 9:1)
Mass spectrum ($ESI^+$): m/z = 396, 398 $[M+H]^+$
(2) 4-[(3-chloro-4-fluoro-phenyl)amino]-6-cyclopentyloxy-7-hydroxy-quinazoline (The reaction is carried out with concentrated aqueous ammonia in methanol.)
$R_f$ value: 0.53 (silica gel, methylene chloride/methanol/concentrated aqueous ammonia = 90:10:0.1)
Mass spectrum ($ESI^+$): m/z = 374, 376 $[M+H]^+$

Example VI

4-[(3-bromophenyl)amino]-6-methylcarbonyloxy-7-methoxyquinazoline

13.0 ml of 3-bromoaniline are added to 30.00 g of 4-chloro-6-methylcarbonyloxy-7-methoxy-quinazoline in 600 ml isopropanol. The reaction mixture is refluxed for about four hours. The reaction mixture is then left to cool. The precipitate formed is suction filtered, washed thoroughly with cold isopropanol and dried.
Yield: 34.57 g (75 % of theory),
melting point: 238°C
Mass spectrum ($ESI^+$): m/z = 388, 390 $[M+H]^+$

The following compounds are obtained analogously to Example VI:

(1) 4- [(3-chloro-4-fluoro-phenyl)amino]-6-benzyloxy-7-methylcarbonyloxy-quinazoline
Melting point: 267-268 °C
Mass spectrum ($ESI^+$): m/z = 438, 440 $[M+H]^+$

(2) 4-[(3-chloro-4-fluoro-phenyl)amino]-6-cyclopentyloxy-7-methylcarbonyloxy-quinazoline
$R_f$ value: 0.73 (silica gel, methylene chloride/methanol/concentrated aqueous ammonia = 90:10:0.1)
Mass spectrum ($ESI^+$): m/z = 416, 418 $[M+H]^+$

Example VII

4-[(3-bromophenyl)amino]-6-oxiranylmethoxy-7-methoxy-quinazoline

1.50 ml of epibromohydrin are added to 5.00 g of 4-[(3-bromophenyl)amino]-6-hydroxy-7-methoxy-quinazoline and 4.75 g of potassium carbonate in 50 ml dimethylsulphoxide. The reaction mixture is stirred for two days at

50°C. Then it is diluted with about 150 ml of water and stirred for a further two hours. The precipitate formed is suction filtered and purified by chromatography on a silica gel column with ethyl acetate as eluant.
Yield: 850 mg (15 % of theory),
melting point: 230-245°C
Mass spectrum (ESI$^+$): m/z = 402, 404 $[M+H]^+$

Example VIII

Dimethyl 2-(piperazin-1-yl)-succinate dihydrochloride

8.70 g of dimethyl 2-(4-benzyl-piperazin-1-yl)-succinate are hydrogenated in a mixture of 100 ml methanol and 4.50 ml of concentrated hydrochloric acid in the presence of 4.00 g of palladium (10% on activated charcoal) at ambient temperature until the calculated amount of.hydrogen is taken up (about an hour). Then the catalyst is removed by suction filtering and the filtrate is concentrated by evaporation. A white gel-like solid is left.
Yield: 4.18 g
$R_f$ value: 0.80 (Reversed phase ready-made TLC plate (E. Merck), acetonitrile/water/trifluoroacetic acid = 1:1:1)
Mass spectrum (ESI$^+$): m/z = 231 $[M+H]^+$
The following compound is obtained analogously to Example VIII:

(1) dimethyl 3-(piperazin-1-yl)-glutarate dihydrochloride $R_f$ value: 0.80 (Reversed phase ready-made TLC plate (E. Merck), acetonitrile/water/trifluoroacetic acid = 1:1:1)
Mass spectrum (ESI$^+$): m/z = 254 $[M+H]^+$

Example IX

Dimethyl 2-(4-benzyl-piperazin-1-yl)-succinate

7.22 ml of dimethyl maleate are added to 10.0 ml of N-benzylpiperazine in 15 ml dioxane. The reaction mixture is stirred for half an hour at ambient temperature. Then the mixture is refluxed for about a further three hours. For working up the reaction mixture is evaporated to dryness. An orange-yellow oil remains, which slowly crystallises.
Yield: 21.3 g (crude product),
$R_f$ value: 0.85 (silica gel, methylene chloride/methanol/concentrated aqueous ammonia solution = 90:10:0.5)
Mass spectrum (EI) : m/z = 320 $[M]^+$
The following compound is obtained analogously to Example IX:

(1) dimethyl 3-(4-benzyl-piperazin-1-yl)-glutarate (reaction with dimethyl glutaconate)
$R_f$ value: 0.49 (silica gel, cyclohexane/ethyl acetate = 1:1) Mass spectrum (EI): m/z = 334 $[M]^+$

Example X

4-[(3-Chloro-4-fluoro-phenyl)amino]-6-hydroxy-7-cyclopentyloxy-quinazoline

10 ml of trifluoroacetic acid are added to 1.95 g of 4-[(3-chloro-4-fluoro-phenyl)amino]-6-benzyloxy-7-cyclopentyloxy-quinazoline and the resulting dark brown solution is stirred at room temperature over night. Another 5 ml of trifluoroacetic acid are added and the mixture is stirred for approximately 2.5 hours at 50°C until the reaction is completed. The reaction mixture is concentrated *in vacuo*, diluted with water, and adjustet to pH 8-9 by addition of concentrated aqueous ammonia. The precipitate is filtered off with suction, washed with water, and dried in vacuo at 60°C.
Yield: 1.45 g (92 % of theory),
$R_f$ value: 0.56 (silica gel, methylene chloride/methanol 9:1)
Mass spectrum (ESI$^-$): m/z = 372, 374 $[M-H]^-$
The following compound is obtained analogously to Example X:

(1) 4-[(3-chloro-4-fluoro-phenyl)amino]-6-hydroxy-7-cyclopentylmethoxy-quinazoline
$R_f$ value: 0.73 (silica gel, methylene chloride/methanol/concentrated aqueous ammonia = 90:10:0.1)
Mass spectrum (ESI$^-$): m/z = 386, 388 $[M-H]^-$

Example XI

4-[(3-chloro-4-fluoro-phenyl)amino]-6-benzyloxy-7-cyclopentyloxy-quinazoline

0.65 ml of bromocyclopentane are added to a mixture of 2.30 g 4-[(3-chloro-4-fluoro-phenyl)amino]-6-benzyloxy-7-hydroxyquinazoline and 6.00 g potassium carbonate in 6 ml of N,N-dimethyl-formamide and the reaction mixture is stirred for 18 hours at room temperature. Another 3.00 g of potassium carbonate and 4 drops of bromocyclopentane are added, and the resulting mixture is stirred for 2.5 hours at 50°C. The reaction mixture is partitioned between ethyl acetate and water, and the aqueous layer is extracted with ethyl acetate. The combined organic extracts are washed with concentrated aqueous sodium chloride solution, dried over magnesium sulfate and concentrated *in vacuo*. The oily residue is triturated with methanol, the resulting solid precipitate is filtered off, washed with cold methanol, and dried *in vacuo*.
Yield: 2.09 g (77 % of theory),
$R_f$ value: 0.63 (silica gel, methylene chloride/methanol 9:1)
Mass spectrum ($ESI^-$): m/z = 462, 464 $[M-H]^-$
The following compound is obtained analogouslyto Example XI:

(1) 4-[(3-chloro-4-fluoro-phenyl)amino]-6-benzyloxy-7-cyclopentylmethoxy-quinazoline
$R_f$ value: 0.84 (silica gel, methylene chloride/methanol/concentrated aqueous ammonia = 90:10: 1)
Mass spectrum ($ESI^+$): m/z = 478, 480 $[M+H]^+$

Example XII

4-chloro-6-benzyloxy-7-methylcarbonyloxy-quinazoline

Prepared by reaction of 6-benzyloxy-7-methylcarbonyloxy-3H-quinazolin-4-one with thionyl chloride in the presence of catalytic amounts of N,N-dimethyl-formamide.
Yield: 98 % of theory,
$R_f$ value: 0.86 (silica gel, methylene chloride/methanol/concentrated aqueous ammonia = 90:10:0.1)
The following compound is obtained analogously to Example XII:

(1) 4-chloro-6-cyclopentyloxy-7-methylcarbonyloxy-quinazoline $R_f$ value: 0.69 (silica gel, methylene chloride/methanol/concentrated aqueous ammonia = 90:10:0.1)

Example XIII

6-benzyloxy-7-methylcarbonyloxy-3*H*-quinazolin-4-one

Prepared by reaction of 6-benzyloxy-7-hydroxy-3H-quinazolin-4-one with acetic anhydride in pyridine.
Yield: 68 % of theory,
Melting point: 231-233°C
Mass spectrum ($ESI^-$): m/z = 309 $[M-H]^-$
The following compound is obtained analogously to Example XIII:

(1) 6-cyclopentyloxy-7-methylcarbonyloxy-3*H*-quinazolin-4-one $R_f$ value: 0.57 (silica gel, methylene chloride/methanol/concentrated aqueous ammonia = 90:10:0.1)
Mass spectrum ($ESI^-$): m/z = 287 $[M-H]^-$

Example XIV

6-Benzyloxy-7-hydroxy-3*H*-quinazolin-4-one

Prepared by reaction of 2-amino-4-hydroxy-5-benzyloxy-benzoic acid with formamidine acetate in ethanol.
Yield: 72 % of theory,
$R_f$ value: 0.45 (silica gel, methylene chloride/methanol/concentrated aqueous ammonia = 90:10:0.1)
Mass spectrum ($ESI^-$): m/z = 267 $[M-H]^-$
The following compound is obtained analogously to Example XIV:

(1) 6-cyclopentyloxy-7-hydroxy-3*H*-quinazolin-4-one
$R_f$ value: 0.42 (silica gel, methylene chloride/methanol/concentrated aqueous ammonia = 90:10:0.1)
Mass spectrum (EI): m/z = 246 $[M]^+$

Example XV

2-Amino-4-hydroxy-5-benzyloxy-benzoic acid

Prepared by catalytic hydrogenation of 2-nitro-4-hydroxy-5-benzyloxy-benzoic acid with Raney nickel in methanol.
Yield: 71 % of theory,
$R_f$ value: 0.53 (silica gel, methylene chloride/methanol/concentrated aqueous ammonia = 90:10:0.1)
Mass spectrum ($ESI^-$): m/z = 258 $[M-H]^-$
The following compound is obtained analogously to Example XV:

(1) 2-amino-4-hydroxy-5-cyclopentyloxy-benzoic acid
$R_f$ value: 0.38 (silica gel, methylene chloride/methanol/concentrated aqueous ammonia = 90:10:0.1)
Mass spectrum ($ESI^-$): m/z = 236 $[M-H]^-$

Example XVI

2-Nitro-4-hydroxy-5-benzyloxy-benzoic acid

4.8 g of sodium are added portionwise to a mixture of 20.30 g 6-nitro-benzo[1,3]dioxole-5-carboxylic acid and 81.2 ml of benzyl alcohol in 120 ml of dimethyl sulfoxide cooled in an ice/water bath. The reaction mixture is allowed to warm up to room temperature and stirred for approximately 21 hours. The brownish red solution is diluted with 600 ml of water and extracted with methylene chloride. The aqueous layer is acidified with concentrated hydrochloric acid and stirred for two hours at room temperature. The precipitate is filtered off, washed with water, and dried.
Yield: 18.63g (67 % of theory),
Melting point: 172-175°C
Mass spectrum ($ESI^-$): m/z = 288 $[M-H]^-$
The following compound is obtained analogously to Example XVI:

(1) 2-nitro-4-hydroxy-5-cyclopentyloxy-benzoic acid $R_f$ value: 0.61 (silica gel, toluene/1,4-dioxane/ethanol/acetic acid = 90:10:10:6)
Mass spectrum ($ESI^-$): m/z = 266 $[M-H]^-$

Preparation of the end products:

Example 1

4-(3-chloro-4-fluorophenylamino)-6-{3-[4-(methoxycarbonylmethyl)-1-piperazinyl]propyloxy}-7-methoxy-quinazoline

0.07 ml of methyl bromoacetate in 1 ml of acetonitrile is added dropwise to 250 mg of 4-(3-chloro-4-fluorophenylamino)-6-[3-(1-piperazinyl)propyloxy}-7-methoxy-quinazoline and 0.13 ml N-ethyl-diisopropylamine in 5 ml of acetonitrile. After 2 hours' stirring at ambient temperature the mixture is concentrated by evaporation, mixed with water and extracted with ethyl acetate. The organic phases are washed with saline solution, then dried with magnesium sulphate and concentrated by evaporation.
Yield: 150 mg (51 % of theory),
$R_f$ value: 0.54 (silica gel; ethyl acetate/methanol/conc.
aqueous ammonia = 9:1:0.1)
Mass spectrum: (M-H) = 516, 518
The following compounds are obtained analogously to Example 1:

(1) 4-(3-chloro-4-fluorophenylamino)-6-{3-[1- (methoxycarbonylmethyl)-4-piperidinyl]propyloxy}-7-methoxy-quinazoline
$R_f$ value: 0.79 (silica gel; ethyl acetate/methanol/conc.
aqueous ammonia = 9:1:0.1)

Mass spectrum: $M^+$ = 516, 518

(2) (*S*)-4-[(3-bromophenyl)amino]-6-({1-[(ethoxycarbonyl)methyl]-pyrrolidin-2-yl}methoxy)-7-methoxy-quinazoline
$R_f$ value: 0.68 (silica gel, ethyl acetate/methanol/concentrated aqueous ammonia solution = 9:1:0.1)
Mass spectrum (EI) : m/z = 514, 516 $[M]^+$

(3) (*R*)-4-[(3-bromophenyl)amino]-6-({1-[(ethoxycarbonyl)methyl]-pyrrolidin-2-yl}methoxy)-7-methoxy-quinazoline
$R_f$ value: 0.75 (silica gel, ethyl acetate/methanol = 9:1)
Mass spectrum (EI) : m/z = 514, 516 $[M]^+$

(4) (*S*)-4-[(3-chloro-4-fluoro-phenyl)amino]-6-({1-(methoxycarbonyl)methyl]-pyrrolidin-2-yl}methoxy)-7-cyclopentyloxyquinazoline
$R_f$ value: 0.59 (silica gel, methylene chloride/methanol/concentrated aqueous ammonia = 90:10:0.1)
Mass spectrum ($ESI^-$): m/z = 527, 529 $[M-H]^-$

(5) (*S*)-4-[(3-chloro-4-fluoro-phenyl)amino]-6-({1-(methoxycarbonyl)methyl]-pyrrolidin-2-yl}methoxy)-7-cyclopentylmethoxy-quinazoline
$R_f$ value: 0.67 (silica gel, methylene chloride/methanol/concentrated aqueous ammonia = 90:10:0.1)
Mass spectrum ($ESI^-$): m/z = 541, 543 $[M-H]^-$

Example 2

4-(3-chloro-4-fluorophenylamino)-6-{3-[N-(ethoxycarbonylmethyl)-N-methylamino]propyloxy}-7-methoxy-quinazoline

380 mg of a mixture of N-(3-bromopropyl)sarcosine ethyl ester and N-(3-chloropropyl)sarcosine ethyl ester in 5 ml dimethylformamide are added dropwise to 500 mg of 4-(3-chloro-4-fluorophenylamino)-6-hydroxy-7-methoxy-quinazoline and 220 mg of potassium tert.butoxide in 15 ml dimethylformamide. After 3 hours' stirring at 80°C and standing overnight a further 110 mg of potassium tert.butoxide and 190 mg of the sarcosine mixture are added and the reaction mixture is stirred for 4 hours at 80°C. It is filtered, the filtrate is concentrated by evaporation, the residue is taken up in water and extracted with ethyl acetate. The organic phase is separated off, dried and concentrated by evaporation. The residue is purified by chromatography on a silica gel column.
Yield: 390 mg of (52 % of theory),
$R_f$ value: 0.68 (silica gel; ethyl acetate/methanol/conc.
aqueous ammonia = 9:1:0.1)
Mass spectrum: (M-H) = 475, 477

The following compounds are obtained analogously to Example 2:

(1) (*S*)-4-[(3-bromophenyl)amino]-6-[3-(2-methoxycarbonylpyrrolidin-1-yl)propyloxyl-7-methoxy-quinazoline
$R_f$ value: 0.38 (silica gel, ethyl acetate/methanol = 9:1)
Mass spectrum (EI): m/z = 514, 516 $[M]^+$

(2) (*R*)-4-[(3-bromophenyl)amino]-6-[3-(2-methoxycarbonylpyrrolidin-1-yl)propyloxy]-7-methoxy-quinazoline
$R_f$ value: 0.41 (silica gel, ethyl acetate/methanol = 9:1)
Mass spectrum (EI) : m/z = 514, 516 $[M]^+$

Example 3

4-[(3-bromophenyl)amino]-6-(2-{4-[(ethoxycarbonyl)methyl]-piperazin-1-yl}ethoxy)-7-methoxy-quinazoline

1.50 ml of diisopropyl-ethylamine and 1.10 ml of 1-[(ethoxycarbonyl)methyl]-piperazine are added to 1.00 g of 4-[(3-bromophenyl)amino]-6-(2-bromoethoxy)-7-methoxy-quinazoline in 20 ml acetonitrile. The reaction mixture is stirred for two days at ambient temperature. The precipitate formed is filtered off and the filtrate is concentrated by evaporation. The residue is taken up in ethyl acetate and washed once with saturated sodium hydrogen carbonate solution and once with water. The organic phase is dried over magnesium sulphate and concentrated by evaporation. The crude product is purified on a silica gel column with ethyl acetate/ethanol/concentrated aqueous ammonia solution (9:1:0.1) as eluant.
Yield: 450 mg of (38 % of theory),
melting point: 155°C

Mass spectrum (EI) : m/z = 543, 545 $[M]^+$

The following compounds are obtained analogously to Example 3:

(1) 4-[(3-bromophenyl)amino]-6-(2-{N-[(ethoxycarbonyl)methyl]-N-methylamino}ethoxy)-7-methoxy-quinazoline
$R_f$ value: 0.55 (silica gel, ethyl acetate/methanol = 9:1)
Mass spectrum (EI): m/z = 488, 490 $[M]^+$

(2) 4-[(3-bromophenyl)amino]-6- (2-{N,N-bis[(ethoxycarbonyl)methyl]amino}ethoxy)-7-methoxy-quinazoline
$R_f$ value: 0.38 (silica gel, ethyl acetate)
Mass spectrum (EI): m/z = 560, 562 $[M]^+$

(3) 4-[(3-bromophenyl)amino]-6-(2-{4-[1,2 bis(methoxycarbonyl)-ethyl]-piperazin-1-yl}ethoxy)-7-methoxy-quinazoline
$R_f$ value: 0.61 (silica gel, ethyl acetate/methanol = 9:1)
Mass spectrum (EI): m/z = 601, 603 $[M]^+$

(4) 4-[(3-bromophenyl)amino]-6-[2-(4-{1-[(methoxycarbonyl)methyl]-2-(methoxycarbonyl)-ethyl}-piperazin-1-yl) ethoxy]-7-methoxy-quinazoline
$R_f$ value: 0.51 (silica gel, ethyl acetate/methanol = 9:1)
Mass spectrum ($ESI^+$): m/z = 616, 618 $[M+H]^+$

(5) (*R*)-4- [(3-chloro-4-fluoro-phenyl)amino]-6-{2-[2- (methoxycarbonyl)-pyrrolidin-1-yl]-ethoxy}-7-cyclopentyloxy-quinazoline
$R_f$ value: 0.65 (silica gel, methylene chloride/methanol/concentrated aqueous ammonia = 90:10:0.1)
Mass spectrum ($ESI^-$): m/z = 527, 529 $[M-H]^-$

(6) 4-[(3-chloro-4-fluoro-phenyl)amino]-6-(2-{4-[(ethoxycarbonyl)methyl]-piperazin-1-yl}-ethoxy)-7-cyclopentyloxyquinazoline
$R_f$ value: 0.54 (silica gel, methylene chloride/methanol/concentrated aqueous ammonia = 90:10:0.1)
Mass spectrum ($ESI^-$): m/z = 570, 572 $[M-H]^-$

(7)4-[(3-chloro-4-fluoro-phenyl)amino]-6-cyclopentyloxy-7-(2-{N-(2-hydroxy-2-methyl-prop-1-yl)-N-[(ethoxycarbonyl)-methyl]-amino}-ethoxy)-quinazoline
$R_f$ value: 0.28 (silica gel, ethyl acetate)
Mass spectrum ($ESI^-$): m/z = 573, 575 $[M-H]^-$

(8) 4-[(3-chloro-4-fluoro-phenyl)amino]-6-cyclopentyloxy-7-[2-(6,6-dimethyl-2-oxo-morpholin-4-yl)-ethoxy]-quinazoline (This compound was obtained by treatment of the compound prepared by example 3(7) with toluene-4-sulfonic acid in toluene.)
$R_f$ value: 0.23 (silica gel, ethyl acetate)
Mass spectrum ($ESI^-$): m/z = 527, 529 $[M-H]^-$

(9) 4-[(3-chloro-4-fluoro-phenyl)amino]-6-cyclopentyloxy-7-{2-[N-(2-oxo-tetrahydrofuran-3-yl)-N-methyl-amino]-ethoxy}-quinazoline (The starting material 3-methylamino-dihydrofuran-2-one was prepared by reaction of 3-bromo-dihydro-furan-2-one with N-methylbenzylamine and subsequent hydrogenolytic removal of the benzyl group)
$R_f$ value: 0.42 (silica gel, ethyl acetate/methanol = 9:1)
Mass spectrum ($ESI^+$): m/z = 515, 517 $[M+H]^+$

(10) 4- [(3-bromo-phenyl)amino]-6- (2-{N-(2-hydroxy-2-methylprop-1-yl)-N-[(ethoxycarbonyl)methyl]-amino}-ethoxy)-7-methoxy-quinazoline

(11) 4-[(3-bromo-phenyl)amino]-6-[2-(6,6-dimethyl-2-oxo-morpholin-4-yl)-ethoxy]-7-methoxy-quinazoline
$R_f$ value: 0.33 (silica gel, ethyl acetate)
Mass spectrum ($ESI^-$) : m/z = 499, 500 $[M+H]^-$

(12) 4-[(3-bromo-phenyl)amino]-6-{2-[N- (2-oxo-tetrahydrofuran-4-yl)-N-methyl-amino]-ethoxy}-7-methoxy-quinazoline (The starting material 4-methylamino-dihydro-furan-2-one was prepared by reaction of 5H-furan-2-one with N-methyl-benzylamine and subsequent hydrogenolytic removal of the benzyl group)

$R_f$ value: 0.38 (silica gel, ethyl acetate/methanol = 9:1)
Mass spectrum ($ESI^-$) : m/z = 485, 487 $[M-H]^-$

Example 4

4-[(3-bromophenyl)amino]-6-(3-{4-[(ethoxycarbonyl)methyl]-piperazin-1-yl}-2-hydroxy-propyloxy)-7-methoxy-quinazoline

0.16 ml of 1-[(ethoxycarbonyl)methyl]-piperazine are added to 500 mg of 4-[(3-bromophenyl)amino]-6-oxiranylmethoxy-7-methoxy-quinazoline in 5 ml ethanol. The reaction mixture is refluxed for about 6 hours. Then the mixture is concentrated by evaporation and the crude product is purified by chromatography on a silica gel column with ethyl acetate/ethanol/concentrated aqueous ammonia solution (9:1:0.1) as eluant.
Yield: 97 mg of (14 % of theory),
melting point: 118-122°C
Mass spectrum (EI) : m/z = 573, 575 $[M]^+$

Example 5

4- [(3-bromophenyl)amino]-6-{2-[4-(carboxymethyl)-piperazin-1-yl]ethoxy}-7-methoxy-quinazoline

0.19 ml of 1N sodium hydroxide solution are added to 100 mg of 4- [(3-bromophenyl)amino]-6-(2-{4-[(ethoxycarbonyl)methyl]-piperazin-1-yl}ethoxy)-7-methoxy-quinazoline in 0.30 ml of tetrahydrofuran. The reaction mixture is stirred for three hours at ambient temperature. Another 0.9 ml of 1N sodium hydroxide solution are added and the mixture is stirred overnight. Then it is neutralised with 1N hydrochloric acid and concentrated by evaporation. The solid residue is triturated with ethyl acetate and suction filtered.
Yield: 100 mg (contains about 0.5 equivalents sodium chloride),
$R_f$ value: 0.50 (Reversed phase ready-made TLC plate (E. Merck), acetonitrile/water/trifluoroacetic acid = 50:50:1)
Mass spectrum ($ESI^-$): m/z = 514, 516 $[M-H]^-$

The following compounds may also be obtained analogously to the foregoing Examples and other methods known from the literature:

(1) 4-[(3-bromophenyl)amino]-6-({1-[(methoxycarbonyl)methyl]-piperidin-4-yl}methoxy)-7-methoxy-quinazoline

(2) 4-[(3-methylphenyl)amino]-6-({1-[(ethoxycarbonyl)methyl]-piperidin-4-yl}methoxy)-7-methoxy-quinazoline

(3) 4-[(3-chlorophenyl)amino]-6-({1-[(ethoxycarbonyl)methyl]-piperidin-4-yl}methoxy)-7-methoxy-quinazoline

(4) 4-[(3-chloro-4-fluoro-phenyl)amino]-6-({1-[(ethoxycarbonyl)methyl]-piperidin-4-yl}methoxy)-7-methoxy-quinazoline

(5) 4-[(1-phenylethyl)amino]-6-({1-[(ethoxycarbonyl)methyl]-piperidin-4-yl}methoxy)-7-methoxy-quinazoline

(6) 4-[(3-ethynylphenyl)amino]-6-({1-[(ethoxycarbonyl)methyl]-piperidin-4-yl}methoxy)-7-methoxy-quinazoline

(7) 4-[(3-bromophenyl)amino]-6-({1-[(ethoxycarbonyl)methyl]-piperidin-4-yl}methoxy)-7-methoxy-quinazoline

(8) 4-[(3-bromophenyl)amino]-6-({1-[(hexyloxycarbonyl)methyl]-piperidin-4-yl}methoxy)-7-methoxy-quinazoline

(9) 4-[(3-bromophenyl)amino]-6-({1-[2-(ethoxycarbonyl)ethyl]-piperidin-4-yl}methoxy)-7-methoxy-quinazoline

(10) 4-[(3-bromophenyl)amino]-6-({1-[3-(ethoxycarbonyl)propyl]-piperidin-4-yl}methoxy)-7-methoxy-quinazoline

(11) 4-[(3-bromophenyl)amino]-6-({1-[(ethoxycarbonyl)methyl]-piperidin-3-yl}methoxy)-7-methoxy-quinazoline

(12) 4-[(3-bromophenyl)amino]-6-({1-[(ethoxycarbonyl)methyl]-pyrrolidin-2-yl}methoxy)-7-methoxy-quinazoline

(13) 4-[(3-bromophenyl)amino]-6-({1-[(dimethoxyphosphoryl)-methyl]-piperidin-4-yl}methoxy)-7-methoxy-quinazoline

(14) 4-[(3-bromophenyl)amino]-6-[(1-{[(methoxy)(methyl)phosphoryl]methyl}-piperidin-4-yl)methoxy]-7-methoxy-quinazoline

(15) 4-[(3-bromophenyl)amino]-6-({1-[1,2-bis(ethoxycarbonyl)-ethyl]-piperidin-4-yl}methoxy)-7-methoxy-quinazoline

(16) 4-[(3-bromophenyl)amino]-6-[(1-{1-[(ethoxycarbonyl)methyl]-2-(ethoxycarbonyl)-ethyl}-piperidin-4-yl)methoxy]-7-methoxy-quinazoline

(17) 4-[(3-bromophenyl)amino]-6- (2-{1-[1-(methoxycarbonyl)-ethyl]-piperidin-4-yl}ethoxy)-7-methoxy-quinazoline

(18) 4-[(3-bromophenyl)amino]-6-(2-{1-[(methoxycarbonyl)methyl]-piperidin-4-yl}ethoxy)-7-methoxy-quinazoline

(19) 4-[(3-bromophenyl)amino]-6-(2-{4-[(methoxycarbonyl)methyl]-piperazin-1-yl}ethoxy)-7-methoxy-quinazoline

(20) 4-[(3-bromophenyl)amino]-6-(2-{4-[(ethoxycarbonyl)methyl]-piperazin-1-yl}ethoxy)-7-methoxy-quinazoline

(21) 4-[(3-bromophenyl)amino]-6-(2-{1-[(ethoxycarbonyl)methyl]-piperidin-4-yl}ethoxy)-7-methoxy-quinazoline

(22) 4-[(3-bromophenyl)amino]-6-(2-{1-[1,2-bis (ethoxycarbonyl)-ethyl]-piperidin-4-yl}ethoxy)-7-methoxy-quinazoline

(23) 4-[(3-bromophenyl)amino]-6-(2-{4-[1,2-bis(ethoxycarbonyl)ethyl]-piperazin-1-yl}ethoxy)-7-methoxy-quinazoline

(24) 4- [(3-bromophenyl)amino]-6-[2-(4-{1-[(ethoxycarbonyl)methyl]-2-(ethoxycarbonyl)-ethyl}-piperazin-1-yl) ethoxy]-7-methoxy-quinazoline

(25) 4-[(3-bromophenyl)amino]-6-[2-(1-{1-[(ethoxycarbonyl)methyl]-2-(ethoxycarbonyl)-ethyl}-piperidin-4-yl) ethoxy]-7-methoxy-quinazoline

(26) 4-[(3-bromophenyl)amino]-6-{2-[2-(methoxycarbonyl)-pyrrolidin-1-yl]ethoxy}-7-methoxy-quinazoline

(27) 4-[(3-bromophenyl)amino]-6-{2-[2-(ethoxycarbonyl)-piperidin-1-yl]ethoxy}-7-methoxy-quinazoline

(28) 4- [(3-bromophenyl)amino]-6-(3-{1-[(methoxycarbonyl)methyl]-piperidin-4-yl}propyloxy)-7-methoxy-quinazoline

(29) 4-[(3-bromophenyl)amino]-6-(3-{4-[(methoxycarbonyl)methyl]-piperazin-1-yl}propyloxy)-7-methoxy-quinazoline

(30) 4-[(3-bromophenyl)amino]-6-(3-{4-[(ethoxycarbonyl)methyl]-piperazin-3-yl}propyloxy)-7-methoxy-quinazoline

(31) 4-[(3-bromophenyl)amino]-6-(3-{1-[(ethoxycarbonyl)methyl]-piperidin-4-yl}propyloxy)-7-methoxy-quinazoline

(32) 4-[(3-bromophenyl)amino]-6-(3-{1-[(ethoxycarbonyl)methyl]-piperidin-4-yl}-2-hydroxy-propyloxy)-7-methoxy-quinazoline

(33) 4-[(3-bromophenyl)amino]-6-(3-{4-[(ethoxycarbonyl)methyl]-piperazin-1-yl}-2-hydroxy-propyloxy)-7-methoxy-quinazoline

(34) 4-[(3-methylphenyl)amino]-6-(3-{4-[(ethoxycarbonyl)methyl]-piperazin-1-yl}propyloxy)-7-methoxy-quinazoline

(35) 4-[(3-chlorophenyl)amino]-6-(3-{4-[(ethoxycarbonyl)methyl]-piperazin-1-yl}propyloxy)-7-methoxy-quinazoline

(36) 4-[(1-phenylethyl)amino]-6-(3-{4-[(ethoxycarbonyl)methyl]-piperazin-1-yl}propyloxy)-7-methoxy-quinazoline

(37) 4-[(3-bromophenyl)amino]-6-{3-[2-(methoxycarbonyl)-pyrrolidin-1-yl]propyloxy}-7-methoxy-quinazoline

(38) 4-[(3-bromophenyl)amino]-6-{3-[3-(methoxycarbonyl)-4-methyl-piperazin-1-yl]propyloxy}-7-methoxy-quinazoline

(39) 4-[(3-bromophenyl)amino]-6-({1-[(ethoxycarbonyl)methyl]-piperidin-4-yl}methoxy)-7-ethoxy-quinazoline

(40) 4-[(3-bromophenyl)amino]-6-({1-[(ethoxycarbonyl)methyl]-piperidin-4-yl}methoxy)-7-(2-methoxyethoxy)-quinazoline

(41) 4-[(3-bromophenyl)amino]-6-(2-{1-[(ethoxycarbonyl)methyl]-piperidin-4-yl}ethoxy)-7-(2-methoxyethoxy)-quinazoline

(42) 4-[(3-bromophenyl)amino]-6-(2-{4-[(ethoxycarbonyl)methyl]-piperazin-1-yl}ethoxy)-7-(2-methoxyethoxy)-quinazoline

(43) 4-[(3-bromophenyl)amino]-6-(2-{4-[(ethoxycarbonyl)methyl]-piperazin-1-yl}ethoxy)-7-ethoxy-quinazoline

(44) 4-[(3-bromophenyl)amino]-6-(3-{1-[(ethoxycarbonyl)methyl]-piperidin-4-yl}propyloxy)-7-ethoxy-quinazoline

(45) 4-[(3-bromophenyl)amino]-6-(3-{4-[(ethoxycarbonyl)methyl]-piperazin-1-yl}propyloxy)-7-(2-methoxyethoxy)-quinazoline

(46) 4-[(3-bromophenyl)amino]-6-(3-{1-[(dimethoxyphosphoryl)-methyl]-piperidin-4-yl}propyloxy)-7-methoxy-quinazoline

(47) 4-[(3-bromophenyl)amino]-6-(3-{4-[(dimethoxyphosphoryl)-methyl]-piperazin-1-yl}propyloxy)-7-methoxy-quinazoline

(48) 4-[(3-bromophenyl)amino]-6-[3-(4-{[(methoxy)(ethyl) phosphoryl]methyl}-piperazin-1-yl)propyloxy]-7-methoxy-quinazoline

(49) 4-[(3-bromophenyl)amino]-6-[3-(1-{[(methoxy)(ethyl)phosphoryl]methyl}-piperidin-4-yl)propyloxy]-7-methoxy-quinazoline

(50) 4-[(3-bromophenyl)amino]-6-(3-{4-[1,2-bis(ethoxycarbonyl)-ethyl]-piperazin-1-yl}propyloxy)-7-methoxy-quinazoline

(51) 4-[(3-bromophenyl)amino]-6-[3-(1-{1-[(ethoxycarbonyl)methyl]-2-(ethoxycarbonyl)-ethyl}-piperidin-4-yl)propyloxy]-7-methoxy-quinazoline

(52) 4-[(3-bromophenyl)amino]-6-(4-{1-[(ethoxycarbonyl)methyl]-piperidin-4-yl}butyloxy)-7-methoxy-quinazoline

(53) 4-[(3-bromophenyl)amino]-6-(4-{4-[(ethoxycarbonyl)methyl]-piperazin-1-yl}butyloxy)-7-methoxy-quinazoline

(54) 4-[(3-bromophenyl)amino]-6-(2-{N-[(ethoxycarbonyl)methyl]-N-methylamino}ethoxy)-7-methoxy-quinazoline

(55) 4-[(3-bromophenyl)amino]-6-(2-{N,N-bis[(ethoxycarbonyl)methyl]amino}ethoxy)-7-methoxy-quinazoline

(56) 4-[(3-bromophenyl)amino]-6-(2-{N-[(ethoxycarbonyl)methyl]-N-ethylamino}ethoxy)-7-methoxy-quinazoline

(57) 4- [(3-bromophenyl)amino]-6-(2-{N-[(ethoxycarbonyl)methyl]-N-(cyclopropylmethyl)-amino}ethoxy)-7-methoxy-quinazoline

(58) 4-[(3-bromophenyl)amino]-6-(2-{[(ethoxycarbonyl)methyl]-amino}ethoxy)-7-methoxy-quinazoline

(59) 4-[(3-bromophenyl)amino]-6-(2-{N-[(ethoxycarbonyl)methyl]-N-cyclopropyl-amino}ethoxy)-7-methoxy-quinazoline

(60) 4-[(3-bromophenyl)amino]-6-(2-{N-[(methoxycarbonyl)methyl]-N-methylamino}ethoxy)-7-methoxy-quinazoline

(61) 4-[(3-bromophenyl)amino]-6-(3-{N-[(methoxycarbonyl)methyl]-N-methylamino}propyloxy)-7-methoxy-quinazoline

(62) 4-[(3-bromophenyl)amino]-6-(3-{N,N-bis[(methoxycarbonyl)-methyl]amino}propyloxy)-7-methoxy-quinazoline

(63) 4-[(3-bromophenyl)amino]-6-(3-{[(ethoxycarbonyl)methyl]-amino}propyloxy)-7-methoxy-quinazoline

(64) 4-[(3-bromophenyl)amino]-6-(4-{N-[(ethoxycarbonyl)methyl]-N-methylamino}butyloxy)-7-methoxy-quinazoline

(65) 4-[(3-bromophenyl)amino]-6-(4-{N,N-bis[(ethoxycarbonyl)methyl]amino}butyloxy)-7-methoxy-quinazoline

(66) 4-[(3-bromophenyl)amino]-6-({4-[(methoxycarbonyl)methyl]-2-oxo-morpholin-6-yl}methyloxy)-7-methoxy-quinazoline

(67) 4-[(3-bromophenyl)amino]-6-[(4-methyl-2-oxo-morpholin-6-yl)methyloxyl-7-methoxy-quinazoline

(68) 4-[(3-bromophenyl)amino]-6-[(2-oxo-morpholin-6-yl)methyloxy]-7-methoxy-quinazoline

(69) 4-[(3-bromophenyl)amino]-7-({1-[(methoxycarbonyl)methyl]-piperidin-4-yl}methoxy)-6-methoxy-quinazoline

(70) 4-[(3-methylphenyl)amino]-7-({1-[(ethoxycarbonyl)methyl]-piperidin-4-yl}methoxy)-6-methoxy-quinazoline

(71) 4-[(3-chlorophenyl)amino]-7-({1-[(ethoxycarbonyl)methyl]-piperidin-4-yl}methoxy)-6-methoxy-quinazoline

(72) 4-[(3-chloro-4-fluoro-phenyl)amino]-7-({1-[(ethoxycarbonyl)methyl]-piperidin-4-yl}methoxy)-6-methoxy-quinazoline

(73) 4-[(1-phenylethyl)amino]-7-({1-[(ethoxycarbonyl)methyl]-piperidin-4-yl}methoxy)-6-methoxy-quinazoline

(74) 4-[(3-ethynylphenyl)amino]-7-({1-[(ethoxycarbonyl)methyl]-piperidin-4-yl}methoxy)-6-methoxy-quinazoline

(75) 4-[(3-bromophenyl)amino]-7-({1-[(ethoxycarbonyl)methyl]-piperidin-4-yl}methoxy)-6-methoxy-quinazoline

(76) 4-[(3-bromophenyl)amino]-7-({1-[(hexyloxycarbonyl)methyl]-piperidin-4-yl}methoxy)-6-methoxy-quinazoline

(77) 4-[(3-bromophenyl)amino]-7-({1-[2-(ethoxycarbonyl)ethyl]-piperidin-4-yl}methoxy)-6-methoxy-quinazoline

(78) 4-[(3-bromophenyl)amino]-7-({1-[3- (ethoxycarbonyl)propyl]-piperidin-4-yl}methoxy)-6-methoxy-quinazoline

(79) 4-[(3-bromophenyl)amino]-7-({1-[(ethoxycarbonyl)methyl]-piperidin-3-yl}methoxy)-6-methoxy-quinazoline

(80) 4-[(3-bromophenyl)amino]-7-({1-[(ethoxycarbonyl)methyl]-pyrrolidin-2-yl}methoxy)-6-methoxy-quinazoline

(81) 4-[(3-bromophenyl)amino]-7-({1-[(dimethoxyphosphoryl)methyl]-piperidin-4-yl}methoxy)-6-methoxy-quinazoline

(82) 4-[(3-bromophenyl)amino]-7-[(1-{[(methoxy)(methyl)phosphoryl]methyl}-piperidin-4-yl)methoxy]-6-methoxy-quinazoline

(83) 4-[(3-bromophenyl)amino]-7-({1-[1,2-bis(ethoxycarbonyl)-ethyl]-piperidin-4-yl}methoxy)-6-methoxy-quinazoline

(84) 4-[(3-bromophenyl)amino]-7-[(1-{1-[(ethoxycarbonyl)methyl]-2-(ethoxycarbonyl)-ethyl}-piperidin-4-yl)methoxy]-6-methoxy-quinazoline

(85) 4-[(3-bromophenyl)amino]-7-(2-{1-[1-(methoxycarbonyl)-ethyl]-piperidin-4-yl}ethoxy)-6-methoxy-quinazoline

(86) 4-[(3-bromophenyl)amino]-7-(2-{1-[(methoxycarbonyl)methyl]-piperidin-4-yl}ethoxy)-6-methoxy-quinazoline

(87) 4- [(3-bromophenyl)amino]-7-(2-{4-[(methoxycarbonyl)methyl]-piperazin-1-yl}ethoxy)-6-methoxy-quinazoline

(88) 4-[(3-bromophenyl)amino]-7-(2-{4-[(ethoxycarbonyl)methyl]-piperazin-1-yl}ethoxy)-6-methoxy-quinazoline

(89) 4-[(3-bromophenyl)amino]-7-(2-{1-[(ethoxycarbonyl)methyl]-piperidin-4-yl}ethoxy)-6-methoxy-quinazoline

(90) 4-[(3-bromophenyl)amino]-7-(2-{1-[1,2-bis(ethoxycarbonyl)ethyl]-piperidin-4-yl}ethoxy)-6-methoxy-quinazoline

(91) 4-[(3-bromophenyl)amino]-7-(2-{4-[1,2-bis(ethoxycarbonyl)ethyl]-piperazin-1-yl}ethoxy)-6-methoxy-quinazoline

(92) 4-[(3-bromophenyl)amino]-7-[2-(4-{1-[(ethoxycarbonyl)methyl]-2-(ethoxycarbonyl)-ethyl}-piperazin-1-yl)ethoxy]-6-methoxy-quinazoline

(93) 4-[(3-bromophenyl)amino]-7-[2-(1-{1-[(ethoxycarbonyl)methyl]-2-(ethoxycarbonyl)-ethyl}-piperidin-4-yl)ethoxy]-6-methoxy-quinazoline

(94) 4-[(3-bromophenyl)amino]-7-{2-[2-(methoxycarbonyl)-pyrrolidin-1-yl]ethoxy}-6-methoxy-quinazoline

(95) 4-[(3-bromophenyl)amino]-7-{2-[2-(ethoxycarbonyl)-piperidin-1-yl]ethoxy}-6-methoxy-quinazoline

(96) 4-[(3-bromophenyl)amino]-7-(3-{1-[(methoxycarbonyl)methyl]-piperidin-4-yl}propyloxy)-6-methoxy-quinazoline

(97) 4-[(3-bromophenyl)amino]-7-(3-{4-[(methoxycarbonyl)methyl]-piperazin-1-yl}propyloxy)-6-methoxy-quinazoline

(98) 4-[(3-bromophenyl)amino]-7-(3-{4-[(ethoxycarbonyl)methyl]-piperazin-1-yl}propyloxy)-6-methoxy-quinazoline

(99) 4-[(3-bromophenyl)amino]-7-(3-{1-[(ethoxycarbonyl)methyl]-piperidin-4-yl}propyloxy)-6-methoxy-quinazoline

(100) 4-[(3-bromophenyl)amino]-7-(3-{1-[(ethoxycarbonyl)methyl]-piperidin-4-yl}-2-hydroxy-propyloxy)-6-methoxy-quinazoline

(101) 4-[(3-bromophenyl)amino]-7-(3-{4-[(ethoxycarbonyl)methyl]-piperazin-1-yl}-2-hydroxy-propyloxy)-6-methoxy-quinazoline

(102) 4-[(3-methylphenyl)amino]-7-(3-{4-[(ethoxycarbonyl)methyl]-piperazin-1-yl}propyloxy)-6-methoxy-quinazoline

(103) 4-[(3-chlorophenyl)amino]-7-(3-{4-[(ethoxycarbonyl)methyl]-piperazin-1-yl}propyloxy)-6-methoxy-quinazoline

(104) 4-[(1-phenylethyl)amino]-7-(3-{4-[(ethoxycarbonyl)methyl]-piperazin-1-yl}propyloxy)-6-methoxy-quinazoline

(105) 4-[(3-bromophenyl)amino]-7-{3-[2-(methoxycarbonyl)-pyrrolidin-1-yl]propyloxy}-6-methoxy-quinazoline

(106) 4-[(3-bromophenyl)amino]-7-{3-[3-(methoxycarbonyl)-4-methyl-piperazin-1-yl]propyloxy}-6-methoxy-quinazoline

(107) 4-[(3-bromophenyl)amino]-7-({1-[(ethoxycarbonyl)methyl]-piperidin-4-yl}methoxy)-6-ethoxy-quinazoline

(108) 4-[(3-bromophenyl)amino]-7-({1-[(ethoxycarbonyl)methyl]-piperidin-4-yl}methoxy)-6-(2-methoxyethoxy)-quinazoline

(109) 4-[(3-bromophenyl)amino]-7-(2-{1-[(ethoxycarbonyl)methyl]-piperidin-4-yl}ethoxy)-6-(2-methoxyethoxy)-quinazoline

(110) 4-[(3-bromophenyl)amino]-7-(2-{4-[(ethoxycarbonyl)methyl]-piperazin-1-yl}ethoxy)-6-(2-methoxyethoxy)-quinazoline

(111) 4-[(3-bromophenyl)amino]-7-(2-{4-[(ethoxycarbonyl)methyl]-piperazin-1-yl}ethoxy)-6-ethoxy-quinazoline

(112) 4-[(3-bromophenyl)amino]-7-(3-{1-[(ethoxycarbonyl)methyl]-piperidin-4-yl}propyloxy)-6-ethoxy-quinazoline

(113) 4-[(3-bromophenyl)amino]-7-(3-{4-[(ethoxycarbonyl)methyl]-piperazin-1-yl}propyloxy)-6-(2-methoxyethoxy)-quinazoline

(114) 4-[(3-bromophenyl)amino]-7-(3-{1-[(dimethoxyphosphoryl)-methyl]-piperidin-4-yl}propyloxy)-6-methoxy-quinazoline

(115) 4-[(3-bromophenyl)amino]-7-(3-{4-[(dimethoxyphosphoryl)-methyl]-piperazin-1-yl}propyloxy)-6-methoxy-quinazoline

(116) 4-[(3-bromophenyl)amino]-7-[3-(4 {[(methoxy)(ethyl) phosphoryl]methyl}-piperazin-1-yl)propyloxy]-6-methoxy-quinazoline

(117) 4-[(3-bromophenyl)amino]-7-[3-(1-{[(methoxy)(ethyl) phosphoryl]methyl}-piperidin-4-yl)propyloxy]-6-methoxy-quinazoline

(118) 4-[(3-bromophenyl)amino]-7-(3-{4-[1,2-bis(ethoxycarbonyl)ethyl]-piperazin-1-yl}propyloxy)-6-methoxy-quinazoline

(119) 4-[(3-bromophenyl)amino]-7-[3-(1-{1-[(ethoxycarbonyl)-methyl]-2-(ethoxycarbonyl)-ethyl}-piperidin-4-yl) propyloxy]-6-methoxy-quinazoline

(120) 4-[(3-bromophenyl)amino]-7-(4-{1-[(ethoxycarbonyl)methyl]-piperidin-4-yl}butyloxy)-6-methoxy-quinazoline

(121) 4-[(3-bromophenyl)amino]-7-(4-{4-[(ethoxycarbonyl)methyl]-piperazin-1-yl}butyloxy)-6-methoxy-quinazoline

(122) 4-[(3-bromophenyl)amino]-7-(2-{N-[(ethoxycarbonyl)methyl]-N-methylamino}ethoxy)-6-methoxy-quinazoline

(123) 4-[(3-bromophenyl)amino]-7-(2-{N,N-bis[(ethoxycarbonyl)-methyl]amino}ethoxy)-6-methoxy-quinazoline

(124) 4-[(3-bromophenyl)amino]-7-(2-{N-[(ethoxycarbonyl)methyl]-N-ethylamino}ethoxy)-6-methoxy-quinazoline

(125) 4-[(3-bromophenyl)amino]-7-(2-{N-[(ethoxycarbonyl)methyl]-N-(cyclopropylmethyl)-amino}ethoxy)-6-methoxy-quinazoline

(126) 4- [(3-bromophenyl)amino]-7-(2-{[(ethoxycarbonyl)methyl]-amino}ethoxy)-6-methoxy-quinazoline

(127) 4-[(3-bromophenyl)amino]-7-(2-{N-[(ethoxycarbonyl)methyl]-N-cyclopropyl-amino}ethoxy)-6-methoxy-quinazoline

(128) 4-[(3-bromophenyl)amino]-7-(2-{N-[(methoxycarbonyl)methyl]-N-methylamino}ethoxy)-6-methoxy-quinazoline

(129) 4-[(3-bromophenyl)amino]-7-(3-{N-[(methoxycarbonyl)methyl]-N-methylamino}propyloxy)-6-methoxy-quinazoline

(130) 4-[(3-bromophenyl)amino]-7-(3-{N,N-bis[(methoxycarbonyl)-methyl]amino}propyloxy)-6-methoxy-quinazoline

(131) 4- [(3-bromophenyl)amino]-7- (3-{[(ethoxycarbonyl)methyl]-amino}propyloxy)-6-methoxy-quinazoline

(132) 4-[(3-bromophenyl)amino]-7-(4-{N-[(ethoxycarbonyl)methyl]-N-methylamino}butyloxy)-6-methoxy-quinazoline

(133) 4-[(3-bromophenyl)amino]-7-(4-{N,N-bis[(ethoxycarbonyl)-methyl]amino}butyloxy)-6-methoxy-quinazoline

(134) 4-[(3-bromophenyl)amino]-7-({4-[(methoxycarbonyl)methyl]-2-oxo-morpholin-6-yl}methyloxy)-6-methoxy-quinazoline

(135) 4-[(3-bromophenyl)amino]-7-[(4-methyl-2-oxo-morpholin-6-yl)methyloxy]-6-methoxy-quinazoline

(136) 4-[(3-bromophenyl)amino]-7-[(2-oxo-morpholin-6-yl)methyloxy]-6-methoxy-quinazoline

(137) 4-[(3-bromophenyl)amino]-6-[2-(2-oxo-morpholin-4-yl)-ethoxy]-7-methoxy-quinazoline

(138) 4-[(3-bromophenyl)amino]-6-[3-(2-oxo-morpholin-4-yl)propyloxy]-7-methoxy-quinazoline

(139) 4-[(3-bromophenyl)amino]-6-[2-(3-methyl-2-oxo-morpholin-4-yl)ethoxy]-7-methoxy-quinazoline

(140) 4-[(3-bromophenyl)amino]-6-[2-(5,5-dimethyl-2-oxo-morpholin-4-yl)ethoxy]-7-methoxy-quinazoline

(141) 4-[(3-bromophenyl)amino]-6-(2-{4-[(ethoxycarbonyl)methyl]-piperazin-1-yl}ethoxy)-7-cyclopropylmethoxy-quinazoline

(142) 4-[(3-bromophenyl)amino]-6-(2-{4-[(ethoxycarbonyl)methyl]-piperazin-1-yl}ethoxy)-7-cyclobutyloxy-quinazoline

(143) 4-[(3-bromophenyl)amino]-6-(2-{4-[(ethoxycarbonyl)methyl]-piperazin-1-yl}ethoxy)-7-cyclopentyloxy-quinazoline

(144) 4-[(3-bromophenyl)amino]-6-(2-{4-[(ethoxycarbonyl)methyl]-piperazin-1-yl}ethoxy)-7-cyclohexyloxy-quinazoline

(145) 4-[(3-bromophenyl)amino]-6-(2-{4-[(ethoxycarbonyl)methyl]-piperazin-1-yl}ethoxy)-7-cyclopentylmethoxy-quinazoline

(146) 4-[(3-bromophenyl)amino]-6-(2-{4-[(ethoxycarbonyl)methyl]-piperazin-1-yl}ethoxy)-7-cyclohexylmethoxy-quinazoline

(147) 4-[(3-bromophenyl)amino]-6-(2-{4-[(benzyloxycarbonyl)methyl]-piperazin-1-yl}ethoxy)-7-methoxy-quinazoline

(148) 4-[(3-bromophenyl)amino]-6-(2-{4-[(phenyloxycarbonyl)-methyl]-piperazin-1-yl}ethoxy)-7-methoxy-quinazoline

(149) 4-[(3-bromophenyl)amino]-6-(2-{4-[(indan-5-yloxycarbonyl)methyl]-piperazin-1-yl}ethoxy)-7-rnethoxy-quinazoline

(150) 4-[(3-bromophenyl)amino]-6-(2-{4-[(cyclohexyloxycarbonyl)methyl]-piperazin-1-yl}ethoxy)-7-methoxy-quinazoline

(151) 4-[(3-bromophenyl)amino]-6-(2-{4-[(cyclohexylmethoxycarbonyl)methyl]-piperazin-1-yl}ethoxy)-7-methoxy-quinazoline

(152) 4-[(3-bromophenyl)amino]-6-cyclopropylmethoxy-7-(2-{4-[(ethoxycarbonyl)methyl]-piperazin-1-yl}ethoxy) quinazoline

(153) 4-[(3-bromophenyl)amino]-6-cyclobutyloxy-7-(2-{4-[(ethoxycarbonyl)methyl]-piperazin-1-yl}ethoxy)quinazoline

(154) 4-[(3-bromophenyl)amino]-6-cyclopentyloxy-7- (2-{4-[(ethoxycarbonyl)methyl]-piperazin-1-yl}ethoxy)quinazoline

(155) 4-[(3-bromophenyl)amino]-6-cyclopentylmethoxy-7-(2-{4-[(ethoxycarbonyl)methyl]-piperazin-1-yl}ethoxy)quinazoline

(156) 4-[(3-bromophenyl)amino]-6-cyclohexylmethoxy-7-(2-{4-[(ethoxycarbonyl)methyl]-piperazin-1-yl}ethoxy)quinazoline

(157) 4-[(3-bromophenyl)amino]-6-cyclohexyloxy-7-(2-{4-[(ethoxycarbonyl)methyl]-piperazin-1-yl}ethoxy)quinazoline

(158) 4-[(3-chloro-4-fluoro-phenyl)amino]-6-[2-(6,6-dimethyl-2-oxo-morpholin-4-yl)-ethoxy]-7-methoxy-quinazoline

(159) 4-[(3-chloro-4-fluoro-phenyl)amino]-6-[2-(6,6-dimethyl-2-oxo-morpholin-4-yl)-ethoxy]-7-cyclobutyloxy-quinazoline

(160) 4-[(3-chloro-4-fluoro-phenyl)amino]-6-[2-(6, 6-dimethyl-2-oxo-morpholin-4-yl)-ethoxy]-7-cyclopentyloxy-quinazoline

(161) 4-[(3-chloro-4-fluoro-phenyl)amino]-6-[2-(6,6-dimethyl-2-oxo-morpholin-4-yl)-ethoxy]-7-cyclohexyloxy-quinazoline

(162) 4-[(3-chloro-4-fluoro-phenyl)amino]-6-[2-(6, 6-dimethyl-2-oxo-morpholin-4-yl)-ethoxy]-7-cyclopropylmethoxy-quinazoline

(163) 4- [(3-chloro-4-fluoro-phenyl]amino]-6-[2-(6,6-dimethyl-2-oxo-morpholin-4-yl)-ethoxy]-7-cyclopentylmethoxy-quinazoline

(164) 4-[(3-chloro-4-fluoro-phenyl)amino]-6-[2-(6, 6-dimethyl-2-oxo-morpholin-4-yl)-ethoxy]-7-cyclohexylmethoxy-quinazoline

(165) 4-[(3-chloro-4-fluoro-phenyl)amino]-6-{2-[N-(2-oxotetrahydrofuran-4-yl)-N-methyl-amino]-ethoxy}-7-methoxy-quinazoline

(166) 4-[(3-chloro-4-fluoro-phenyl)amino]-6-{2-[N-(2-oxotetrahydrofuran-4-yl)-N-methyl-amino]-ethoxy}-7-cyclopentyloxy-quinazoline

(167) 4-[(3-chloro-4-fluoro-phenyl)amino]-6-{2-[N-(2-oxotetrahydrofuran-4-yl)-N-methyl-amino]-ethoxy}-7-cyclopentylmethoxy-quinazoline

(168) 4-[(3-chloro-4-fluoro-phenyl)amino]-6-{2-[N-(2-oxotetrahydrofuran-3-yl)-N-methyl-amino]-ethoxy}-7-methoxy-quinazoline

(169) 4-[(3-chloro-4-fluoro-phenyl)amino]-6-{2-[N-(2-oxotetrahydrofuran-3-yl)-N-methyl-amino]-ethoxy}-7-cyclopentyloxy-quinazoline

(170) 4-[(3-chloro-4-fluoro-phenyl)amino]-6-{2-[N-(2-oxotetrahydxofuran-3-yl)-N-methyl-amino]-ethoxy}-7-cyclopentylmethoxy-quinazoline

(171) 4-[(3-chloro-4-fluoro-phenyl)amino]-6-[3-(6,6-dimethyl-2-oxo-morpholin-4-yl)-propyloxy]-7-methoxy-quinazoline

(172) 4-[(3-chloro-4-fluoro-phenyl)amino]-6-[3-(6,6-dimethyl-2-oxo-morpholin-4-yl)-propyloxy]-7-cyclopentyloxy-quinazoline

(173) 4-[(3-chloro-4-fluoro-phenyl)amino]-6-[3-(6,6-dimethyl-2-oxo-morpholin-4-yl)-propyloxy]-7-cyclopentylmethoxy-quinazoline

(174) (R)-4-[(1-phenyl-ethyl)amino]-6-[3-(6,6-dimethyl-2-oxomorpholin-4-yl)-propyloxy]-7-cyclopentyloxy-quinazoline

(175) 4-[(3-chloro-4-fluoro-phenyl)amino]-7-[2-(6,6-dimethyl-2-oxo-morpholin-4-yl)-ethoxy]-6-methoxy-quinazoline

(176) 4-[(3-chloro-4-fluoro-phenyl)amino]-7-[2-(6,6-dimethyl-2-oxo-morpholin-4-yl)-ethoxy]-6-cyclobutyloxy-quinazoline

(177) 4-[(3-chloro-4-fluoro-phenyl)amino]-7-[2-(6,6-dimethyl-2-oxo-morpholin-4-yl)-ethoxy]-6-cyclopentyloxy-quinazoline

(178) 4-[(3-chloro-4-fluoro-phenyl)amino]-7-[2-(6,6-dimethyl-2-oxo-morpholin-4-yl)-ethoxy]-6-cyclohexyloxy-quinazoline

(179) 4-[(3-chloro-4-fluoro-phenyl)amino]-7-[2-(6,6-dimethyl-2-oxo-morpholin-4-yl)-ethoxy]-6-cyclopropylmethoxy-quinazoline

(180) 4-[(3-chloro-4-fluoro-phenyl]amino]-7-[2-(6,6-dimethyl-2-oxo-moxpholin-4-yl)-ethoxy]-6-cyclopentylmethoxy-quinazoline

(181) 4-[(3-chloro-4-fluoro-phenyl)amino]-7-[2-(6,6-dimethyl-2-oxo-morpholin-4-yl)-ethoxy]-6-cyclohexylmethoxy-quinazoline

(182) 4-[(3-chloro-4-fluoro-phenyl)amino]-7-{2-[N-(2-oxotetrahydrofuran-4-yl)-N-methyl-amino]-ethoxy}-6-methoxyquinazoline

(183) 4-[(3-chloro-4-fluoro-phenyl)amino]-7-{2-[N-(2-oxotetrahydrofuran-4-yl)-N-methyl-amino]-ethoxy}-6-cyclopentyloxy-quinazoline

(184) 4-[(3-chloro-4-fluoro-phenyl)amino]-7-{2-[N-(2-oxotetrahydrofuran-4-yl)-N-methyl-amino]-ethoxy}-6-cyclopentylmethoxy-quinazoline

(185) 4-[(3-chloro-4-fluoro-phenyl)amino]-7-{2-(N-(2-oxotetrahydrofuran-3-yl)-N-methyl-amino]-ethoxy}-6-methoxy-quinazoline

(186) 4-[(3-chloro-4-fluoro-phenyl)amino]-7-{2-[N-(2-oxotetrahydrofuran-3-yl)-N-methyl-amino]-ethoxy}-6-cyclopentyloxy-quinazoline

(187) 4-[(3-chloro-4-fluoro-phenyl)amino]-7-{2-[N-(2-oxotetrahydrofuran-3-yl)-N-methyl-amino]-ethoxy}-6-cyclopentylmethoxy-quinazoline

(188) 4-[(3-chloro-4-fluoro-phenyl)amino]-7- [3- (6, 6-dimethyl-2-oxo-morpholin-4-yl)-propyloxy]-6-methoxy-quinazoline

(189) 4-[(3-chloro-4-fluoro-phenyl)amino]-7-[3-(6,6-dimethyl-2-oxo-morpholin-4-yl)-propyloxy]-6-cyclopentyloxy-quinazoline

(190) 4-[(3-chloro-4-fluoro-phenyl)amino]-7-[3-(6,6-dimethyl-2-oxo-morpholin-4-yl)-propyloxy]-6-cyclopentylmethoxy-quinazoline

(191) (R)-4-[(1-phenyl-ethyl)amino]-7-[3-(6,6-dimethyl-2-oxomorpholin-4-yl)-propyloxy]-6-cyclopentyloxy-quinazoline

Example 6

Coated tablets containing 75 mg of active substance

| 1 tablet core contains: | |
|---|---|
| active substance | 75.0 mg |
| calcium phosphate | 93.0 mg |
| corn starch | 35.5 mg |
| polyvinylpyrrolidone | 10.0 mg |
| hydroxypropylmethylcellulose | 15.0 mg |
| magnesium stearate | 1.5 mg |
| | 230.0 mg |

Preparation:

The active substance is mixed with calcium phosphate, corn starch, polyvinylpyrrolidone, hydroxypropylmethylcellulose and half the specified amount of magnesium stearate. Blanks 13 mm in diameter are produced in a tablet-making machine and these are then rubbed through a screen with a mesh size of 1.5 mm using a suitable machine and mixed with the rest of the magnesium stearate. This granulate is compressed in a tablet-making machine to form tablets of the desired shape.

| | |
|---|---|
| Weight of core | 230 mg |
| die | 9 mm, convex |

The tablet cores thus produced are coated with a film consisting essentially of hydroxypropylmethylcellulose. The finished film-coated tablets are polished with beeswax.

| | |
|---|---|
| Weight of coated tablet | 245 mg. |

Example 7

Tablets containing 100 mg of active substance

Composition:

| 1 tablet contains: | |
|---|---|
| active substance | 100.0 mg |
| lactose | 80.0 mg |
| corn starch | 34.0 mg |
| polyvinylpyrrolidone | 4.0 mg |
| magnesium stearate | 2.0 mg |
| | 220.0 mg |

Method of Preparation:

The active substance, lactose and starch are mixed together and uniformly moistened with an aqueous solution of the polyvinylpyrrolidone. After the moist composition has been screened (2.0 mm mesh size) and dried in a rack-type drier at 50°C it is screened again (1.5 mm mesh size) and the lubricant is added. The finished mixture is compressed to form tablets.

| | |
|---|---|
| Weight of tablet | 220 mg |
| Diameter | 10 mm, biplanar, facetted on both sides and notched on one side. |

Example 8

Tablets containing 150 mg of active substance

Composition:

| 1 tablet contains: | |
|---|---|
| active substance | 150.0 mg |
| powdered lactose | 89.0 mg |
| corn starch | 40.0 mg |
| colloidal silica | 10.0 mg |
| polyvinylpyrrolidone | 10.0 mg |
| magnesium stearate | 1.0 mg |
| | 300.0 mg |

Preparation:

The active substance mixed with lactose, corn starch and silica is moistened with a 20% aqueous polyvinylpyrrolidone solution and passed through a screen with a mesh size of 1.5 mm. The granules, dried at 45°C, are passed through the same screen again and mixed with the specified amount of magnesium stearate. Tablets are pressed from the mixture.

| | |
|---|---|
| Weight of tablet | 300 mg |
| die | 10 mm, flat |

Example 9

Hard gelatine capsules containing 150 mg of active substance

| 1 capsule contains: | | |
|---|---|---|
| active substance | | 150.0 mg |
| corn starch (dried | approx. | 180.0 mg |
| lactose (powdered) | approx. | 87.0 mg |
| magnesium stearate | | 3.0 mg |
| | approx. | 420.0 mg |

Preparation:

The active substance is mixed with the excipients, passed through a screen with a mesh size of 0.75 mm and homogeneously mixed using a suitable apparatus. The finished mixture is packed into size 1 hard gelatine capsules.

| | |
|---|---|
| Capsule filling | approx. 320 mg |
| Capsule shell | size 1 hard gelatine capsule. |

Example 10

Suppositories containing 150 mg of active substance

| 1 suppository contains: | |
|---|---|
| active substance | 150.0 mg |
| polyethyleneglycol 1500 | 550.0 mg |
| polyethyleneglycol 6000 | 460.0 mg |
| polyoxyethylene sorbitan monostearate | 840.0 mg |
| | 2,000.0 mg |

Preparation:

After the suppository mass has been melted the active substance is homogeneously distributed therein and the melt is poured into chilled moulds.

Example 11

Suspension containing 50 mg of active substance

| 100 ml of suspension contain: | |
|---|---|
| active substance | 1.00 g |
| carboxymethylcellulose-Na-salt | 0.10 g |
| methyl p-hydroxybenzoate | 0.05 g |
| propyl p-hydroxybenzoate | 0.01 g |
| glucose | 10.00 g |
| glycerol | 5.00 g |
| 70% sorbitol solution | 20.00 g |
| flavouring | 0.30 g |
| dist. water | ad 100 ml |

Preparation:

The distilled water is heated to 70°C. The methyl and propyl p-hydroxybenzoates together with the glycerol and sodium salt of carboxymethylcellulose are dissolved therein with stirring. The solution is cooled to ambient temperature and the active substance is added and homogeneously dispersed therein with stirring. After the sugar, the sorbitol solution and the flavouring have been added and dissolved, the suspension is evacuated with stirring to eliminate air.

5 ml of suspension contain 50 mg of active substance.

Example 12

Ampoules containing 10 mg active substance

Composition:

| | | |
|---|---|---|
| active substance | | 10.0 mg |
| 0.01 N hydrochloric acid q.s. | | |
| double-distilled water | ad | 2.0 ml |

Preparation:

The active substance is dissolved in the necessary amount of 0.01 N HCl, made isotonic with common salt, filtered sterile and transferred into 2 ml ampoules.

Example 13

Ampoules containing 50 mg nf active substance

Composition:

| | | |
|---|---|---|
| active substance | | 50.0 mg |
| 0.01 N hydrochloric acid q.s. | | |
| double-distilled water | ad | 10.0 ml |

Preparation:

The active substance is dissolved in the necessary amount of 0.01 N HCl, made isotonic with common salt, filtered sterile and transferred into 10 ml ampoules.

Example 14

Capsules for powder inhalation containing 5 mg of active substance

| 1 capsule contains: | |
|---|---|
| active substance | 5.0 mg |
| lactose for inhalation | 15.0 mg |
| | 20.0 mg |

Preparation:

The active substance is mixed with lactose for inhalation. The mixture is packed into capsules in a capsule-making machine (weight of the empty capsule approx. 50 mg).

| | |
|---|---|
| weight of capsule: | 70.0 mg |
| size of capsule | = 3 |

Example 15

Solution for inhalation for hand-held nebulisers containing 2.5 mg active substance

| 1 spray contains: | |
|---|---|
| active substance | 2.500 mg |
| benzalkonium chloride | 0.001 mg |
| 1N hydrochloric acid q.s. | |
| ethanol/water (50/50) | ad 15.000 mg |

Preparation:

The active substance and benzalkonium chloride are dissolved in ethanol/water (50/50). The pH of the solution is adjusted with 1N hydrochloric acid. The resulting solution is filtered and transferred into suitable containers for use in hand-held nebulisers (cartridges).
contents of the container: 4.5 g

## Claims

**1.** Bicyclic heterocycles of general formula

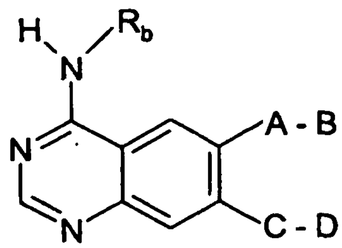

, (I)

wherein
$R_b$ denotes a phenyl, benzyl or 1-phenylethyl group wherein the phenyl nucleus is substituted in each case by the groups $R_1$ to $R_2$, whilst
$R_1$ and $R_2$, which may be identical or different, each denote a hydrogen, fluorine, chlorine or bromine atom, or a methyl, trifluoromethyl, methoxy, ethynyl or cyano group,
A denotes an -O-$C_{1-4}$-alkylene or -O-$CH_2$-CH(OH)-$CH_2$ group, whilst the oxygen atom of the abovementioned groups in each case is linked to the bicyclic heteroaromatic ring,
B denotes an $R_6$O-CO-alkylene-$NR_5$ group wherein the alkylene moiety, which is straight-chained and contains 1 or 2 carbon atoms, may additionally be substituted by an $R_6$O-CO or $R_6$O-CO-methyl group, whilst
$R_5$ denotes a hydrogen atom,
a $C_{1-2}$-alkyl group which may be substituted by an $R_6$O-CO group,
a $C_{2-4}$-alkyl group which is substituted from position 2 onwards by a hydroxy group,
a $C_{3-6}$-cycloalkyl or $C_{3-6}$-cycloalkylmethyl group and
$R_6$ denotes a hydrogen atom,
a $C_{1-6}$-alkyl, cyclopentyl, cyclopentylmethyl, cyclohexyl, cyclohexylmethyl, phenyl, benzyl, 5-indanyl or $R_g$-CO-O-($R_e$C$R_f$) group, whilst
$R_e$ denotes a hydrogen atom or a $C_{1-4}$-alkyl group,
$R_f$ denotes a hydrogen atom and
$R_g$ denotes a $C_{1-4}$-alkyl, cyclopentyl, cyclohexyl, $C_{1-4}$-alkoxy, cyclopentyloxy or cyclohexyloxy group,
a pyrrolidino or piperidino group which is substituted by an $R_6$O-CO or $R_6$O-CO-$C_{1-2}$-alkyl group wherein $R_6$ is as hereinbefore defined,
a pyrrolidino or piperidino group which is substituted by two $R_6$O-CO or $R_6$O-CO-$C_{1-2}$-alkyl groups wherein $R_6$ is as hereinbefore defined,
a piperazino group which is substituted in the 4 position by the group $R_{10}$ and additionally at a cyclic carbon atom by an $R_6$O-CO or $R_6$O-CO-$C_{1-2}$-alkyl group wherein $R_6$ is as hereinbefore defined and
$R_{10}$ denotes a hydrogen atom, a methyl or ethyl group,
a piperazino group which is substituted in the 4 position by an $R_6$O-CO-$C_{1-4}$-alkyl, bis- ($R_6$O-CO)-$C_{1-4}$-alkyl, ($R_7$O-PO-$OR_8$)-methyl or ($R_7$O-PO-$R_9$)-methyl group wherein $R_6$ is as hereinbefore defined,
$R_7$ and $R_8$, which may be identical or different, in each case denote a hydrogen atom, a methyl, ethyl, phenyl, benzyl, 5-indanyl or $R_g$CO-O-($R_e$C$R_f$) group, whilst
$R_e$ to $R_g$ are as hereinbefore defined,
and $R_9$ denotes a methyl or ethyl group,
a piperazino group which is substituted in the 4 position by an $R_6$O-CO-$C_{1-2}$-alkyl group and is additionally substituted at a cyclic carbon atom by an $R_6$O-CO or $R_6$O-CO-$C_{1-2}$-alkyl group wherein $R_6$ is as hereinbefore defined,
a morpholino group which is substituted by an $R_6$O-CO or $R_6$O-CO-$C_{1-2}$-alkyl group, whilst $R_6$ is as hereinbefore

defined,
a pyrrolidinyl or piperidinyl group substituted in the 1 position by an $R_6O$-CO-$C_{1-4}$-alkyl, bis-($R_6O$-CO)-$C_{1-4}$-alkyl, ($R_7O$-PO-$OR_8$)-methyl or ($R_7O$-PO-$R_9$)-methyl group wherein $R_6$ to $R_9$ are as hereinbefore defined,
a 2-oxo-morpholino group which may be substituted by 1 or 2 methyl groups,
a 2-oxo-morpholinyl group which is substituted in the 4 position by a methyl, ethyl or $R_6O$-CO-$C_{1-2}$-alkyl group, whilst $R_6$ is as hereinbefore defined and the abovementioned 2-oxo-morpholinyl groups in each case are linked to a carbon atom of the group A, or
a $R_{11}N(C_{1-2}$-alkyl) group wherein $R_{11}$ denotes a 2-oxo-tetrahydrofuran-3-yl or 2-oxo-tetrahydrofuran-4-yl group, and
C and D together denote a hydrogen atom, a methoxy, ethoxy, 2-methoxy-ethoxy, $C_{4-6}$-cycloalkoxy or $C_{3-6}$-cycloalkyl-$C_{1-3}$-alkoxy group,
the tautomers, the stereoisomers and the salts thereof.

**2.** Bicyclic heterocycles of general formula I, wherein
$R_b$ denotes a phenyl, benzyl or 1-phenylethyl group wherein the phenyl nucleus is substituted in each case by the groups $R_1$ to $R_2$, whilst
$R_1$ and $R_2$, which may be identical or different, each denote a hydrogen, fluorine, chlorine or bromine atom, or a methyl, trifluoromethyl, methoxy, ethynyl or cyano group,
A and B together denote a hydrogen atom, a methoxy, ethoxy, 2-methoxy-ethoxy, $C_{4-6}$-cycloalkoxy or $C_{3-6}$-cycloalkyl-$C_{1-3}$-alkoxy group,
C denotes an -O-$C_{1-4}$-alkylene or -O-$CH_2$-CH(OH)-$CH_2$ group, whilst the oxygen atom of the abovementioned groups in each case is linked to the bicyclic heteroaromatic ring, and
D denotes an $R_6O$-CO-alkylene-$NR_5$ group wherein the alkylene moiety, which is straight-chained and contains 1 or 2 carbon atoms, may additionally be substituted by an $R_6O$-CO or $R_6O$-CO-methyl group, whilst
$R_5$ denotes a hydrogen atom,
a $C_{1-2}$-alkyl group which may be substituted by an $R_6O$-CO group,
a $C_{2-4}$-alkyl group which is substituted from position 2 by a hydroxy group,
a $C_{3-6}$-cycloalkyl or $C_{3-6}$-cycloalkylmethyl group and
$R_6$ denotes a hydrogen atom,
a $C_{1-6}$-alkyl, cyclopentyl, cyclopentylmethyl, cyclohexyl, cyclohexylmethyl, phenyl, benzyl, 5-indanyl or $R_g$-CO-O-($R_eCR_f$) group, whilst
$R_e$ denotes a hydrogen atom or a $C_{1-4}$-alkyl group,
$R_f$ denotes a hydrogen atom and
$R_g$ denotes a $C_{1-4}$-alkyl, cyclopentyl, cyclohexyl, $C_{1-4}$-alkoxy, cyclopentyloxy or cyclohexyloxy group,
a pyrrolidino or piperidino group which is substituted by an $R_6O$-CO or $R_6O$-CO-$C_{1-2}$-alkyl group wherein $R_6$ is as hereinbefore defined,
a pyrrolidino or piperidino group which is substituted by two $R_6O$-CO or $R_6O$-CO-$C_{1-2}$-alkyl groups wherein $R_6$ is as hereinbefore defined,
a piperazino group which is substituted in the 4 position by the group $R_{10}$ and additionally at a cyclic carbon atom by an $R_6O$-CO or $R_6O$-CO-$C_{1-2}$-alkyl group wherein $R_6$ is as hereinbefore defined and
$R_{10}$ denotes a hydrogen atom, a methyl or ethyl group,
a piperazino group which is substituted in the 4 position by an $R_6O$-CO-$C_{1-4}$-alkyl, bis- ($R_6O$-CO)-$C_{1-4}$-alkyl, ($R_7O$-PO-$OR_8$)-methyl or ($R_7O$-PO-$R_9$)-methyl group wherein $R_6$ is as hereinbefore defined,
$R_7$ and $R_8$, which may be identical or different, in each case denote a hydrogen atom, a methyl, ethyl, phenyl, benzyl, 5-indanyl or $R_g$CO-O-($R_eCR_f$) group, whilst
$R_e$ to $R_g$ are as hereinbefore defined,
and $R_9$ denotes a methyl or ethyl group,
a piperazino group which is substituted in the 4 position by an $R_6O$-CO-$C_{1-2}$-alkyl group and is additionally substituted at a cyclic carbon atom by an $R_6O$-CO or $R_6O$-CO-$C_{1-2}$-alkyl group wherein $R_6$ is as hereinbefore defined,
a morpholino group which is substituted by an $R_6O$-CO or $R_6O$-CO-$C_{1-2}$-alkyl group, whilst $R_6$ is as hereinbefore defined,
a pyrrolidinyl or piperidinyl group substituted in the 1 position by an $R_6O$-CO-$C_{1-4}$-alkyl, bis-($R_6O$-CO)-$C_{1-4}$-alkyl, ($R_7O$-PO-$OR_8$)-methyl or ($R_7O$-PO-$R_9$)-methyl group wherein $R_6$ to $R_9$ are as hereinbefore defined,
a 2-oxo-morpholino group which may be substituted by 1 or 2 methyl groups.,
a 2-oxo-morpholinyl group which is substituted in the 4 position by a methyl, ethyl or $R_6O$-CO-$C_{1-2}$-alkyl group, whilst $R_6$ is as hereinbefore defined and the abovementioned 2-oxo-morpholinyl groups are in each case linked to a carbon atom of the group C, or
a $R_{11}N(C_{1-2}$-alkyl) group wherein $R_{11}$ denotes a 2-oxo-tetrahydrofuran-3-yl or 2-oxo-tetrahydrofuran-4-yl group,

the tautomers, stereoisomers and salts thereof.

**3.** Bicyclic heterocycles of general formula I according to claim 1, wherein
$R_b$ denotes a phenyl group wherein the phenyl nucleus is substituted in each case by the groups $R_1$ to $R_2$, whilst
$R_1$ and $R_2$, which may be identical or different, each denote a hydrogen, fluorine, chlorine or bromine atom,
A denotes an $-O-C_{1-4}$-alkylene or $-O-CH_2-CH(OH)-CH_2$ group, whilst the oxygen atom of the abovementioned groups in each case is linked to the bicyclic heteroaromatic ring,
B denotes an $R_6O-CO-CH_2-NR_5$ group wherein
$R_5$ denotes a hydrogen atom or a methyl group which may be substituted by an $R_6O-CO$ group, or
a $C_{2-4}$-alkyl group substituted from position 2 onwards by a hydroxy group, and
$R_6$ denotes a hydrogen atom, a methyl or ethyl group,
a pyrrolidino or piperidino group which is substituted by an $R_6O-CO$ group, whilst $R_6$ is as hereinbefore defined,
a piperazino group which is substituted in the 4 position by an $R_6O-CO-CH_2$ or bis-$(R_6O-CO)-C_{1-3}$-alkyl group, whilst $R_6$ is as hereinbefore defined,
a pyrrolidinyl or piperidinyl group substituted in the 1 position by an $R_6O-CO-CH_2$ group, whilst $R_6$ is as hereinbefore defined,
a 2-oxo-morpholino group which may be substituted by one or two methyl groups, or
a $R_{11}N(C_{1-2}$-alkyl) group wherein $R_{11}$ denotes a 2-oxo-tetrahydrofuran-3-yl or 2-oxo-tetrahydrofuran-4-yl group, and
C and D together denote a methoxy, $C_{4-6}$-cycloalkoxy or $C_{3-6}$-cycloalkylmethoxy group,
the tautomers, stereoisomers and salts thereof.

**4.** Bicyclic heterocycles of general formula I according to claim 2, wherein
$R_b$ denotes a phenyl group wherein the phenyl nucleus is substituted in each case by the groups $R_1$ to $R_2$, whilst
$R_1$ and $R_2$, which may be identical or different, each denote a hydrogen, fluorine, chlorine or bromine atom,
A and B together denote a $C_{4-6}$-cycloalkoxy or $C_{3-6}$-cycloalkylmethoxy group,
C denotes an $-O-CH_2CH_2$ group, whilst the oxygen atom of the abovementioned group is linked to the bicyclic heteroaromatic ring,
D denotes an $R_6O-CO-CH_2-NR_5$ group wherein
$R_5$ denotes a $C_{2-4}$-alkyl group substituted from position 2 onwards by a hydroxy group, and
$R_6$ denotes a methyl or ethyl group,
a 2-oxo-morpholino group which may be substituted by one or two methyl groups, or
a $R_{11}N(C_{1-2}$-alkyl) group wherein $R_{11}$ denotes a 2-oxo-tetrahydrofuran-3-yl or 2-oxo-tetrahydrofuran-4-yl group,
the tautomers, stereoisomers and salts thereof.

**5.** The following bicyclic heterocycles of general formula I according to claim 1 or 2:

(1) 4-(3-chloro-4-fluorophenylamino)-6-{3-[4-(methoxycarbonylmethyl)-1-piperazinyl]propyloxy}-7-methoxy-quinazoline,

(2) 4-[(3-bromophenyl)amino]-6-(2-{4-[(ethoxycarbonyl)methyl]-piperazin-1-yl}ethoxy)-7-methoxy-quinazoline,

(3) (*S*)-4-[(3-bromophenyl)amino]-6-[3-(2-methoxycarbonylpyrrolidin-1-yl)propyloxy]-7-methoxy-quinazoline,

(4) 4-[(3-bromophenyl)amino]-6-(3-{4-[(ethoxycarbonyl)methyl]-piperazin-1-yl}-2-hydroxy-propyloxy)-7-methoxy-quinazoline,

(5) (*S*)-4-[(3-bromophenyl)amino]-6-({1-[(ethoxycarbonyl)methyl]-pyrrolidine-2-yl}methoxy)-7-methoxy-quinazoline and

(6) 4-[(3-bromophenyl)amino]-6-(2-{4-[1,2-bis(methoxycarbonyl)ethyl]-piperazin-1-yl}ethoxy)-7-methoxy-quinazoline

and the salts thereof.

**6.** Physiologically acceptable salts of the compounds according to claims 1 to 5 with inorganic or organic acids of bases.

**7.** Pharmaceutical compositions containing a compound according to claims 1 to 5 or a physiologically acceptable salt according to claim 6 optionally together with one or more inert carriers and/or diluents.

**8.** Use of a compound according to at least one of claims 1 to 6 for preparing a pharmaceutical compositions which is suitable for treating benign or malignant tumours, for preventing and treating diseases of the airways and lungs, for treating polyps, diseases of the gastro-intestinal tract, the bile ducts and gall bladder and the kidneys and skin.

**9.** Process for preparing a pharmaceutical composition according to claim 7, **characterised in that** a compound according to at least one of claims 1 to 6 is incorporated in one or more inert carriers and/or diluents by a non-chemical method.

**10.** Process for preparing the compounds of general formula I according to claims 1 to 6, **characterised in that**

a) a compound of general formula

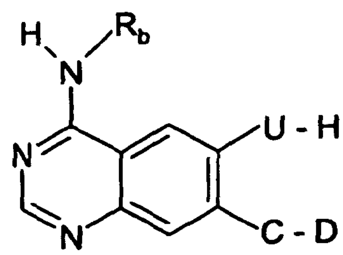

, (II)

wherein
$R_b$, C and D are defined as in claims 1 to 5 and
U denotes an oxygen atom or an $R_4N$ group, whilst $R_4$ is defined as in claims 1 to 5, is reacted with a compound of general formula

$$Z_1 - A' - B , \qquad (III)$$

wherein
B is defined as in claims 1 to 5,
A' denotes one of the optionally substituted alkylene, cycloalkylene, alkylene-cycloalkylene, cycloalkylene-alkylene or alkylene-cycloalkylene-alkylene moieties mentioned in claims 1 to 5 for the group A, which are linked to the heteroaromatic group via an oxygen atom or via an $NR_4$ group, and $Z_1$ denotes a leaving group, or
b) a compound of general formula

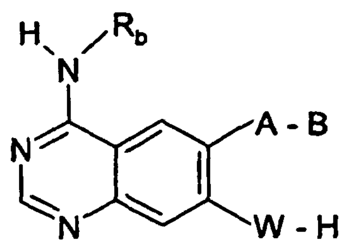

, (IV)

wherein
$R_b$, A and B are defined as in claims 1 to 5 and W denotes an oxygen atom or an $R_4N$ group, whilst $R_4$ is defined as in claims 1 to 5, is reacted with a compound of general formula

$$Z_2 - C' - D , \qquad (V)$$

wherein
D is defined as in claims 1 to 5,
C' denotes one of the optionally substituted alkylene, cycloalkylene, alkylene-cycloalkylene, cycloalkylene-alkylene or alkylene-cycloalkylene-alkylene moieties mentioned above for the group C, which are linked to the heteroaromatic group via an oxygen atom or via an $NR_4$ group, and $Z_2$ denotes a leaving group, or
c) in order to prepare a compound of general formula I wherein A is defined as in claims 1 to 5 with the exception of the oxygen atom and the -$NR_4$ group:

a compound of general formula

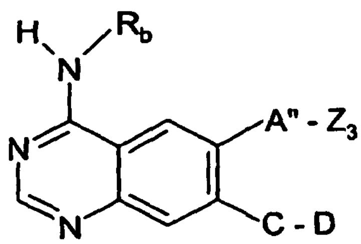
, (VI)

wherein
$R_b$, C and D are defined as in claims 1 to 5 and
A" has the meanings given for A in claims 1 to 5 with the exception of the oxygen atom and the -$NR_4$ group and $Z_3$ denotes a leaving group or together with a hydrogen atom of an adjacent hydrocarbon group denotes an oxygen atom, is reacted with a compound of general formula

$$H - B , \qquad (VII)$$

wherein
B is defined as in claims 1 to 5, or
d) in order to prepare a compound of general formula I wherein C is defined as in claims 1 to 5 with the exception of the oxygen atom and the -$NR_4$ group:

a compound of general formula

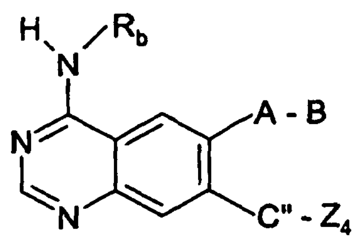
, (VIII)

wherein
$R_b$, A and B are defined as in claims 1 to 5 and
C" has the meanings given for C in claims 1 to 5 with the exception of the oxygen atom and the -$NR_4$ group and $Z_4$ denotes a leaving group or together with a hydrogen atom of an adjacent hydrocarbon group denotes an oxygen atom, is reacted with a compound of general formula

H - D , (IX)

wherein
D is defined as in claims 1 to 5, or
e) in order to prepare a compound of general formula I wherein B denotes an $R_6$O-CO-alkylene-$NR_5$ group wherein the alkylene moiety, which is straight-chained and contains 1 to 6 carbon atoms, may additionally be substituted by one or two $C_{1-2}$-alkyl groups or by an $R_6$O-CO or $R_6$O-CO-$C_{1-2}$-alkyl group, a piperazino or homopiperazino group substituted in the 4 position by an $R_6$O-CO-$C_{1-4}$-alkyl or bis-($R_6$O-CO)-$C_{1-4}$-alkyl group or a pyrrolidinyl, piperidinyl or hexahydroazepinyl group substituted in the 1 position by an $R_6$O-CO-$C_{1-4}$-alkyl or bis-($R_6$O-CO)-$C_{1-4}$-alkyl group, whilst in each case $R_5$ and $R_6$ are defined as in claims 1 to 5:

a compound of general formula

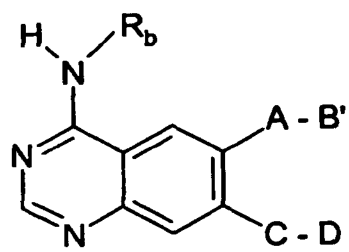
, (X)

wherein
$R_b$, A, C and D are defined as in claims 1 to 5 and
B' denotes an $R_5$NH group wherein $R_5$ is defined as in claims 1 to 5, a piperazino or homopiperazino group unsubstituted in the 4 position, a pyrrolidinyl, piperidinyl or hexahydroazepinyl group unsubstituted in the 1 position, is reacted with a compound of general formula

$R_6$O-CO-alkylene-$Z_5$ , (XI)

wherein
the alkylene moiety, which is straight-chained and contains 1 to 6 carbon atoms, may additionally be substituted by one or two $C_{1-2}$-alkyl groups or by an $R_6$O-CO or $R_6$O-CO-$C_{1-2}$-alkyl group, whilst $R_6$ in each case is defined as in claims 1 to 5, and $Z_5$ denotes an exchangeable group, or
f) in order to prepare a compound of general formula I wherein D denotes an $R_6$O-CO-alkylene-$NR_5$ group wherein the alkylene moiety, which is straight-chained and contains 1 to 6 carbon atoms, may additionally be substituted by one or two $C_{1-2}$-alkyl groups or by an $R_6$O-CO or $R_6$O-CO-$C_{1-2}$-alkyl group, a piperazino or homopiperazino group substituted in the 4 position by an $R_6$O-CO-$C_{1-4}$-alkyl or bis-($R_6$O-CO)-$C_{1-4}$-alkyl group or a pyrrolidinyl, piperidinyl or hexahydroazepinyl group substituted in the 1 position by an $R_6$O-CO-$C_{1-4}$-alkyl or bis-($R_6$O-CO)-$C_{1-4}$-alkyl group, whilst in each case $R_5$ and $R_6$ are defined as in claims 1 to 5:

a compound of general formula

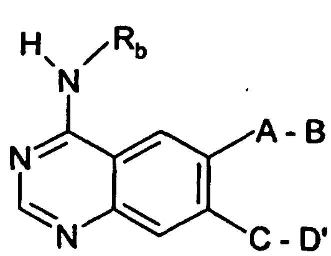
, (XII)

wherein
$R_b$, A, B and C are defined as in claims 1 to 5 and
D' denotes an $R_5NH$ group wherein $R_5$ is defined as in claims 1 to 5, a piperazino or homopiperazino group unsubstituted in the 4 position, a pyrrolidinyl, piperidinyl or hexahydroazepinyl group unsubstituted in the 1 position, is reacted with a compound of general formula

$$R_6O\text{-}CO\text{-alkylene-}Z_5 \ , \qquad (XI)$$

wherein
the alkylene moiety, which is straight-chained and contains 1 to 6 carbon atoms, may additionally be substituted by one or two $C_{1-2}$-alkyl groups or by an $R_6O$-CO or $R_6O$-CO-$C_{1-2}$-alkyl group, whilst $R_6$ in each case is defined as in claims 1 to 5, and $Z_5$ denotes an exchangeable group, or
g) in order to prepare a compound of general formula I wherein at least one of the groups $R_6$ to $R_8$ denotes a hydrogen atom:

a compound of general formula

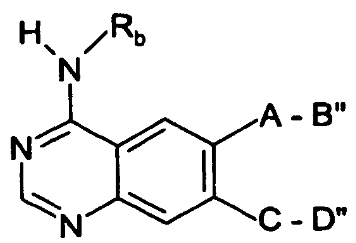

, (XIII)

wherein
$R_b$, A and C are defined as in claims 1 to 5,
B" and D" have the meanings given for B and D in claims 1 to 5, with the proviso that at least one of the groups B" or D" contains an $R_6O$-CO, ($R_7O$-PO-$OR_8$) or ($R_7O$-PO-$R_9$) group wherein $R_9$ is defined as in claims 1 to 5 and at least one of the groups $R_6$ to $R_8$ does not represent a hydrogen atom, is converted by hydrolysis, treating with acids, thermolysis or hydrogenolysis into a compound of general formula I wherein at least one of the groups $R_6$ to $R_8$ denotes a hydrogen atom,
and subsequently, if desired, a compound of general formula I thus obtained which contains a carboxy or hydroxyphosphoryl group is converted by esterification into a corresponding ester of general formula I and/or
a compound of general formula I thus obtained wherein B or D denotes an optionally substituted N-(2-hydroxyethyl)-glycine or N-(2-hydroxyethyl)-glycine ester group is converted by cyclisation into a corresponding 2-oxo-morpholino compound, and/or
if necessary any protecting group used during the reactions described above is cleaved again and/or
if desired a compound of general formula I thus obtained is resolved into its stereoisomers and/or
a compound of general formula I thus obtained is converted into the salts thereof, more particularly, for pharmaceutical use, into the physiologically acceptable salts thereof.

**Patentansprüche**

**1.** Bicyclische Heterocyclen der allgemeinen Formel

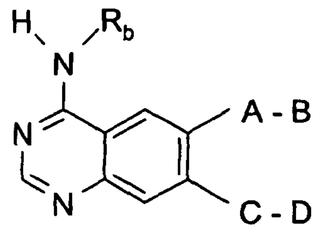

, (I)

in der

$R_b$ eine Phenyl-, Benzyl- oder 1-Phenylethylgruppe, in denen der Phenylkern jeweils durch die Reste $R_1$ bis $R_2$ substituiert ist, wobei

$R_1$ und $R_2$, die gleich oder verschieden sein können, jeweils ein Wasserstoff-, Fluor-, Chlor- oder Bromatom, oder eine Methyl-, Trifluormethyl-, Methoxy-, Ethinyl- oder Cyanogruppe bedeuten,

A eine -O-$C_{1-4}$-Alkylen- oder -O-$CH_2$-CH(OH)-$CH_2$-Gruppe, wobei das Sauerstoffatom der vorstehend erwähnten Gruppen jeweils mit dem bicyclischen Heteroaromaten verknüpft ist,

B eine $R_6$O-CO-alkylen-$NR_5$-Gruppe, in der der Alkylenteil, welcher geradkettig ist und 1 oder 2 Kohlenstoffatome enthält, zusätzlich durch eine $R_6$O-CO- oder $R_6$O-CO-methylgruppe substituiert sein kann, wobei

$R_5$ ein Wasserstoffatom,

eine $C_{1-2}$-Alkylgruppe, die durch eine $R_6$O-CO-Gruppe substituiert sein kann,

eine $C_{2-4}$-Alkylgruppe, die ab Position 2 durch eine Hydroxygruppe substituiert ist,

eine $C_{3-6}$-Cycloalkyl- oder $C_{3-6}$-Cycloalkylmethylgruppe und

$R_6$ ein Wasserstoffatom,

eine $C_{1-6}$-Alkyl-, Cyclopentyl-, Cyclopentylmethyl-, Cyclohexyl-, Cyclohexylmethyl-, Phenyl-, Benzyl-, 5-Indanyl- oder $R_g$CO-O-($R_e$C$R_f$)-Gruppe darstellen, wobei

$R_e$ ein Wasserstoffatom oder eine $C_{1-4}$-Alkylgruppe,

$R_f$ ein Wasserstoffatom und

$R_g$ eine $C_{1-4}$-Alkyl-, Cyclopentyl-, Cyclohexyl-, $C_{1-4}$-Alkoxy-, Cyclopentyloxy- oder Cyclohexyloxygruppe darstellen,

eine Pyrrolidino- oder Piperidinogruppe, die durch eine $R_6$O-CO-oder $R_6$O-CO-$C_{1-2}$-alkylgruppe substituiert ist, in denen $R_6$ wie vorstehend erwähnt definiert ist,

eine Pyrrolidino- oder Piperidinogruppe, die durch zwei $R_6$O-CO- oder $R_6$O-CO-$C_{1-2}$-alkylgruppen substituiert ist, in denen $R_6$ wie vorstehend erwähnt definiert ist,

eine Piperazinogruppe, die in 4-Stellung durch den Rest $R_{10}$ und zusätzlich an einem Ringkohlenstoffatom durch eine $R_6$O-CO- oder $R_6$O-CO-$C_{1-2}$-alkylgruppe substituiert ist, in denen $R_6$ wie vorstehend erwähnt definiert ist und

Rio ein Wasserstoffatom, eine Methyl- oder Ethylgruppe darstellt,

eine Piperazinogruppe, die in 4-Stellung durch eine $R_6$O-CO-$C_{1-4}$-alkyl-, Bis-($R_6$O-CO)-$C_{1-4}$-alkyl-, ($R_7$O-PO-O$R_8$)-methyl- oder ($R_7$O-PO-$R_9$)-methylgruppe substituiert ist, in denen $R_6$ wie vorstehend erwähnt definiert ist,

$R_7$ und $R_8$, die gleich oder verschieden sein können, jeweils ein Wasserstoffatom, eine Methyl-, Ethyl-, Phenyl-, Benzyl-, 5-Indanyl- oder $R_g$CO-O-($R_e$C$R_f$)-Gruppe, wobei

$R_e$ bis $R_g$ wie vorstehend erwähnt definiert sind,

und $R_9$ eine Methyl- oder Ethylgruppe darstellen,

eine Piperazinogruppe, die in 4-Stellung durch eine $R_6$O-CO-$C_{1-2}$-alkylgruppe und zusätzlich an einem Ringkohlenstoffatom durch eine $R_6$O-CO- oder $R_6$O-CO-$C_{1-2}$-alkylgruppe substituiert ist, in denen $R_6$ wie vorstehend erwähnt definiert ist,

eine Morpholinogruppe, die durch eine $R_6$O-CO- oder $R_6$O-CO-$C_{1-2}$-alkylgruppe substituiert ist, wobei $R_6$ wie vorstehend erwähnt definiert ist,

eine in 1-Stellung durch eine $R_6$O-CO-$C_{1-4}$-alkyl-, Bis-($R_6$O-CO)-$C_{1-4}$-alkyl-, ($R_7$O-PO-O$R_8$)-methyl- oder ($R_7$O-PO-$R_9$)-methylgruppe substituierte Pyrrolidinyl- oder Piperidinylgruppe, in denen $R_6$ bis $R_9$ wie vorstehend erwähnt definiert sind,

eine 2-Oxo-morpholinogruppe, die durch 1 oder 2 Methylgruppen substituiert sein kann,

eine 2-Oxo-morpholinylgruppe, die in 4-Stellung durch eine Methyl-, Ethyl- oder $R_6$O-CO-$C_{1-2}$-alkyl-Gruppe substituiert ist,

wobei $R_6$ wie vorstehend erwähnt definiert ist und die vorstehend erwähnten 2-Oxo-morpholinylgruppen jeweils mit einem Kohlenstoffatom der Gruppe A verknüpft sind, oder

eine $R_{11}$N($C_{1-2}$-Alkyl) Gruppe, in der $R_{11}$ eine 2-Oxo-tetrahydrofuran-3-yl oder 2-Oxo-tetrahydrofuran-4-yl Gruppe

darstellt, und
C und D zusammen ein Wasserstoffatom, eine Methoxy-, Ethoxy-, 2-Methoxy-ethoxy-, $C_{4-6}$-Cycloalkoxy- oder $C_{3-6}$-Cycloalkyl-$C_{1-3}$-alkoxygruppe bedeuten,
deren Tautomeren, deren Stereoisomere und deren Salze.

**2.** Bicyclische Heterocyclen der allgemeinen Formel I, in der
$R_b$ eine Phenyl-, Benzyl- oder 1-Phenylethylgruppe, in denen der Phenylkern jeweils durch die Reste $R_1$ bis $R_2$ substituiert ist, wobei
$R_1$ und $R_2$, die gleich oder verschieden sein können, jeweils ein Wasserstoff-, Fluor-, Chlor- oder Bromatom, oder eine Methyl-, Trifluormethyl-, Methoxy-, Ethinyl- oder Cyanogruppe bedeuten,
A und B zusammen ein Wasserstoffatom, eine Methoxy-, Ethoxy-, 2-Methoxy-ethoxy-, $C_{4-6}$-Cycloalkoxy- oder $C_{3-6}$-Cycloalkyl-$C_{1-3}$-alkoxygruppe,
C eine -O-$C_{1-4}$-Alkylen- oder -O-$CH_2$-CH(OH)-$CH_2$-Gruppe, wobei das Sauerstoffatom der vorstehend erwähnten Gruppen jeweils mit dem bicyclischen Heteroaromaten verknüpft ist, und
D eine $R_6$O-CO-alkylen-$NR_5$-Gruppe, in der der Alkylenteil, welcher geradkettig ist und 1 oder 2 Kohlenstoffatome enthält, zusätzlich durch eine $R_6$O-CO- oder $R_6$O-CO-methylgruppe substituiert sein kann, wobei
$R_5$ ein Wasserstoffatom,
eine $C_{1-2}$-Alkylgruppe, die durch eine $R_6$O-CO-Gruppe substituiert sein kann,
eine $C_{2-4}$-Alkylgruppe, die ab Position 2 durch eine Hydroxygruppe substituiert ist,
eine $C_{3-6}$-Cycloalkyl- oder $C_{3-6}$-Cycloalkylmethylgruppe und
$R_6$ ein Wasserstoffatom,
eine $C_{1-6}$-Alkyl-, Cyclopentyl-, Cyclopentylmethyl-, Cyclohexyl-, Cyclohexylmethyl-, Phenyl-, Benzyl-, 5-Indanyl- oder $R_g$CO-O-($R_e$C$R_f$)-Gruppe darstellen, wobei
$R_e$ ein Wasserstoffatom oder eine $C_{1-4}$-Alkylgruppe,
$R_f$ ein Wasserstoffatom und
$R_g$ eine $C_{1-4}$-Alkyl-, Cyclopentyl-, Cyclohexyl-, $C_{1-4}$-Alkoxy-, Cyclopentyloxy- oder Cyclohexyloxygruppe darstellen,
eine Pyrrolidino- oder Piperidinogruppe, die durch eine $R_6$O-CO- oder $R_6$O-CO-$C_{1-2}$-alkylgruppe substituiert ist, in denen $R_6$ wie vorstehend erwähnt definiert ist,
eine Pyrrolidino- oder Piperidinogruppe, die durch zwei $R_6$O-CO- oder $R_6$O-CO-$C_{1-2}$-alkylgruppen substituiert ist, in denen $R_6$ wie vorstehend erwähnt definiert ist,
eine Piperazinogruppe, die in 4-Stellung durch den Rest $R_{10}$ und zusätzlich an einem Ringkohlenstoffatom durch eine $R_6$O-CO- oder $R_6$O-CO-$C_{1-2}$-alkylgruppe substituiert ist, in denen $R_6$ wie vorstehend erwähnt definiert ist und
$R_{10}$ ein Wasserstoffatom, eine Methyl- oder Ethylgruppe darstellt,
eine Piperazinogruppe, die in 4-Stellung durch eine $R_6$O-CO-$C_{1-4}$-alkyl-, Bis-($R_6$O-CO)-$C_{1-4}$-alkyl-, ($R_7$O-PO-O$R_8$)-methyl- oder ($R_7$O-PO-$R_9$)-methylgruppe substituiert ist, in denen $R_6$ wie vorstehend erwähnt definiert ist,
$R_7$ und $R_8$, die gleich oder verschieden sein können, jeweils ein Wasserstoffatom, eine Methyl-, Ethyl-, Phenyl-, Benzyl-, 5-Indanyl- oder $R_g$CO-O-($R_e$C$R_f$)-Gruppe, wobei
$R_e$ bis $R_g$ wie vorstehend erwähnt definiert sind,
und $R_9$ eine Methyl- oder Ethylgruppe darstellen,
eine Piperazinogruppe, die in 4-Stellung durch eine $R_6$O-CO-$C_{1-2}$-alkylgruppe und zusätzlich an einem Ringkohlenstoffatom durch eine $R_6$O-CO- oder $R_6$O-CO-$C_{1-2}$-alkylgruppe substituiert ist, in denen $R_6$ wie vorstehend erwähnt definiert ist,
eine Morpholinogruppe, die durch eine $R_6$O-CO- oder $R_6$O-CO-$C_{1-2}$-alkylgruppe substituiert ist, wobei $R_6$ wie vorstehend erwähnt definiert ist,
eine in 1-Stellung durch eine $R_6$O-CO-$C_{1-4}$-alkyl-, Bis-($R_6$O-CO)-$C_{1-4}$-alkyl-, ($R_7$O-PO-O$R_8$)-methyl- oder ($R_7$O-PO-$R_9$)-methylgruppe substituierte Pyrrolidinyl- oder Piperidinylgruppe, in denen $R_6$ bis $R_9$ wie vorstehend erwähnt definiert sind,
eine 2-Oxo-morpholinogruppe, die durch 1 oder 2 Methylgruppen substituiert sein kann,
eine 2-Oxo-morpholinylgruppe, die in 4-Stellung durch eine Methyl-, Ethyl- oder $R_6$O-CO-$C_{1-2}$-alkyl-Gruppe substituiert ist, wobei $R_6$ wie vorstehend erwähnt definiert ist und die vorstehend erwähnten 2-Oxo-morpholinylgruppen jeweils mit einem Kohlenstoffatom der Gruppe C verknüpft sind, oder
eine $R_{11}$N($C_{1-2}$-Alkyl) Gruppe, in der $R_{11}$ eine 2-Oxo-tetrahydrofuran-3-yl oder 2-Oxo-tetrahydrofuran-4-yl Gruppe darstellt, bedeuten,
deren Tautomeren, deren Stereoisomere und deren Salze.

**3.** Bicyclische Heterocyclen der allgemeinen Formel I gemäß Anspruch 1, in der

$R_b$ eine Phenylgruppe, in denen der Phenylkern jeweils durch die Reste $R_1$ bis $R_2$ substituiert ist, wobei

$R_1$ und $R_2$, die gleich oder verschieden sein können, jeweils ein Wasserstoff-, Fluor-, Chlor- oder Bromatom bedeuten,

A eine $-O-C_{1-4}$-Alkylen- oder $-O-CH_2-CH(OH)-CH_2$-Gruppe, wobei das Sauerstoffatom der vorstehend erwähnten Gruppen jeweils mit dem bicyclischen Heteroaromaten verknüpft ist,

B eine $R_6O-CO-CH_2-NR_5$-Gruppe, in der

$R_5$ ein Wasserstoffatom oder eine Methylgruppe, die durch eine $R_6O-CO$-Gruppe substituiert sein kann, oder eine $C_{2-4}$-Alkylgruppe, die ab Position 2 durch eine Hydroxygruppe substituiert ist, und

$R_6$ ein Wasserstoffatom, eine Methyl- oder Ethylgruppe darstellen,

eine Pyrrolidino- oder Piperidinogruppe, die durch eine $R_6O-CO$-Gruppe substituiert ist, wobei $R_6$ wie vorstehend erwähnt definiert ist,

eine Piperazinogruppe, die in 4-Stellung durch eine $R_6O-CO-CH_2$- oder Bis-$(R_6O-CO)-C_{1-3}$-alkylgruppe substituiert ist, wobei $R_6$ wie vorstehend erwähnt definiert ist,

eine in 1-Stellung durch eine $R_6O-CO-CH_2$-Gruppe substituierte Pyrrolidinyl- oder Piperidinylgruppe, wobei $R_6$ wie vorstehend erwähnt definiert ist,

eine 2-Oxo-morholinogruppe, die durch ein oder zwei Methylgruppen substituiert sein kann, oder

eine $R_{11}N(C_{1-2}$-Alkyl) Gruppe, in der $R_{11}$ eine 2-Oxo-tetrahydrofuran-3-yl oder 2-Oxo-tetrahydrofuran-4-yl Gruppe darstellt, und

C und D zusammen eine Methoxy-, $C_{4-6}$-Cycloalkoxy- oder $C_{3-6}$-Cycloalkylmethoxygruppe bedeuten,

deren Tautomeren, deren Stereoisomere und deren Salze.

**4.** Bicyclische Heterocyclen der allgemeinen Formel I gemäß Anspruch 2, in der

$R_b$ eine Phenylgruppe, in der der Phenylkern jeweils durch die Reste $R_1$ bis $R_2$ substituiert ist, wobei

$R_1$ und $R_2$, die gleich oder verschieden sein können, jeweils ein Wasserstoff-, Fluor-, Chlor- oder Bromatom bedeuten,

A und B zusammen eine $C_{4-6}$-Cycloalkoxy- oder $C_{3-6}$-Cycloalkyl-$C_{1-3}$-methoxygruppe,

C eine $-O-CH_2-CH_2$ Gruppe, wobei das Sauerstoffatom der vorstehend erwähnten Gruppen jeweils mit dem bicyclischen Heteroaromaten verknüpft ist,

D eine $R_6O-CO-CH_2-NR_5$ Gruppe, in der

$R_5$ eine $C_{2-4}$-Alkylgruppe, die ab Position 2 durch eine Hydroxygruppe substituiert ist, und

$R_6$ eine Methyl- oder Ethylgruppe darstellen,

eine 2-Oxo-morpholinogruppe, die durch 1 oder 2 Methylgruppen substituiert sein kann, oder

eine $R_{11}N(C_{1-2}$-Alkyl) Gruppe, in der $R_{11}$ eine 2-Oxo-tetrahydrofuran-3-yl oder 2-Oxo-tetrahydrofuran-4-yl Gruppe darstellt, bedeuten,

deren Tautomeren, deren Stereoisomere und deren Salze.

**5.** Folgende bicyclische Heterocyclen der allgemeinen Formel I gemäß Anspruch 1 oder 2:

(1) 4-(3-Chlor-4-fluorphenylamino)-6-{3-[4-(methoxycarbonylmethyl)-1-piperazinyl]propyloxy}-7-methoxy-chinazolin,

(2) 4-[(3-Bromphenyl)amino]-6-(2-{4-[(ethoxycarbonyl)methyl]-piperazin-1-yl}ethoxy)-7-methoxy-chinazolin,

(3) (*S*)-4-[(3-Bromphenyl)amino]-6-[3-(2-methoxycarbonyl-pyrrolidin-1-yl)propyloxy]-7-methoxy-chinazolin,

(4) 4-[(3-Bromphenyl)amino]-6-(3-{4-[(ethoxycarbonyl)methyl]-piperazin-1-yl}-2-hydroxy-propyloxy)-7-methoxy-chinazolin,

(5) (*S*)-4-[(3-Bromphenyl)amino]-6-({1-[(ethoxycarbonyl)methyl]-pyrrolidin-2-yl}methoxy)-7-methoxy-chinazolin und

(6) 4-[(3-Bromphenyl)amino]-6-(2-{4-[1,2 bis(methoxycarbonyl)-ethyl]-piperazin-1-yl}ethoxy)-7-methoxy-chinazolin

sowie deren Salze.

**6.** Physiologisch verträgliche Salze der Verbindungen gemäß den Ansprüchen 1 bis 5 mit anorganischen oder organischen Säuren oder Basen.

**7.** Arzneimittel, enthaltend eine Verbindung gemäß den Ansprüchen 1 bis 5 oder ein physiologisch verträgliches Salz gemäß Anspruch 6 neben gegebenenfalls einem oder mehreren inerten Trägerstoffen und/oder Verdünnungsmitteln.

**8.** Verwendung einer Verbindung gemäß den Ansprüchen 1 bis 6 zur Herstellung eines Arzneimittels, das zur Behandlung von benignen oder malignen Tumoren, zur Vorbeugung und Behandlung von Erkrankungen der Atemwege und der Lunge, zur Behandlung von Polypen, von Erkrankungen des Magen-Darm-Traktes, der Gallengänge und -blase sowie der Niere und Haut geeignet ist.

**9.** Verfahren zur Herstellung eines Arzneimittels gemäß Anspruch 7, **dadurch gekennzeichnet, daß** auf nichtchemischem Wege eine Verbindung gemäß den Ansprüchen 1 bis 6 in einen oder mehrere inerte Trägerstoffe und/oder Verdünnungsmittel eingearbeitet wird.

**10.** Verfahren zur Herstellung der Verbindungen der allgemeinen Formel I gemäß den Ansprüchen 1 bis 6, **dadurch gekennzeichnet, daß**

a) eine Verbindung der allgemeinen Formel

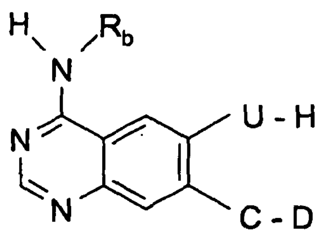

, (II)

in der
$R_b$, C und D wie in den Ansprüchen 1 bis 5 erwähnt definiert sind und
U ein Sauerstoffatom oder eine $R_4N$-Gruppe darstellt, wobei $R_4$ wie in den Ansprüchen 1 bis 5 erwähnt definiert ist, mit einer Verbindung der allgemeinen Formel

$$Z_1 - A' - B , \qquad (III)$$

in der
B wie in den Ansprüchen 1 bis 5 erwähnt definiert ist,
A' einen der in den Ansprüchen 1 bis 5 für den Rest A erwähnten gegebenenfalls substituierten Alkylen-, Cycloalkylen-, Alkylen-cycloalkylen-, Cycloalkylen-alkylen- oder Alkylen-cycloalkylen-alkylen-Teile darstellt, die über ein Sauerstoffatom oder über eine $NR_4$-Gruppe mit dem Heteroaromaten verknüpft sind, und
$Z_1$ eine Austrittsgruppe bedeutet, umgesetzt wird oder

b) eine Verbindung der allgemeinen Formel

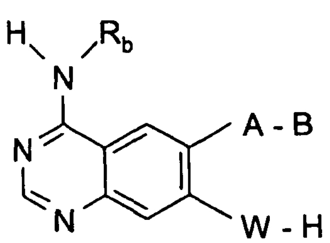

, (IV)

in der
$R_b$, A und B wie in den Ansprüchen 1 bis 5 erwähnt definiert sind und

W ein Sauerstoffatom oder eine $R_4N$-Gruppe darstellt, wobei $R_4$ wie in den Ansprüchen 1 bis 5 erwähnt definiert ist, mit einer Verbindung der allgemeinen Formel

$$Z_2 - C' - D , \qquad (V)$$

in der
D wie in den Ansprüchen 1 bis 5 erwähnt definiert ist,
C' einen der in den Ansprüchen 1 bis 5 für den Rest C erwähnten gegebenenfalls substituierten Alkylen-, Cycloalkylen-, Alkylen-cycloalkylen-, Cycloalkylen-alkylen- oder Alkylen-cycloalkylen-alkylen-Teile darstellt, die über ein Sauerstoffatom oder über eine $NR_4$-Gruppe mit dem Heteroaromaten verknüpft sind, und
$Z_2$ eine Austrittsgruppe darstellt, umgesetzt wird oder

c) zur Herstellung einer Verbindung der allgemeinen Formel I, in der A mit Ausnahme des Sauerstoffatoms und der $-NR_4$-Gruppe wie in den Ansprüchen 1 bis 5 erwähnt definiert ist, eine Verbindung der allgemeinen Formel

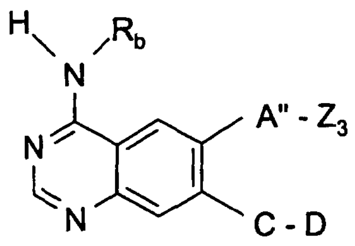

, (VI)

in der
$R_b$, C und D wie in den Ansprüchen 1 bis 5 erwähnt definiert sind und
A" mit Ausnahme des Sauerstoffatoms und der $-NR_4$-Gruppe die für A in den Ansprüchen 1 bis 5 erwähnten Bedeutungen aufweist und
$Z_3$ eine Austrittsgruppe oder zusammen mit einem Wasserstoffatom einer benachbarten Kohlenwasserstoffgruppe ein Sauerstoffatom darstellt, mit einer Verbindung der allgemeinen Formel

$$H - B , \qquad (VII)$$

in der
B wie in den Ansprüchen 1 bis 5 erwähnt definiert ist, umgesetzt wird oder

d) zur Herstellung einer Verbindung der allgemeinen Formel I, in der C mit Ausnahme des Sauerstoffatoms und der $-NR_4$-Gruppe wie in den Ansprüchen 1 bis 5 erwähnt definiert ist, eine Verbindung der allgemeinen Formel

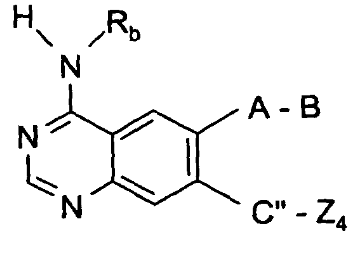

, (VIII)

in der
$R_b$, A und B wie in den Ansprüchen 1 bis 5 erwähnt definiert sind und
C" mit Ausnahme des Sauerstoffatoms und der $-NR_4$-Gruppe die für C in den Ansprüchen 1 bis 5 erwähnten

Bedeutungen aufweist und $Z_4$ eine Austrittsgruppe oder zusammen mit einem Wasserstoffatom einer benachbarten Kohlenwasserstoffgruppe ein Sauerstoffatom darstellt, mit einer Verbindung der allgemeinen Formel

$$H - D , \qquad (IX)$$

in der
D wie in den Ansprüchen 1 bis 5 erwähnt definiert ist, umgesetzt wird oder

e) zur Herstellung einer Verbindung der allgemeinen Formel I, in der B eine $R_6O$-CO-alkylen-$NR_5$-Gruppe, in der der Alkylenteil, welcher geradkettig ist und 1 bis 6 Kohlenstoffatome enthält, zusätzlich durch eine oder zwei $C_{1-2}$-Alkylgruppen oder durch eine $R_6O$-CO- oder $R_6O$-CO-$C_{1-2}$-alkylgruppe substituiert sein kann,
eine in 4-Stellung durch eine $R_6O$-CO-$C_{1-4}$-alkyl- oder Bis-($R_6O$-CO)-$C_{1-4}$-alkylgruppe substituierte Piperazino- oder Homopiperazinogruppe oder
eine in 1-Stellung durch eine $R_6O$-CO-$C_{1-4}$-alkyl- oder Bis-($R_6O$-CO)-$C_{1-4}$-alkylgruppe substituiert Pyrrolidinyl-, Piperidinyl- oder Hexahydroazepinylgruppe, wobei jeweils $R_5$ und $R_6$ wie in den Ansprüchen 1 bis 5 erwähnt definiert sind, darstellt, eine Verbindung der allgemeinen Formel

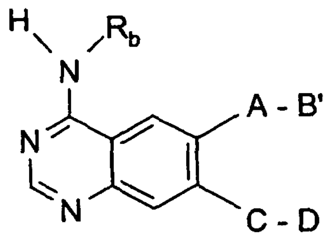

, (X)

in der
$R_b$, A, C und D wie in den Ansprüchen 1 bis 5 erwähnt definiert sind und
B' eine $R_5$NH-Gruppe, in der $R_5$ wie in den Ansprüchen 1 bis 5 erwähnt definiert ist, eine in 4-Stellung unsubstituierte Piperazino- oder Homopiperazinogruppe, eine in 1-Stellung unsubstituierte Pyrrolidinyl-, Piperidinyl- oder Hexahydroazepinylgruppe bedeutet, mit einer Verbindung der allgemeinen Formel

$$R_6O\text{-CO-alkylen-}Z_5 , \qquad (XI)$$

in der
der Alkylenteil, welcher geradkettig ist und 1 bis 6 Kohlenstoffatome enthält, zusätzlich durch eine oder zwei $C_{1-2}$-Alkylgruppen oder durch eine $R_6O$-CO- oder $R_6O$-CO-$C_{1-2}$-alkylgruppe substituiert sein kann, wobei $R_6$ jeweils wie in den Ansprüchen 1 bis 5 erwähnt definiert ist, und
$Z_5$ eine austauschbare Gruppe bedeutet, umgestzt wird oder

f) zur Herstellung einer Verbindung der allgemeinen Formel I, in der D eine $R_6O$-CO-alkylen-$NR_5$-Gruppe, in der der Alkylenteil, welcher geradkettig ist und 1 bis 6 Kohlenstoffatome enthält, zusätzlich durch eine oder zwei $C_{1-2}$-Alkylgruppen oder durch eine $R_6O$-CO- oder $R_6O$-CO-$C_{1-2}$-alkylgruppe substituiert sein kann,
eine in 4-Stellung durch eine $R_6O$-CO-$C_{1-4}$-alkyl- oder Bis-($R_6O$-CO)-$C_{1-4}$-alkylgruppe substituierte Piperazino- oder Homopiperazinogruppe oder
eine in 1-Stellung durch eine $R_6O$-CO-$C_{1-4}$-alkyl- oder Bis-($R_6O$-CO)-$C_{1-4}$-alkylgruppe substituiert Pyrrolidinyl-, Piperidinyl- oder Hexahydroazepinylgruppe, wobei jeweils $R_5$ und $R_6$ wie in den Ansprüchen 1 bis 5 erwähnt definiert sind, darstellt, eine Verbindung der allgemeinen Formel

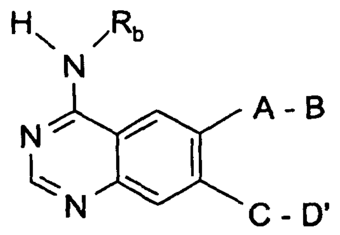

, (XII)

in der
$R_b$, A, B und C wie in den Ansprüchen 1 bis 5 erwähnt definiert sind und
D' eine $R_5$NH-Gruppe, in der $R_5$ wie in den Ansprüchen 1 bis 5 erwähnt definiert ist, eine in 4-Stellung unsubstituierte Piperazino- oder Homopiperazinogruppe, eine in 1-Stellung unsubstituierte Pyrrolidinyl-, Piperidinyl- oder Hexahydroazepinylgruppe bedeutet, mit einer Verbindung der allgemeinen Formel

$$R_6O\text{-}CO\text{-alkylen-}Z_5 \ , \qquad (XI)$$

in der
der Alkylenteil, welcher geradkettig ist und 1 bis 6 Kohlenstoffatome enthält, zusätzlich durch eine oder zwei $C_{1-2}$-Alkylgruppen oder durch eine $R_6O$-CO- oder $R_6O$-CO-$C_{1-2}$-alkylgruppe substituiert sein kann, wobei $R_6$ jeweils wie in den Ansprüchen 1 bis 5 erwähnt definiert ist, und
$Z_5$ eine austauschbare Gruppe bedeutet, umgesetzt wird oder

g) zur Herstellung einer Verbindung der allgemeinen Formel I, in der mindestens einer der Reste $R_6$ bis $R_8$ ein Wasserstoffatom darstellen, eine Verbindung der allgemeinen Formel

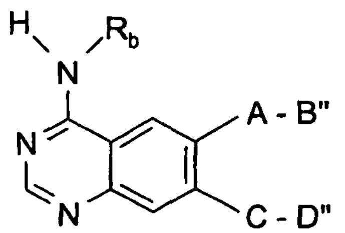

, (XIII)

in der
$R_b$, A und C wie in den Ansprüchen 1 bis 5 erwähnt definiert sind,
B" und D" die für B und D in den Ansprüchen 1 bis 5 erwähnten Bedeutungen mit der Maßgabe aufweisen, dass mindestens einer der Reste B" oder D" eine $R_6O$-CO-, ($R_7O$-PO-$OR_8$)- oder ($R_7O$-PO--$R_9$)-Gruppe enthält, in der $R_9$ wie in den Ansprüchen 1 bis 5 erwähnt definiert ist und mindestens einer der Reste $R_6$ bis $R_8$ kein Wasserstoffatom darstellt, mittels Hydrolyse, Behandeln mit Säuren, Thermolyse oder Hydrogenolyse in eine Verbindung der allgemeinen Formel I übergeführt wird, in der mindestens einer der Reste $R_6$ bis $R_8$ ein Wasserstoffatom darstellt,
und gewünschtenfalls anschließend eine so erhaltene Verbindung der allgemeinen Formel I, die eine Carboxy- oder Hydroxyphosphorylgruppe enthält, mittels Veresterung in einen entsprechenden Ester der allgemeinen Formel I übergeführt wird und/oder
eine so erhaltene Verbindung der allgemeinen Formel I, in der B oder D einen gegebenenfalls substituierten N-(2-Hydroxyethyl)-glycin- oder N-(2-Hydroxyethyl)-glycinesterrest darstellt, mittels Cyclisierung in eine entsprechende 2-Oxo-morpholinoverbindung übergeführt wird und/oder
erforderlichenfalls ein bei den vorstehend beschriebenen Umsetzungen verwendeter Schutzrest wieder abgespalten wird und/oder
gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische

Anwendung in ihre physiologisch verträglichen Salze übergeführt wird.

## Revendications

**1.** Hétérocycles bicycliques de formule générale

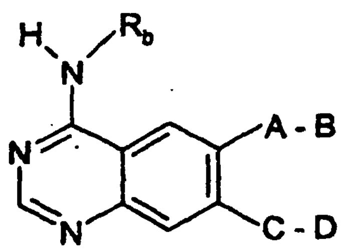

, (I)

où

$R_b$ représente un groupe phényle, benzyle ou 1-phényléthyle où le noyau phényle est substitué dans chaque cas par les groupes $R_1$ à $R_2$, tandis que

$R_1$ et $R_2$, qui peuvent être identiques ou différents, représentent chacun un atome d'hydrogène, de fluor, de chlore ou de brome,

ou un groupe méthyle, trifluorométhyle, méthoxy, éthynyle ou cyano,

A représente un groupe -O-$C_{1-4}$-alkylène ou -O-$CH_2$-CH(OH)-$CH_2$, tandis que l'atome d'oxygène des groupes mentionnés précédemment est lié dans chaque cas au cycle hétéroaromatique bicyclique,

B représente un groupe $R_6$O-CO-alkylène-$NR_5$ où le groupement alkylène, qui est linéaire et contient 1 ou 2 atomes de carbone, peut être substitué en outre par un groupe $R_6$O-CO ou $R_6$O-CO-méthyle, tandis que

$R_5$ représente un atome d'hydrogène,

un groupe $C_{1-2}$-alkyle qui peut être substitué par un groupe $R_6$O-CO,

un groupe $C_{2-4}$-alkyle qui est substitué à partir de la position 2 par un groupe hydroxy,

un groupe $C_{3-6}$-cycloalkyle ou $C_{3-6}$-cycloalkylméthyle et

$R_6$ représente un atome d'hydrogène,

un groupe $C_{1-6}$-alkyle, cyclopentyle, cyclopentylméthyle, cyclohexyle, cyclohexylméthyle, phényle, benzyle, 5-indanyle ou $R_g$CO-O-($R_e$C$R_f$), tandis que

$R_e$ représente un atome d'hydrogène ou un groupe $C_{1-4}$-alkyle,

$R_f$ représente un atome d'hydrogène et

$R_g$ représente un groupe $C_{1-4}$-alkyle, cyclopentyle, cyclohexyle, $C_{1-4}$-alcoxy, cyclopentyloxy ou cyclohexyloxy,

un groupe pyrrolidino ou pipéridino qui est substitué par un groupe $R_6$O-CO ou $R_6$O-CO-$C_{1-2}$-alkyle où $R_6$ est défini comme précédemment,

un groupe pyrrolidino ou pipéridino qui est substitué par deux groupes $R_6$O-CO ou $R_6$O-CO-$C_{1-2}$-alkyle où $R_6$ est défini comme précédemment,

un groupe pipérazino qui est substitué à la position 4 par le groupe $R_{10}$ et en outre au niveau d'un atome de carbone cyclique par un groupe $R_6$O-CO ou $R_6$O-CO-$C_{1-2}$-alkyle

où $R_6$ est défini comme précédemment et

$R_{10}$ représente un atome d'hydrogène, un groupe méthyle ou éthyle, un groupe pipérazino qui est substitué à la position 4 par un groupe $R_6$O-CO-$C_{1-4}$-alkyle, bis-($R_6$O-CO)-$C_{1-4}$-alkyle, ($R_7$O-PO-O$R_8$)-méthyle ou ($R_7$O-PO-$R_9$)-méthyle où $R_6$ est défini comme précédemment,

$R_7$ et $R_8$, qui peuvent être identiques ou différents, représentent dans chaque cas un atome d'hydrogène, un groupe méthyle, éthyle, phényle, benzyle, 5-indanyle ou $R_g$CO-O-($R_e$C$R_f$), tandis que

$R_e$ à $R_g$ sont définis comme précédemment,

et $R_9$ représente un groupe méthyle ou éthyle,

un groupe pipérazino qui est substitué à la position 4 par un groupe $R_6$O-CO-$C_{1-2}$-alkyle et qui est substitué en outre au niveau d'un atome de carbone cyclique par un groupe $R_6$O-CO ou $R_6$O-CO-$C_{1-2}$-alkyle où $R_6$ est défini comme précédemment,

un groupe morpholino qui est substitué par un groupe $R_6$O-CO ou $R_6$O-CO-$C_{1-2}$-alkyle, tandis que $R_6$ est défini comme précédemment,

un groupe pyrrolidinyle ou pipéridinyle substitué à la position 1 par un groupe $R_6O$-CO-$C_{1-4}$-alkyle, bis-($R_6O$-CO)-$C_{1-4}$-alkyle, ($R_7O$-PO-$OR_8$)-méthyle ou ($R_7O$-PO-$R_9$)-méthyle où $R_6$ à $R_9$ sont définis comme précédemment,
un groupe 2-oxo-morpholino qui peut être substitué par 1 ou 2 groupes méthyle,
un groupe 2-oxo-morpholinyle qui est substitué à la position 4 par un groupe méthyle, éthyle ou $R_6O$-CO-$C_{1-2}$-alkyle, tandis que $R_6$ est défini comme précédemment et les groupes 2-oxo-morpholinyle mentionnés précédemment sont liés dans chaque cas à un atome de carbone du groupe A, ou
un groupe $R_{11}N$($C_{1-2}$-alkyle) où $R_{11}$ représente un groupe 2-oxo-tétrahydrofuran-3-yle ou 2-oxo-tétrahydrofuran-4-yle, et
C et D représentent ensemble un atome d'hydrogène, un groupe méthoxy, éthoxy, 2-méthoxy-éthoxy, $C_{4-6}$-cycloalcoxy ou $C_{3-6}$-cycloalkyl-$C_{1-3}$-alcoxy,
leurs tautomères, leurs stéréoisomères et leurs sels.

**2.** Hétérocycles bicycliques de formule générale I où
$R_b$ représente un groupe phényle, benzyle ou 1-phényléthyle où le noyau phényle est substitué dans chaque cas par les groupes $R_1$ à $R_2$, tandis que
$R_1$ et $R_2$, qui peuvent être identiques ou différents, représentent chacun un atome d'hydrogène, de fluor, de chlore ou de brome,
ou un groupe méthyle, trifluorométhyle, méthoxy, éthynyle ou cyano,
A et B représentent ensemble un atome d'hydrogène, un groupe méthoxy, éthoxy, 2-méthoxy-éthoxy, $C_{4-6}$-cycloalcoxy ou $C_{3-6}$-cycloalkyl-$C_{1-3}$-alcoxy,
C représente un groupe -O-$C_{1-4}$-alkylène ou -O-$CH_2$-CH(OH)-$CH_2$, tandis que l'atome d'oxygène des groupes mentionnés précédemment est lié dans chaque cas
au cycle hétéroaromatique bicyclique, et
D représente un groupe $R_6O$-CO-alkylène-$NR_5$ où le groupement alkylène, qui est linéaire et contient 1 ou 2 atomes de carbone, peut être substitué en outre par un groupe $R_6O$-CO ou $R_6O$-CO-méthyle, tandis que
$R_5$ représente un atome d'hydrogène,
un groupe $C_{1-2}$-alkyle qui peut être substitué par un groupe $R_6O$-CO,
un groupe $C_{2-4}$-alkyle qui est substitué à partir de la position 2 par un groupe hydroxy,
un groupe $C_{3-6}$-cycloalkyle ou $C_{3-6}$-cycloalkylméthyle et
$R_6$ représente un atome d'hydrogène,
un groupe $C_{1-6}$-alkyle, cyclopentyle, cyclopentylméthyle, cyclohexyle, cyclohexylméthyle, phényle, benzyle, 5-indanyle ou $R_gCO$-O-($R_eCR_f$), tandis que
$R_e$ représente un atome d'hydrogène ou un groupe $C_{1-4}$-alkyle,
$R_f$ représente un atome d'hydrogène et
$R_g$ représente un groupe $C_{1-4}$-alkyle, cyclopentyle, cyclohexyle, $C_{1-4}$-alcoxy, cyclopentyloxy ou cyclohexyloxy,
un groupe pyrrolidino ou pipéridino qui est substitué par un groupe $R_6O$-CO ou $R_6O$-CO-$C_{1-2}$-alkyle où $R_6$ est défini comme précédemment,
un groupe pyrrolidino ou pipéridino qui est substitué par deux groupes $R_6O$-CO ou $R_6O$-CO-$C_{1-2}$-alkyle où $R_6$ est défini comme précédemment,
un groupe pipérazino qui est substitué à la position 4 par le groupe $R_{10}$ et en outre au niveau d'un atome de carbone cyclique par un groupe $R_6O$-CO ou $R_6O$-CO-$C_{1-2}$-alkyle
où $R_6$ est défini comme précédemment et
$R_{10}$ représente un atome d'hydrogène, un groupe méthyle ou éthyle,
un groupe pipérazino qui est substitué à la position 4 par un groupe $R_6O$-CO-$C_{1-4}$-alkyle, bis-($R_6O$-CO)-$C_{1-4}$-alkyle, ($R_7O$-PO-$OR_8$)-méthyle ou ($R_7O$-PO-$R_9$)-méthyle où $R_6$ est défini comme précédemment,
$R_7$ et $R_8$, qui peuvent être identiques ou différents, représentent dans chaque cas
un atome d'hydrogène, un groupe méthyle, éthyle, phényle, benzyle, 5-indanyle ou $R_gCO$-O-($R_eCR_f$), tandis que
$R_e$ à $R_g$ sont définis comme précédemment,
et $R_9$ représente un groupe méthyle ou éthyle,
un groupe pipérazino qui est substitué à la position 4 par un groupe $R_6O$-CO-$C_{1-2}$-alkyle et qui est substitué en outre au niveau d'un atome de carbone cyclique par
un groupe $R_6O$-CO ou $R_6O$-CO-$C_{1-2}$-alkyle où $R_6$ est défini comme précédemment,
un groupe morpholino qui est substitué par un groupe $R_6O$-CO ou $R_6O$-CO-$C_{1-2}$-alkyle, tandis que $R_6$ est défini comme précédemment,
un groupe pyrrolidinyle ou pipéridinyle substitué à la position 1 par un groupe $R_6O$-CO-$C_{1-4}$-alkyle, bis-($R_6O$-CO)-$C_{1-4}$-alkyle, ($R_7O$-PO-$OR_8$)-méthyle ou ($R_7O$-PO-$R_9$)-méthyle où $R_6$ à $R_9$ sont définis comme précédemment,

un groupe 2-oxo-morpholino qui peut être substitué par 1 ou 2 groupes méthyle,
un groupe 2-oxo-morpholinyle qui est substitué à la position 4 par un groupe méthyle, éthyle ou $R_6O$-CO-$C_{1-2}$-alkyle, tandis que $R_6$ est défini comme précédemment et les groupes 2-oxo-morpholinyle mentionnés précédemment sont liés dans chaque cas à un atome de carbone du groupe C, ou
un groupe $R_{11}N(C_{1-2}$-alkyle) où $R_{11}$ représente un groupe 2-oxo-tétrahydrofuran-3-yle ou 2-oxo-tétrahydrofuran-4-yle,
leurs tautomères, leurs stéréoisomères et leurs sels.

**3.** Hétérocycles bicycliques de formule générale I selon la revendication 1 où
$R_b$ représente un groupe phényle où le noyau phényle est substitué dans chaque cas par les groupes $R_1$ à $R_2$, tandis que
$R_1$ et $R_2$, qui peuvent être identiques ou différents, représentent chacun un atome d'hydrogène, de fluor, de chlore ou de brome,
A représente un groupe -O-$C_{1-4}$-alkylène ou -O-$CH_2$-CH(OH)-$CH_2$, tandis que l'atome d'oxygène des groupes mentionnés précédemment est lié dans chaque cas au cycle hétéroaromatique bicyclique,
B représente un groupe $R_6O$-CO-$CH_2$-$NR_5$ où
$R_5$ représente un atome d'hydrogène ou un groupe méthyle qui peut être substitué par un groupe $R_6O$-CO, ou un groupe $C_{2-4}$-alkyle substitué à partir de la position 2 par un groupe hydroxy, et
$R_6$ représente un atome d'hydrogène, un groupe méthyle ou éthyle,
un groupe pyrrolidino ou pipéridino qui est substitué par un groupe $R_6O$-CO tandis que $R_6$ est défini comme précédemment,
un groupe pipérazino qui est substitué à la position 4 par un groupe $R_6O$-CO-$CH_2$ ou bis-($R_6O$-CO)-$C_{1-3}$-alkyle, tandis que $R_6$ est défini comme précédemment,
un groupe pyrrolidinyle ou pipéridinyle substitué à la position 1 par un groupe $R_6O$-CO-$CH_2$, tandis que $R_6$ est défini comme précédemment,
un groupe 2-oxo-morpholino qui peut être substitué par un ou deux groupes méthyle, ou
un groupe $R_{11}N(C_{1-2}$-alkyle) où $R_{11}$ représente un groupe 2-oxo-tétrahydrofuran-3-yle ou 2-oxo-tétrahydrofuran-4-yle, et
C et D représentent ensemble un groupe méthoxy, $C_{4-6}$-cycloalcoxy ou $C_{3-6}$-cycloalkylméthoxy,
leurs tautomères, leurs stéréoisomères et leurs sels.

**4.** Hétérocycles bicycliques de formule générale I selon la revendication 2 où
$R_b$ représente un groupe phényle où le noyau phényle est substitué dans chaque cas par les groupes $R_1$ à $R_2$, tandis que
$R_1$ et $R_2$, qui peuvent être identiques ou différents, représentent chacun un atome d'hydrogène, de fluor, de chlore ou de brome,
A et B représentent ensemble un groupe $C_{4-6}$-cycloalcoxy ou $C_{3-6}$-cycloalkylméthoxy,
C représente un groupe -O-$CH_2CH_2$, tandis que l'atome d'oxygène du groupe mentionné précédemment est lié au cycle hétéroaromatique bicyclique,
D représente un groupe $R_6O$-CO-$CH_2$-$NR_5$ où
$R_5$ représente un groupe $C_{2-4}$-alkyle substitué à partir de la position 2 par un groupe hydroxy, et
$R_6$ représente un groupe méthyle ou éthyle,
un groupe 2-oxo-morpholino qui peut être substitué par un ou deux groupes méthyle, ou
un groupe $R_{11}N(C_{1-2}$-alkyle) où $R_{11}$ représente un groupe 2-oxo-tétrahydrofuran-3-yle ou 2-oxo-tétrahydrofuran-4-yle, et
leurs tautomères, leurs stéréoisomères et leurs sels.

**5.** Hétérocycles bicycliques de formule générale I selon la revendication 1 ou 2 suivants :

(1) 4-(3-chloro-4-fluorophénylamino)-6-{3-[4-(méthoxycarbonylméthyl)-1-pipérazinyl]propyloxy}-7-méthoxy-quinazoline,
(2) 4-[(3-bromophényl)amino]-6-(2-{4-[(éthoxycarbonyl)méthyl]-pipérazin-1-yl}éthoxy)-7-méthoxy-quinazoline,
(3) (*S*)-4-[(3-bromophényl)amino]-6-[3-(2-méthoxycarbonyl-pyrrrolidin-1-yl)propyloxy]-7-méthoxy-quinazoline,
(4) 4-[(3-bromophényl)amino]-6-(3-{4-[(éthoxycarbonyl)méthyl]-pipérazin-1-yl}-2-hydroxy-propyloxy)-7-méthoxy-quinazoline,
(5) (*S*)-4-[(3-bromophényl)amino]-6-({1-[(éthoxycarbonyl)méthyl]-pyrrolidin-2-yl}méthoxy)-7-méthoxy-quinazoline et
(6) 4-[(3-bromophényl)amino]-6-(2-{4-[1,2-bis(méthoxycarbonyl)éthyl]-pipérazin-1-yl}éthoxy)-7-méthoxy-quinazoline

et leurs sels.

**6.** Sels physiologiquement acceptables des composés selon les revendications 1 à 5 avec des acides ou bases inorganiques ou organiques.

**7.** Compositions pharmaceutiques contenant un composé selon les revendications 1 à 5 ou un sel physiologiquement acceptable selon la revendication 6 éventuellement avec un ou plusieurs supports et/ou diluants inertes.

**8.** Utilisation d'un composé selon au moins l'une des revendications 1 à 6 pour préparer une composition pharmaceutique qui est appropriée pour traiter les tumeurs bénignes ou malignes, pour prévenir et traiter les maladies des voies aériennes et des poumons, pour traiter les polypes, les maladies des voies gastro-intestinales, des canaux biliaires et de la vésicule biliaire et des reins et de la peau.

**9.** Procédé pour préparer une composition pharmaceutique selon la revendication 7 **caractérisé en ce qu'**un composé selon au moins l'une des revendications 1 à 6 est incorporé dans un ou plusieurs supports et/ou diluants inertes par un procédé non chimique.

**10.** Procédé pour préparer les composés de formule générale I selon les revendications 1 à 6, **caractérisé en ce que**

a) un composé de formule générale

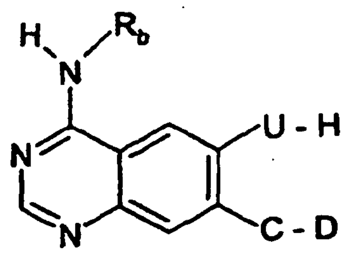

, (II)

où
$R_b$, C et D sont définis comme dans les revendications 1 à 5 et
U représente un atome d'oxygène ou un groupe $R_4N$, tandis que $R_4$ est défini comme dans les revendications 1 à 5, est mis à réagir avec un composé de formule générale

$$Z_1\text{-A'-B} \qquad \text{(III)}$$

où
B est défini comme dans les revendications 1 à 5,
A' représente l'un des groupements alkylène, cycloalkylène, alkylène-cycloalkylène, cycloalkylène-alkylène ou alkylène-cycloalkylène-alkylène éventuellement substitués mentionnés dans les revendications 1 à 5 pour le groupe A, qui sont liés au groupe hétéroaromatique par le biais d'un atome d'oxygène ou par le biais d'un groupe $NR_4$, et $Z_1$ représente un groupe partant, ou
b) un composé de formule générale

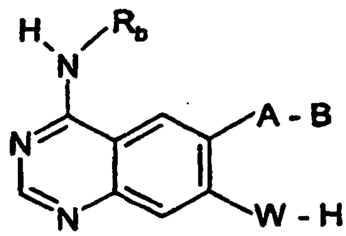

, (IV)

où

$R_b$, A et B sont définis comme dans les revendications 1 à 5 et W représente un atome d'oxygène ou un groupe $R_4N$, tandis que $R_4$ est défini comme dans les revendications 1 à 5, est mis à réagir avec un composé de formule générale

$Z_2$-C'-D (V)

où

D est défini comme dans les revendications 1 à 5,

C' représente l'un des groupements alkylène, cycloalkylène, alkylène-cycloalkylène, cycloalkylène-alkylène ou alkylène-cycloalkylène-alkylène éventuellement substitués mentionnés ci-dessus pour le groupe C, qui sont liés au groupe hétéroaromatique par le biais d'un atome d'oxygène ou par le biais d'un groupe $NR_4$, et

$Z_2$ représente un groupe partant, ou

c) pour préparer un composé de formule générale I où A est défini comme dans les revendications 1 à 5 à l'exception de l'atome d'oxygène et du groupe $-NR_4$ :

un composé de formule générale

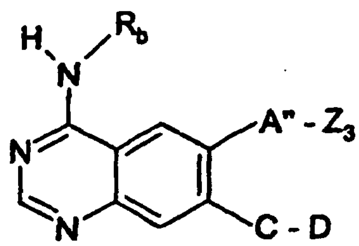

, (VI)

où

$R_b$, C et D sont définis comme dans les revendications 1 à 5 et

A" a les significations données pour A dans les revendications 1 à 5 à l'exception de l'atome d'oxygène et du groupe $-NR_4$ et

$Z_3$ représente un groupe partant ou, avec un atome d'hydrogène d'un groupe hydrocarboné adjacent, représente un atome d'oxygène, est mis à réagir avec un composé de formule générale

H - B (VII)

où

B est défini comme dans les revendications 1 à 5, ou

d) pour préparer un composé de formule générale I où C est défini comme dans les revendications 1 à 5 à l'exception de l'atome d'oxygène et du groupe $-NR_4$ :

un composé de formule générale

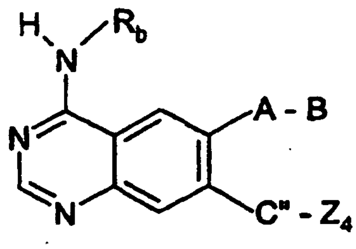
, (VIII)

où

$R_b$, A et B sont définis comme dans les revendications 1 à 5 et

C" a les significations données pour C dans les revendications 1 à 5 à l'exception de l'atome d'oxygène et du groupe $-NR_4$ et $Z_4$ représente un groupe partant ou, avec un atome d'hydrogène d'un groupe hydrocarboné adjacent, représente un atome d'oxygène, est mis à réagir avec un composé de formule générale

H-D (IX)

où

D est défini comme dans les revendications 1 à 5, ou

e) pour préparer un composé de formule générale I où B représente un groupe $R_6O$-CO-alkylène-$NR_5$ où le groupement allcylène, qui est linéaire et contient 1 à 6 atomes de carbone, peut être substitué en outre par un ou deux groupes $C_{1-2}$-alkyle ou par un groupe $R_6O$-CO ou $R_6O$-CO-$C_{1-2}$-alkyle, un groupe pipérazino ou homopipérazino substitué à la position 4 par un groupe $R_6O$-CO-$C_{1-4}$-alkyle ou bis-($R_6O$-CO)-$C_{1-4}$-alkyle ou un groupe pyrrolidinyle, pipéridinyle ou hexahydroazépinyle substitué à la position 1 par un groupe $R_6O$-CO-$C_{1-4}$-alkyle ou bis-($R_6O$-CO)-$C_{1-4}$-alkyle, tandis que, dans chaque cas, $R_5$ et $R_6$ sont définis comme dans les revendications 1 à 5 :

un composé de formule générale

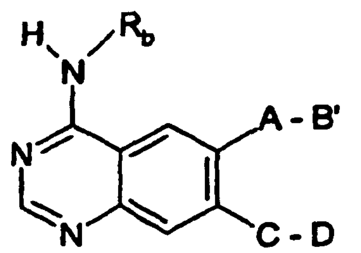
, (X)

où

$R_b$, A, C et D sont définis comme dans les revendications 1 à 5 et

B' représente un groupe $R_5NH$ où $R_5$ est défini comme dans les revendications 1 à 5, un groupe pipérazino ou homopipérazino non substitué à la position 4, un groupe pyrrolidinyle, pipéridinyle ou hexahydroazépinyle non substitué à la position 1, est mis à réagir avec un composé de formule générale

$R_6O$-CO-alkylène-$Z_5$ (XI)

où

le groupement allcylène, qui est linéaire et contient 1 à 6 atomes de carbone, peut être substitué en outre par un ou deux groupes $C_{1-2}$-alkyle ou par un groupe $R_6O$-CO ou $R_6O$-CO-$C_{1-2}$-alkyle, tandis que $R_6$ est défini dans chaque cas comme dans les revendications 1 à 5 et $Z_5$ représente un groupe échangeable, ou

f) pour préparer un composé de formule générale I où D représente un groupe $R_6O$-CO-alkylène-$NR_5$ où le groupement alkylène, qui est linéaire et contient 1 à 6 atomes de carbone, peut être substitué en outre par un ou deux groupes $C_{1-2}$-alkyle
ou par un groupe $R_6O$-CO ou $R_5O$-CO-$C_{1-2}$-alkyle, un groupe pipérazino ou homopipérazino substitué à la position 4 par un groupe $R_6O$-CO-$C_{1-4}$-alkyle ou bis-($R_6O$-CO)-$C_{1-4}$-alkyle ou un groupe pyrolidinyle, pipéridinyle ou hexahydroazépinyle substitué à la position 1 par un groupe $R_6O$-CO-$C_{1-4}$-alkyle ou bis-($R_6O$-CO) -$C_{1-4}$-alkyle, tandis que, dans chaque cas, $R_5$ et $R_6$ sont définis comme dans les revendications 1 à 5 :

un composé de formule générale

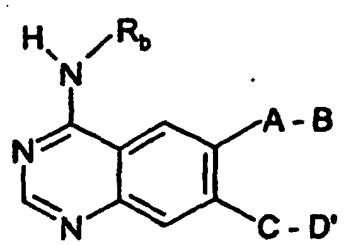

, (XII)

où
$R_b$, A, B et C sont définis comme dans les revendications 1 à 5 et
D' représente un groupe $R_5NH$ où $R_5$ est défini comme dans les revendications 1 à 5, un groupe pipérazino ou homopipérazino non substitué à la position 4, un groupe pyrrolidinyle, pipéridinyle ou hexahydroazépinyle non substitué à la position 1, est mis à réagir avec un composé de formule générale

$R_6O$-CO-alkylène-$Z_5$ (XI)

où
le groupement alkylène, qui est linéaire et contient 1 à 6 atomes de carbone, peut être substitué en outre par un ou deux groupes $C_{1-2}$-alkyle ou par un groupe $R_6O$-CO ou $R_6O$-CO-$C_{1-2}$-alkyle, tandis que $R_6$ est défini dans chaque cas comme dans les revendications 1 à 5 et $Z_5$ représente un groupe échangeable, ou
g) pour préparer un composé de formule générale I où au moins l'un des groupes $R_6$ à $R_8$ représente un atome d'hydrogène :

un composé de formule générale

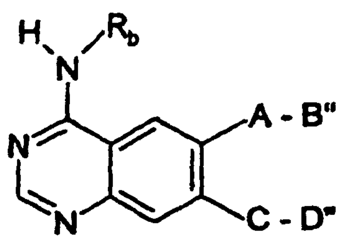

, (XIII)

où
$R_b$, A et C sont définis comme dans les revendications 1 à 5,
B" et D" ont les significations données pour B et D dans les revendications 1 à 5, avec la condition qu'au moins l'un des groupes B" ou D" contient un groupe $R_6O$-CO, ($R_7O$-PO-$OR_8$) ou ($R_7O$-PO-$R_9$) où $R_9$ est défini comme dans les revendications 1 à 5 et au moins l'un des groupes $R_6$ à $R_8$ ne représente pas un atome d'hydrogène, est converti par hydrolyse, traitement avec des acides, thermolyse ou hydrogénolyse en un composé de formule générale I où au moins l'un des groupes $R_6$ à $R_8$ représente un atome d'hydrogène,
puis, si on le souhaite, un composé de formule générale I ainsi obtenu qui contient un groupe carboxy ou hydroxyphosphoryle est converti par estérification en un ester correspondant de formule générale I et/ou
un composé de formule générale I ainsi obtenu où B ou D représente un groupe N-(2-hydroxyéthyl)-glycine ou ester de N-(2-hydroxyéthyl)-glycine éventuellement substitué est converti par cyclisation en un composé 2-oxo-morpholino correspondant, et/ou
si nécessaire, tout groupe protecteur utilisé pendant les réactions décrites ci-dessus est clivé de nouveau et/ou
si on le souhaite un composé de formule générale I ainsi obtenu est résolu en ses stéréoisomères et/ou
un composé de formule générale I ainsi obtenu est converti en ses sels, plus particulièrement, pour l'utilisation pharmaceutique, en ses sels physiologiquement acceptables.